# EUROPEAN PATENT APPLICATION

(11) **EP 4 043 565 A1**
(43) Date of publication of application: **17.08.2022**
(21) Application number: 20875085.1
(22) Date of filing: 09.10.2020
(51) Int. Cl.: C12N 15/113, A61K 31/713

(54) **MODIFIED HETERONUCLEIC ACID**

(30) Priority: 11.10.2019 JP 2019188042
(71) Applicant: National University Corporation Tokyo Medical and Dental University, Tokyo 113-8510 (JP); Takeda Pharmaceutical Company Limited, Osaka-shi, Osaka 541-0045 (JP)
(72) Inventor: YOKOTA Takanori, Tokyo 113-8510 (JP); NAGATA Tetsuya, Tokyo 113-8510 (JP); YAMADA Hiroki, Tokyo 113-8510 (JP); FURUKAWA Hideki, Fujisawa-shi, Kanagawa 251-0012 (JP); YOGO Takatoshi, Fujisawa-shi, Kanagawa 251-0012 (JP); MIYATA Kenichi, Fujisawa-shi, Kanagawa 251-0012 (JP); UCHIDA Akio, Fujisawa-shi, Kanagawa 251-0012 (JP); TOMITA Naoki, Fujisawa-shi, Kanagawa 251-0012 (JP)
(74) Representative: Jones, Nicholas Andrew
(86) International application number: PCT/JP2020/038380
(87) International publication number: WO 2021/070959

(57) **Abstract**

In an embodiment, an object of the present invention is to provide a double-stranded nucleic acid complex having a novel structure.

In an embodiment, the present invention relates to a nucleic acid complex comprising a first nucleic acid strand and a second nucleic acid strand, wherein said first nucleic acid strand: (1) is capable of hybridizing to at least a part of a target transcriptional product; (2) has an antisense effect on the target transcriptional product; and (3) is a gapmer comprising a central region, and a 5' wing region and a 3' wing region, said second nucleic acid strand comprises at least one sugar-unmodified central region (first exposed region) consisting of one sugar-unmodified ribonucleoside or two or three contiguous sugar-unmodified ribonucleosides linked by an internucleoside bond, which is or are complementary to a part of said first nucleic acid strand, and said first nucleic acid strand is annealed to said second nucleic acid strand.

SEQUENCE LISTING

<110>  National University Corporation Tokyo Medical and Dental
       University
       TAKEDA PHARMACEUTICAL COMPANY LIMITED

<120>  Modified hetero nucleic acids

<130>  PH-8549-PCT

<150>  JP 2019-188042
<151>  2019-10-11

<160>  67

<170>  PatentIn version 3.5

<210>  1
<211>  6983
<212>  DNA
<213>  Mus musculus

<400>  1
caggcattca ggcagcgaga gcagagcagc gtagagcagc acagctgagc tcgtgaggca      60

ggagactcag cccgaggaaa tcgcagataa gtttttaatt aaaaagattg agcagtaaaa     120

agaattagaa ctctaaactt aagctaatag agtagcttat cgaaatatta cttagtctta     180

ataatctaag aagatcttaa gagataacat gaaggcttat ttaaacagtt tgaaaaagga     240

aatgaggaga aaagtatttg tactgtataa tggaggctga ccagagcagt ttaggagatt     300

gtaaagggag gttttgtgaa gttctaaaag gttctagttt gaaggtcggc cttgtagatt     360

aaaacgaagg ttacctaaat agaatctaag tggcatttaa aacagtaaag ttgtagagaa     420

tagtttgaaa atgaggtgta gttttaaaag attgagaaaa gtaggttaag ttgacggccg     480

ttataaaaat ccttcgactg gcgcatgtac gtttgaaggc atgagttgga aacagggaag     540

atggaagtgt taggctagcc gggcgatggt ggcgcacgcc tttaatccta gcacttggga     600

ggcagaggca ggcggatttc tgagttcgag gccagcctgg tctacagagt gagttccagg     660

acagccaggg ctacacagag aaaccctgtc ttgaaaaaac aaaaaggtta ggctagtatt     720

tggagaaaga agattagaaa atggaagtga aagacgaaga agacatacag gaaggtgaag     780

aaaaagctgt tagagaagat aggaaaatag aagacaaagc atctttagaa gacagaaaag     840

gtacttaaag gcacaggtag taggaagccg aagaatagaa gatagaaaga agcaagatag     900

aaaaacaaaa tggaagttaa gacaactttg gatgccagca ttcaagatag gcaaagaaga     960

taagattgag gccaaaaggt tggataagat ataaagtcag aaggaaatta tctttaaagc    1020

cataagttca aatttctgat ggagcgagca gtttagaaga gtctttagac agccacatac    1080

aagattgaag ctagcaatca aagctactag gactgaagta aaaagttaag gcagaatgcc    1140

tttgaagagt tagaagaata ttaaaagcct taacttgtag cttaattttg cttgatgaca    1200

aaaggacttt tgataacagt ttcaagattg tcagcatttt gcattggact tgagctgagg 1260

tgcttttaaa atcctaacga ctagcattgg cagctgaccc aggtctacac agaagtgcat 1320

tcagtgaact aggaagacag gagcggcaga caggagtccc gaagccagtt tggtgaagct 1380

aggaaggact gaggagccag cagcagcagt gcatggtgaa gatagcccag gaaagagtgc 1440

ggttcggtgg aggaagctag gaagaaggag ccatacggat gtggtggtga agctgggaaa 1500

gggttccagg atggtggagc gagagcgagt tggtgatgaa gctagctggc ggcttggctt 1560

gtcaactgcg cggaggaggc gagcaggcat tgtggagagg atagatagcg gctcctagac 1620

cagcatgcca gtgtgcaaga aaggctgcag ggagagcatg cggtgcggta acattccttg 1680

aggtcggcaa catggtggtg gttttctgta acttggatgg taacttgttt actttgtctt 1740

aatagttatg ggggagttgt aggcttctgt gtaaagagat atatctgggg ctgtatgtag 1800

gcctttgcgg gtgttgtagg tttttctttt tcagggttat gtcctcttgc atcttgtcag 1860

aagcttttga gggctgactg ccaaggccca gaaagaagaa tggtagatgg caagttgtct 1920

ttaaccgctc agaggggaat gaatggtaga gccagcacaa cctcccagtt ttgtaagacg 1980

ttgtagtttg aacagatgac ctaccacaag cctcactcct gtgtagggga ggtaattggg 2040

caaagtgctt ttgggggaat gggggcaaaa tatattttga gttcttttcc ccttaggtct 2100

gtctagaatc ctaaaggcag atgactcaag ggaaccagaa aaaaggaaat ccactctcag 2160

gataagcaga gctcgccagg tttacagttt gtaggaagta gaggatggat gctagctttc 2220

acactgagtg tggaggagct ggccatggcg gaattgctgg tagtttactc tttccccctc 2280

ccttaatgag atttgtaaaa tcctaaacac ttttacttga aatatttggg agtggtctta 2340

acagggagga gtgggtgggg gaaacgtttt ttttctaaga ttttccacag atgctatagt 2400

tgtgttgaca cactgggtta gagaaggcgt gtactgctat gctgttggca cgacaccttc 2460

agggactgga gctgcctttt gtccttggaa gagttttccc agttgccgct gaagtcagca 2520

cagtgcggct ttggttcaca gtcacctcag gagaacctca ggagcttggc taggccagag 2580

gttgaagtta agttttacag caccgtgatt taaaatattt cattaaaggg gaggggtaaa 2640

acttagttgg ctgtggcctt gtgtttgggt gggtgggggt gttaggtaat tgtttagttt 2700

atgatttcag ataatcatac cagagaactt aaatatttgg aaaaacagga aatctcagct 2760

ttcaagttgg caagtaactc ccaatccagt tttgcttctt tttttccttt ttcttttttt 2820

gaggcgggca gctaaggaag gttggttcct ctgccggtcc ctcgaaagcg tagggcttgg 2880

gggttggtct ggtccactgg gatgatgtga tgctacagtg gggactcttc tgaagctgtt 2940

ggatgaatat agattgtagt gtgtggttct cttttgaaat ttttttcagg tgacttaatg 3000

tatcttaata actactatag gaacaaagga agtggcttta atgaccctga aggaatttct 3060

tctggtgata gcttttatat tatcaagtaa gagatactat ctcagttttg tataagcaag 3120

tctttttcct agtgtaggag aaatgatttt ccttgtgact aaacaagatg taaaggtatg 3180

ctttttttct tcttgtgcat tgtatacttg tgtttatttg taacttataa tttaagaatt 3240

atgataattc agcctgaatg tcttttagag ggtgggcttt tgttgatgag ggaggggaaa 3300

cctttttttt tctgtagacc tttttcagat aacaccatct gagtcataac cagcctggca 3360

gtgtgatgac gtagatgcag agggagcagc tccttggtga atgagtgata agtaaaggca 3420

gaaaaaataa tgtcatgtct ccatggggaa tgagcatgag ccagagattg ttcctactga 3480

tgaaaagctg catatgcaaa aatttaagca aatgaaagca accagtataa agttatggca 3540

atacctttaa aagttatggc ttatctacca agctttatcc acaaaagtaa agaattgatg 3600

aaaaacagtg aagatcaaat gttcatctca aaactgcttt tacaaaagca gaatagaaat 3660

gaagtgaaaa tgctgcatta agcctggagt aaaaagaagc tgagcttgtt gagatgagtg 3720

ggatcgagcg gctgcgaggc ggtgcagtgt gccaatgttt cgtttgcctc agacaggttt 3780

ctcttcataa gcagaagagt tgcttcattc catctcggag caggaaacag cagactgctg 3840

ttgacagata agtgtaactt ggatctgcag tattgcatgt tagggataga taagtgcctt 3900

ttttctcttt ttccaaaaag acctgtagag ctgttgaatg tttgcagctg gcccctctta 3960

ggcagttcag aattttgagt agttttccca tccagcctct taaaaattcc taagccttgc 4020

accgatgggc tttcatgatg ggatagctaa taggcttttg catcgtaaac ttcaacacaa 4080

aagcctacat gattaatgcc tactttaatt acattgctta caagattaag gaatctttat 4140

cttgaagacc ccatgaaagg gatcattatg tgctgaaaat tagatgttca tattgctaaa 4200

atttaaatgt gctccaatgt acttgtgctt aaaatcatta aattatacaa attaataaaa 4260

tacttcacta gagaatgtat gtatttagaa ggctgtctcc ttatttaaat aaagtcttgt 4320

ttgttgtctg tagttagtgt gggcaatttt ggggggatgt tcttctctaa tcttttcaga 4380

aacttgactt cgaacactta agtggaccag atcaggattt gagccagaag accgaaatta 4440

actttaaggc aggaaagaca aattttattc tccatgcagt gatgagcatt taataattgc 4500

aggcctggca tagaggccgt ctaactaagg actaagtacc ttaggcaggt gggagatgat 4560

ggtcagagta aaaggtaact acatattttg tttccagaaa gtcaggggtc taatttgacc 4620

atggctaaac atctagggta agacactttt cccccacatt tccaaatatg catgttgagt 4680

ttaaatgctt acgatcatct catccacttt agccttttgt cacctcactt gagccacgag 4740

tggggtcagg catgtgggtt taaagagttt tcctttgcag agcctcattt catccttcat 4800

ggagctgctc aggactttgc atataagcgc ttgcctctgt cttctgttct gctagtgagt 4860

gtgtgatgtg agaccttgca gtgagtttgt ttttcctgga atgtggaggg agggggggat 4920

ggggcttact tgttctagct ttttttttac agaccacaca gaatgcaggt gtcttgactt 4980

caggtcatgt ctgttctttg gcaagtaata tgtgcagtac tgttccaatc tgctgctatt 5040

agaatgcatt gtgacgcgac tggagtatga ttaaagaaag ttgtgtttcc ccaagtgttt 5100

ggagtagtgg ttgttggagg aaaagccatg agtaacaggc tgagtgttga ggaaatggct 5160

ctctgcagct ttaagtaacc cgtgtttgtg attggagccg agtccctttg ctgtgctgcc 5220

ttaggtaaat gtttttgttc atttctggtg aggggggttg ggagcactga agcctttagt 5280

ctcttccaga ttcaacttaa aatctgacaa gaaataaatc agacaagcaa cattcttgaa 5340

gaaattttaa ctggcaagtg gaaatgtttt gaacagttcc gtggtcttta gtgcattatc 5400

tttgtgtagg tgttctctct cccctccctt ggtcttaatt cttacatgca ggaacattga 5460

caacagcaga catctatcta ttcaaggggc cagagaatcc agacccagta aggaaaaata 5520

gcccatttac tttaaatcga taagtgaagc agacatgcca ttttcagtgt ggggattggg 5580

aagccctagt tctttcagat gtacttcaga ctgtagaagg agcttccagt tgaattgaaa 5640

ttcaccagtg gacaaaatga ggacaacagg tgaacgagcc ttttcttgtt taagattagc 5700

tactggtaat ctagtgttga atcctctcca gcttcatgct ggagcagcta gcatgtgatg 5760

taatgttggc cttggggtgg aggggtgagg tgggcgctaa gccttttttt aagatttttc 5820

aggtacccct cactaaaggc actgaaggct taatgtagga cagcggagcc ttcctgtgtg 5880

gcaagaatca agcaagcagt attgtatcga gaccaaagtg gtatcatggt cggttttgat 5940

tagcagtggg gactacccta ccgtaacacc ttgttggaat tgaagcatcc aaagaaaata 6000

cttgagaggc cctgggcttg ttttaacatc tggaaaaaag gctgttttta tagcagcggt 6060

taccagccca aacctcaagt tgtgcttgca ggggagggaa aagggggaaa gcgggcaacc 6120

agtttcccca gcttttccag aatcctgtta caaggtctcc ccacaagtga tttctctgcc 6180

acatcgccac catgggcctt tggcctaatc acagaccctt cacccctcac cttgatgcag 6240

ccagtagctg gatccttgag gtcacgttgc atatcggttt caaggtaacc atggtgccaa 6300

ggtcctgtgg gttgcaccag aaaaggccat caattttccc cttgcctgta atttaacatt 6360

aaaaccatag ctaagatgtt ttatacatag cacctatgca gagtaaacaa accagtatgg 6420

gtatagtatg tttgatacca gtgctgggtg ggaatgtagg aagtcggatg aaaagcaagc 6480

ctttgtagga agttgttggg gtgggattgc aaaaattctc tgctaagact ttttcaggtg 6540

gacataacag acttggccaa gctagcatct tagtggaagc agattcgtca gtagggttgt 6600

aaaggttttt cttttcctga gaaaacaacc ttttgttttc tcaggttttg ctttttggcc 6660

tttccctagc tttaaaaaaa aaaaagcaaa agacgctggt ggctggcact cctggtttcc 6720

aggacggggt tcaagtccct gcggtgtctt tgcttgactc ttatatcatg aggccattac 6780

atttttcttg gagggttcta aaggctctgg gtatggtagc tgatatcact ggaacactcc 6840

ccagcctcag tgttgaactc ttgataatta actgcattgt ctttcaggtt atgcccaatt 6900

cgtcttatta cctctgagtc gacacacctc ctactattta ttgaatactt tgattttatg 6960

aaataaaaac taaatatctc tca 6983

<210> 2
<211> 8779
<212> DNA
<213> Homo sapiens

<400> 2
cgcagcctgc agcccgagac ttctgtaaag gactggggcc ccgcaactgg cctctcctgc 60

cctcttaagc gcagcgccat tttagcaacg cagaagcccg gcgccgggaa gcctcagctc 120

gcctgaaggc aggtcccctc tgacgcctcc gggagcccag gtttcccaga gtccttggga 180

cgcagcgacg agttgtgctg ctatcttagc tgtccttata ggctggccat tccaggtggt 240

ggtatttaga taaaaccact caaactctgc agtttggtct tggggtttgg aggaaagctt 300

ttatttttct tcctgctccg gttcagaagg tctgaagctc atacctaacc aggcataaca 360

cagaatctgc aaaacaaaaa cccctaaaaa agcagaccca gagcagtgta aacacttctg 420

ggtgtgtccc tgactggctg cccaaggtct ctgtgtcttc ggagacaaag ccattcgctt 480

agttggtcta ctttaaaagg ccacttgaac tcgctttcca tggcgatttg ccttgtgagc 540

actttcagga gagcctggaa gctgaaaaac ggtagaaaaa tttccgtgcg ggccgtgggg 600

ggctggcggc aactgggggg ccgcagatca gagtgggcca ctggcagcca acggcccccg 660

gggctcaggc ggggagcagc tctgtggtgt gggattgagg cgttttccaa gagtgggttt 720

tcacgtttct aagatttccc aagcagacag cccgtgctgc tccgatttct cgaacaaaaa 780

agcaaaacgt gtggctgtct tgggagcaag tcgcaggact gcaagcagtt gggggagaaa 840

gtccgccatt ttgccacttc tcaaccgtcc ctgcaaggct ggggctcagt tgcgtaatgg 900

aaagtaaagc cctgaactat cacactttaa tcttccttca aaaggtggta aactatacct 960

actgtccctc aagagaacac aagaagtgct ttaagaggta ttttaaaagt tccgggggtt 1020

ttgtgaggtg tttgatgacc cgtttaaaat atgatttcca tgtttctttt gtctaaagtt 1080

tgcagctcaa atctttccac acgctagtaa tttaagtatt tctgcatgtg tagtttgcat 1140

tcaagttcca taagctgtta agaaaaatct agaaaagtaa aactagaacc tatttttaac 1200

cgaagaacta ctttttgcct ccctcacaaa ggcggcggaa ggtgatcgaa ttccggtgat 1260

gcgagttgtt ctccgtctat aaatacgcct cgcccgagct gtgcggtagg cattgaggca 1320

gccagcgcag gggcttctgc tgagggggca ggcggagctt gaggaaaccg cagataagtt 1380

tttttctctt tgaaagatag agattaatac aactacttaa aaaatatagt caataggtta 1440

ctaagatatt gcttagcgtt aagtttttaa cgtaatttta atagcttaag attttaagag 1500

aaaatatgaa gacttagaag agtagcatga ggaaggaaaa gataaaaggt ttctaaaaca 1560

tgacggaggt tgagatgaag cttcttcatg gagtaaaaaa tgtatttaaa agaaaattga 1620

gagaaaggac tacagagccc cgaattaata ccaatagaag ggcaatgctt ttagattaaa 1680

atgaaggtga cttaaacagc ttaaagttta gtttaaaagt tgtaggtgat taaaataatt 1740

tgaaggcgat cttttaaaaa gagattaaac cgaaggtgat taaaagacct tgaaatccat 1800

gacgcaggga gaattgcgtc atttaaagcc tagttaacgc atttactaaa cgcagacgaa 1860

aatggaaaga ttaattggga gtggtaggat gaaacaattt ggagaagata gaagtttgaa 1920

gtggaaaact ggaagacaga agtacgggaa ggcgaagaaa agaatagaga agatagggaa 1980

attagaagat aaaaacatac ttttagaaga aaaaagataa atttaaacct gaaaagtagg 2040

aagcagaaga aaaaagacaa gctaggaaac aaaaagctaa gggcaaaatg tacaaactta 2100

gaagaaaatt ggaagataga aacaagatag aaaatgaaaa tattgtcaag agtttcagat 2160

agaaaatgaa aaacaagcta agacaagtat tggagaagta tagaagatag aaaaatataa 2220

agccaaaaat tggataaaat agcactgaaa aaatgaggaa attattggta accaatttat 2280

tttaaaagcc catcaattta atttctggtg gtgcagaagt tagaaggtaa agcttgagaa 2340

gatgagggtg tttacgtaga ccagaaccaa tttagaagaa tacttgaagc tagaagggga 2400

agttggttaa aaatcacatc aaaaagctac taaaaggact ggtgtaattt aaaaaaaact 2460

aaggcagaag gcttttggaa gagttagaag aatttggaag gccttaaata tagtagctta 2520

gtttgaaaaa tgtgaaggac tttcgtaacg gaagtaattc aagatcaaga gtaattacca 2580

acttaatgtt tttgcattgg actttgagtt aagattattt tttaaatcct gaggactagc 2640

attaattgac agctgaccca ggtgctacac agaagtggat tcagtgaatc taggaagaca 2700

gcagcagaca ggattccagg aaccagtgtt tgatgaagct aggactgagg agcaagcgag 2760

caagcagcag ttcgtggtga agataggaaa agagtccagg agccagtgcg atttggtgaa 2820

ggaagctagg aagaaggaag gagcgctaac gatttggtgg tgaagctagg aaaaaggatt 2880

ccaggaagga gcgagtgcaa tttggtgatg aaggtagcag gcggcttggc ttggcaacca 2940

cacggaggag gcgagcaggc gttgtgcgta gaggatccta gaccagcatg ccagtgtgcc 3000

aaggccacag ggaaagcgag tggttggtaa aaatccgtga ggtcggcaat atgttgtttt 3060

tctggaactt acttatggta accttttatt tattttctaa tataatgggg gagtttcgta 3120

ctgaggtgta aagggattta tatggggacg taggccgatt tccgggtgtt gtaggtttct 3180

ctttttcagg cttatactca tgaatcttgt ctgaagcttt tgagggcaga ctgccaagtc 3240

ctggagaaat agtagatggc aagtttgtgg gttttttttt tttacacgaa tttgaggaaa 3300

accaaatgaa tttgatagcc aaattgagac aatttcagca aatctgtaag cagtttgtat 3360

gtttagttgg ggtaatgaag tatttcagtt ttgtgaatag atgacctgtt tttacttcct 3420

caccctgaat tcgttttgta aatgtagagt ttggatgtgt aactgaggcg ggggggagtt 3480

ttcagtattt ttttttgtgg gggtgggggc aaaatatgtt ttcagttctt tttcccttag 3540

gtctgtctag aatcctaaag gcaaatgact caaggtgtaa cagaaaacaa gaaaatccaa 3600

tatcaggata atcagaccac cacaggttta cagtttatag aaactagagc agttctcacg 3660

ttgaggtctg tggaagagat gtccattgga gaaatggctg gtagttactc ttttttcccc 3720

ccaccccctt aatcagactt taaaagtgct taacccctta aacttgttat tttttacttg 3780

aagcattttg ggatggtctt aacagggaag agagagggtg ggggagaaaa tgtttttttc 3840

taagattttc cacagatgct atagtactat tgacaaactg ggttagagaa ggagtgtacc 3900

gctgtgctgt tggcacgaac accttcaggg actggagctg cttttatcct tggaagagta 3960

ttcccagttg aagctgaaaa gtacagcaca gtgcagcttt ggttcatatt cagtcatctc 4020

aggagaactt cagaagagct tgagtaggcc aaatgttgaa gttaagtttt ccaataatgt 4080

gacttcttaa aagttttatt aaaggggagg ggcaaatatt ggcaattagt tggcagtggc 4140

ctgttacggt tgggattggt ggggtgggtt taggtaattg tttagtttat gattgcagat 4200

aaactcatgc cagagaactt aaagtcttag aatggaaaaa gtaaagaaat atcaacttcc 4260

aagttggcaa gtaactccca atgatttagt ttttttcccc ccagtttgaa ttgggaagct 4320

gggggaagtt aaatatgagc cactgggtgt accagtgcat taatttgggc aaggaaagtg 4380

tcataatttg atactgtatc tgttttcctt caaagtatag agcttttggg gaaggaaagt 4440

attgaactgg gggttggtct ggcctactgg gctgacatta actacaatta tgggaaatgc 4500

aaaagttgtt tggatatggt agtgtgtggt tctcttttgg aatttttttc aggtgattta 4560

ataataattt aaaactacta tagaaactgc agagcaaagg aagtggctta atgatcctga 4620

agggatttct tctgatggta gcttttgtat tatcaagtaa gattctattt tcagttgtgt 4680

gtaagcaagt ttttttttag tgtaggagaa atacttttcc attgtttaac tgcaaaacaa 4740

gatgttaagg tatgcttcaa aaattttgta aattgtttat tttaaactta tctgtttgta 4800

aattgtaact gattaagaat tgtgatagtt cagcttgaat gtctcttaga gggtgggctt 4860

ttgttgatga gggaggggaa actttttttt tttctataga cttttttcag ataacatctt 4920

ctgagtcata accagcctgg cagtatgatg gcctagatgc agagaaaaca gctccttggt 4980

gaattgataa gtaaaggcag aaaagattat atgtcatacc tccattgggg aataagcata 5040

accctgagat tcttactact gatgagaaca ttatctgcat atgccaaaaa attttaagca 5100

aatgaaagct accaatttaa agttacggaa tctaccattt taaagttaat tgcttgtcaa 5160

gctataacca caaaaataat gaattgatga gaaatacaat gaagaggcaa tgtccatctc 5220

aaaatactgc ttttacaaaa gcagaataaa agcgaaaaga aatgaaaatg ttacactaca 5280

ttaatcctgg aataaaagaa gccgaaataa atgagagatg agttgggatc aagtggattg 5340

aggaggctgt gctgtgtgcc aatgtttcgt ttgcctcaga caggtatctc ttcgttatca 5400

gaagagttgc ttcatttcat ctgggagcag aaaacagcag gcagctgtta acagataagt 5460

ttaacttgca tctgcagtat tgcatgttag ggataagtgc ttatttttaa gagctgtgga 5520

gttcttaaat atcaaccatg gcactttctc ctgacccctt ccctagggga tttcaggatt 5580

gagaaatttt tccatcgagc ctttttaaaa ttgtaggact tgttcctgtg ggcttcagtg 5640

atgggatagt acacttcact cagaggcatt tgcatcttta aataatttct taaaagcctc 5700

taaagtgatc agtgccttga tgccaactaa ggaaatttgt ttagcattga atctctgaag 5760

gctctatgaa aggaatagca tgatgtgctg ttagaatcag atgttactgc taaaatttac 5820

atgttgtgat gtaaattgtg tagaaaacca ttaaatcatt caaaataata aactattttt 5880

attagagaat gtatactttt agaaagctgt ctccttattt aaataaaata gtgtttgtct 5940

gtagttcagt gttggggcaa tcttgggggg gattcttctc taatctttca gaaactttgt 6000

ctgcgaacac tctttaatgg accagatcag gatttgagcg gaagaacgaa tgtaacttta 6060

aggcaggaaa gacaaatttt attcttcata aagtgatgag catataataa ttccaggcac 6120

atggcaatag aggccctcta aataaggaat aaataacctc ttagacaggt gggagattat 6180

gatcagagta aaaggtaatt acacatttta tttccagaaa gtcaggggtc tataaattga 6240

cagtgattag agtaatactt tttcacattt ccaaagtttg catgttaact ttaaatgctt 6300

acaatcttag agtggtaggc aatgttttac actattgacc ttatataggg aagggagggg 6360

gtgcctgtgg ggttttaaag aatttccttt tgcagaggca tttcatcctt catgaagcca 6420

ttcaggattt tgaattgcat atgagtgctt ggctcttcct tctgttctag tgagtgtatg 6480

agaccttgca gtgagtttat cagcatactc aaaatttttt tcctggaatt tggagggatg 6540

ggaggagggg gtggggctta cttgttgtag cttttttttt ttttacagac ttcacagaga 6600

atgcagttgt cttgacttca ggtctgtctg ttctgttggc aagtaaatgc agtactgttc 6660

tgatcccgct gctattagaa tgcattgtga aacgactgga gtatgattaa aagttgtgtt 6720

ccccaatgct tggagtagtg attgttgaag gaaaaaatcc agctgagtga taaaggctga 6780

gtgttgagga aatttctgca gttttaagca gtcgtatttg tgattgaagc tgagtacatt 6840

ttgctggtgt atttttaggt aaaatgcttt ttgttcattt ctggtggtgg gaggggactg 6900

aagcctttag tcttttccag atgcaacctt aaaatcagtg acaagaaaca ttccaaacaa 6960

gcaacagtct tcaagaaatt aaactggcaa gtggaaatgt ttaaacagtt cagtgatctt 7020

tagtgcattg tttatgtgtg ggtttctctc tcccctccct tggtcttaat tcttacatgc 7080

aggaacactc agcagacaca cgtatgcgaa gggccagaga agccagaccc agtaagaaaa 7140

aatagcctat ttactttaaa taaaccaaac attccatttt aaatgtgggg attgggaacc 7200

actagttctt tcagatggta ttcttcagac tatagaagga gcttccagtt gaattcacca 7260

gtggacaaaa tgaggaaaac aggtgaacaa gctttttctg tatttacata caaagtcaga 7320

tcagttatgg gacaatagta ttgaatagat ttcagcttta tgctggagta actggcatgt 7380

gagcaaactg tgttggcgtg ggggtggagg ggtgaggtgg gcgctaagcc tttttttaag 7440

atttttcagg tacccctcac taaaggcacc gaaggcttaa agtaggacaa ccatggagcc 7500

ttcctgtggc aggagagaca acaaagcgct attatcctaa ggtcaagaga agtgtcagcc 7560

tcacctgatt tttattagta atgaggactt gcctcaactc cctctttctg gagtgaagca 7620

tccgaaggaa tgcttgaagt acccctgggc ttctcttaac atttaagcaa gctgttttta 7680

tagcagctct taataataaa gcccaaatct caagcggtgc ttgaagggga gggaaagggg 7740

gaaagcgggc aaccactttt ccctagcttt tccagaagcc tgttaaaagc aaggtctccc 7800

cacaagcaac ttctctgcca catcgccacc ccgtgccttt tgatctagca cagacccttc 7860

acccctcacc tcgatgcagc cagtagcttg gatccttgtg ggcatgatcc ataatcggtt 7920

tcaaggtaac gatggtgtcg aggtctttgg tgggttgaac tatgttagaa aaggccatta 7980

atttgcctgc aaattgttaa cagaagggta ttaaaaccac agctaagtag ctctattata 8040

atacttatcc agtgactaaa accaacttaa accagtaagt ggagaaataa catgttcaag 8100

aactgtaatg ctgggtggga acatgtaact tgtagactgg agaagatagg catttgagtg 8160

gctgagaggg cttttgggtg ggaatgcaaa aattctctgc taagactttt tcaggtgaac 8220

ataacagact tggccaagct agcatcttag cggaagctga tctccaatgc tcttcagtag 8280

ggtcatgaag gtttttcttt tcctgagaaa acaacacgta ttgttttctc aggttttgct 8340

ttttggcctt tttctagctt aaaaaaaaaa aaagcaaaag atgctggtgg ttggcactcc 8400

tggtttccag gacggggttc aaatccctgc ggcgtctttg ctttgactac taatctgtct 8460

tcaggactct ttctgtattt ctccttttct ctgcaggtgc tagttcttgg agttttgggg 8520

aggtgggagg taacagcaca atatctttga actatataca tccttgatgt ataatttgtc 8580

aggagcttga cttgattgta tattcatatt tacacgagaa cctaatataa ctgccttgtc 8640

tttttcaggt aatagcctgc agctggtgtt ttgagaagcc ctactgctga aaacttaaca 8700

attttgtgta ataaaaatgg agaagctcta aattgttgtg gttcttttgt gaataaaaaa 8760

atcttgattg ggaaaaaa 8779

<210> 3
<211> 16
<212> DNA
<213> Artificial

<220>
<223> synthetic

<220>
<221>  misc_feature
<222>  (1)..(3)
<223>  LNA

<220>
<221>  misc_feature
<222>  (14)..(16)
<223>  LNA

<400>  3
ctagttcact gaatgc                                                    16


<210>  4
<211>  16
<212>  RNA
<213>  Artificial

<220>
<223>  synthetic

<220>
<221>  misc_feature
<222>  (1)..(16)
<223>  2'-O-Me RNA

<400>  4
gcauucagug aacuag                                                    16


<210>  5
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  5
gcauucagug aacuag                                                    16


<210>  6
<211>  16
<212>  RNA
<213>  Artificial

<220>
<223>  synthetic

<220>
<221>  misc_feature

<222>  (1)..(3)
<223>  2'-O-Me RNA

<220>
<221>  misc_feature
<222>  (7)..(16)
<223>  2'-O-Me RNA

<400>  6
gcauucagug aacuag                                                    16


<210>  7
<211>  16
<212>  RNA
<213>  Artificial

<220>
<223>  synthetic

<220>
<221>  misc_feature
<222>  (1)..(4)
<223>  2'-O-Me RNA

<220>
<221>  misc_feature
<222>  (8)..(16)
<223>  2'-O-Me RNA

<400>  7
gcauucagug aacuag                                                    16


<210>  8
<211>  16
<212>  RNA
<213>  Artificial

<220>
<223>  synthetic

<220>
<221>  misc_feature
<222>  (1)..(5)
<223>  2'-O-Me RNA

<220>
<221>  misc_feature
<222>  (9)..(16)
<223>  2'-O-Me RNA

<400>  8
gcauucagug aacuag                                                    16


<210>  9
<211>  16
<212>  RNA
<213>  Artificial

<220>
<223>  synthetic

<220>
<221>  misc_feature
<222>  (1)..(6)
<223>  2'-O-Me RNA

<220>
<221>  misc_feature
<222>  (10)..(16)
<223>  2'-O-Me RNA

<400>  9
gcauucagug aacuag                                                    16


<210>  10
<211>  16
<212>  RNA
<213>  Artificial

<220>
<223>  synthetic

<220>
<221>  misc_feature
<222>  (1)..(7)
<223>  2'-O-Me RNA

<220>
<221>  misc_feature
<222>  (11)..(16)
<223>  2'-O-Me RNA

<400>  10
gcauucagug aacuag                                                    16


<210>  11
<211>  16
<212>  RNA
<213>  Artificial

<220>
<223>  synthetic

<220>
<221>  misc_feature
<222>  (1)..(8)
<223>  2'-O-Me RNA

<220>
<221>  misc_feature
<222>  (12)..(16)
<223>  2'-O-Me RNA

<400>  11
gcauucagug aacuag                                                    16


<210>  12
<211>  16
<212>  RNA
<213>  Artificial

<220>
<223>  synthetic

<220>
<221>  misc_feature
<222>  (1)..(9)
<223>  2'-O-Me RNA

<220>
<221>  misc_feature
<222>  (13)..(16)
<223>  2'-O-Me RNA

<400>  12
gcauucagug aacuag                                                    16


<210>  13
<211>  16
<212>  RNA
<213>  Artificial

<220>
<223>  synthetic

<220>
<221>  misc_feature
<222>  (1)..(10)
<223>  2'-O-Me RNA

<220>
<221>  misc_feature
<222>  (14)..(16)
<223>  2'-O-Me RNA

<400>  13
gcauucagug aacuag                                                    16


<210>  14
<211>  25
<212>  DNA
<213>  Artificial

<220>
<223>  probe

<400>  14
tgttggcacg acaccttcag ggact                                          25


<210>  15
<211>  23
<212>  DNA
<213>  Artificial

<220>
<223>  primer

<400>  15
tgggttagag aaggcgtgta ctg                                            23

<210>  16
<211>  18
<212>  DNA
<213>  Artificial

<220>
<223>  primer

<400>  16
tcagcggcaa ctgggaaa                                                    18


<210>  17
<211>  24
<212>  DNA
<213>  Artificial

<220>
<223>  probe

<400>  17
cacacccgcc accagttcgc catg                                             24


<210>  18
<211>  19
<212>  DNA
<213>  Artificial

<220>
<223>  primer

<400>  18
cgcgagcaca gcttctttg                                                   19


<210>  19
<211>  17
<212>  DNA
<213>  Artificial

<220>
<223>  primer

<400>  19
atgccggagc cgttgtc                                                     17


<210>  20
<211>  16
<212>  RNA
<213>  Artificial

<220>
<223>  synthetic

<220>
<221>  misc_feature
<222>  (1)..(3)
<223>  2'-O-Me RNA

<220>

<221>  misc_feature
<222>  (6)..(16)
<223>  2'-O-Me RNA

<400>  20
gcauucagug aacuag                                                    16


<210>  21
<211>  16
<212>  RNA
<213>  Artificial

<220>
<223>  synthetic

<220>
<221>  misc_feature
<222>  (1)..(4)
<223>  2'-O-Me RNA

<220>
<221>  misc_feature
<222>  (7)..(16)
<223>  2'-O-Me RNA

<400>  21
gcauucagug aacuag                                                    16


<210>  22
<211>  16
<212>  RNA
<213>  Artificial

<220>
<223>  synthetic

<220>
<221>  misc_feature
<222>  (1)..(5)
<223>  2'-O-Me RNA

<220>
<221>  misc_feature
<222>  (8)..(16)
<223>  2'-O-Me RNA

<400>  22
gcauucagug aacuag                                                    16


<210>  23
<211>  16
<212>  RNA
<213>  Artificial

<220>
<223>  synthetic

<220>
<221>  misc_feature
<222>  (1)..(6)

<223>  2'-O-Me RNA

<220>
<221>  misc_feature
<222>  (9)..(16)
<223>  2'-O-Me RNA

<400>  23
gcauucagug aacuag                                                        16


<210>  24
<211>  16
<212>  RNA
<213>  Artificial

<220>
<223>  synthetic

<220>
<221>  misc_feature
<222>  (1)..(7)
<223>  2'-O-Me RNA

<220>
<221>  misc_feature
<222>  (10)..(16)
<223>  2'-O-Me RNA

<400>  24
gcauucagug aacuag                                                        16


<210>  25
<211>  16
<212>  RNA
<213>  Artificial

<220>
<223>  synthetic

<220>
<221>  misc_feature
<222>  (1)..(8)
<223>  2'-O-Me RNA

<220>
<221>  misc_feature
<222>  (11)..(16)
<223>  2'-O-Me RNA

<400>  25
gcauucagug aacuag                                                        16


<210>  26
<211>  16
<212>  RNA
<213>  Artificial

<220>
<223>  synthetic

<220>
<221>  misc_feature
<222>  (1)..(9)
<223>  2'-O-Me RNA

<220>
<221>  misc_feature
<222>  (12)..(16)
<223>  2'-O-Me RNA

<400>  26
gcauucagug aacuag                                                        16


<210>  27
<211>  16
<212>  RNA
<213>  Artificial

<220>
<223>  synthetic

<220>
<221>  misc_feature
<222>  (1)..(10)
<223>  2'-O-Me RNA

<220>
<221>  misc_feature
<222>  (13)..(16)
<223>  2'-O-Me RNA

<400>  27
gcauucagug aacuag                                                        16


<210>  28
<211>  16
<212>  RNA
<213>  Artificial

<220>
<223>  synthetic

<220>
<221>  misc_feature
<222>  (1)..(11)
<223>  2'-O-Me RNA

<220>
<221>  misc_feature
<222>  (14)..(16)
<223>  2'-O-Me RNA

<400>  28
gcauucagug aacuag                                                        16


<210>  29
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (5)..(16)
<223>  2'-O-Me RNA

<400>  29
gcauucagug aacuag                                                        16


<210>  30
<211>  16
<212>  RNA
<213>  Artificial

<220>
<223>  synthetic

<220>
<221>  misc_feature
<222>  (1)..(4)
<223>  2'-O-Me RNA

<220>
<221>  misc_feature
<222>  (6)..(16)
<223>  2'-O-Me RNA

<400>  30
gcauucagug aacuag                                                        16


<210>  31
<211>  16
<212>  RNA
<213>  Artificial

<220>
<223>  synthetic

<220>
<221>  misc_feature
<222>  (1)..(5)
<223>  2'-O-Me RNA

<220>
<221>  misc_feature
<222>  (7)..(16)
<223>  2'-O-Me RNA

<400>  31
gcauucagug aacuag                                                        16


<210>  32
<211>  16

<212>  RNA
<213>  Artificial

<220>
<223>  synthetic

<220>
<221>  misc_feature
<222>  (1)..(6)
<223>  2'-O-Me RNA

<220>
<221>  misc_feature
<222>  (8)..(16)
<223>  2'-O-Me RNA

<400>  32
gcauucagug aacuag                                                       16


<210>  33
<211>  16
<212>  RNA
<213>  Artificial

<220>
<223>  synthetic

<220>
<221>  misc_feature
<222>  (1)..(7)
<223>  2'-O-Me RNA

<220>
<221>  misc_feature
<222>  (9)..(16)
<223>  2'-O-Me RNA

<400>  33
gcauucagug aacuag                                                       16


<210>  34
<211>  16
<212>  RNA
<213>  Artificial

<220>
<223>  synthetic

<220>
<221>  misc_feature
<222>  (1)..(8)
<223>  2'-O-Me RNA

<220>
<221>  misc_feature
<222>  (10)..(16)
<223>  2'-O-Me RNA

<400>  34
gcauucagug aacuag                                                       16

<210>  35
<211>  16
<212>  RNA
<213>  Artificial

<220>
<223>  synthetic

<220>
<221>  misc_feature
<222>  (1)..(9)
<223>  2'-O-Me RNA

<220>
<221>  misc_feature
<222>  (11)..(16)
<223>  2'-O-Me RNA

<400>  35
gcauucagug aacuag                                                    16


<210>  36
<211>  16
<212>  RNA
<213>  Artificial

<220>
<223>  synthetic

<220>
<221>  misc_feature
<222>  (1)..(10)
<223>  2'-O-Me RNA

<220>
<221>  misc_feature
<222>  (12)..(16)
<223>  2'-O-Me RNA

<400>  36
gcauucagug aacuag                                                    16


<210>  37
<211>  16
<212>  RNA
<213>  Artificial

<220>
<223>  synthetic

<220>
<221>  misc_feature
<222>  (1)..(11)
<223>  2'-O-Me RNA

<220>
<221>  misc_feature
<222>  (13)..(16)
<223>  2'-O-Me RNA

<400>  37
gcauucagug aacuag                                                        16


<210>  38
<211>  16
<212>  RNA
<213>  Artificial

<220>
<223>  synthetic

<220>
<221>  misc_feature
<222>  (1)..(12)
<223>  2'-O-Me RNA

<220>
<221>  misc_feature
<222>  (14)..(16)
<223>  2'-O-Me RNA

<400>  38
gcauucagug aacuag                                                        16


<210>  39
<211>  2534
<212>  DNA
<213>  Mus musculus

<220>
<223>  SR-B1 mRNA

<400>  39
ggaatcccgc gccgaactcg ggggcgggct gcccgggcca tggcgcataa agcctctggc        60

cacctgcagg gctactgctg ctccggccac cgccaggcac acaccttgct gctgagggag       120

tctcggcttc tgtcatctct gtggcctccg tcacctctgt ctccgtctcc ttcaggtcct       180

gagccccgag agccccttcc gcgcacgcgg acatgggcgg cagctccagg gcgcgctggg       240

tggccttggg gttgggcgcc ctggggctgc tgtttgctgc gctcggcgtt gtcatgatcc       300

tcatggtgcc ctccctcatc aagcagcagg tgctcaagaa tgtccgcata gacccgagca       360

gcctgtcctt cgggatgtgg aaggagatcc ccgtcccttt ctacttgtct gtctacttct       420

tcgaagtggt caacccaaac gaggtcctca acggccagaa gccagtagtc cgggagcgtg       480

gaccctatgt ctacagggag ttcagacaaa aggtcaacat caccttcaat gacaacgaca       540

ccgtgtcctt cgtggagaac cgcagcctcc atttccagcc tgacaagtcg catggctcag       600

agagtgacta cattgtactg cctaacatct tggtcctggg gggctcgata ttgatggaga       660

gcaagcctgt gagcctgaag ctgatgatga ccttggcgct ggtcaccatg ggccagcgtg       720

cttttatgaa ccgcacagtt ggtgagatcc tgtggggcta tgacgatccc ttcgtgcatt       780

ttctcaacac gtacctccca gacatgcttc ccataaaggg caaatttggc ctgtttgttg       840

ggatgaacaa ctcgaattct ggggtcttca ctgtcttcac gggcgtccag aatttcagca 900

ggatccatct ggtggacaaa tggaacggac tcagcaagat cgattattgg cattcagagc 960

agtgtaacat gatcaatggg acttccgggc agatgtgggc acccttcatg acacccgaat 1020

cctcgctgga attcttcagc ccggaggcat gcaggtccat gaagctgacc tacaacgaat 1080

caagggtgtt tgaaggcatt cccacgtatc gcttcacggc ccccgatact ctgtttgcca 1140

acgggtccgt ctacccaccc aacgaaggct tctgcccatg ccgagagtct ggcattcaga 1200

atgtcagcac ctgcaggttt ggtgcgcctc tgtttctctc ccacccccac ttttacaacg 1260

ccgaccctgt gttgtcagaa gctgttcttg gtctgaaccc taacccaaag gagcattcct 1320

tgttcctaga catccatccg gtcactggga tccccatgaa ctgttctgtg aagatgcagc 1380

tgagcctcta catcaaatct gtcaagggca tcgggcaaac agggaagatc gagccagtag 1440

ttctgccgtt gctgtggttc gaacagagcg gagcaatggg tggcaagccc ctgagcacgt 1500

tctacacgca gctggtgctg atgccccagg ttcttcacta cgcgcagtat gtgctgctgg 1560

ggcttggagg cctcctgttg ctggtgccca tcatctgcca actgcgcagc caggagaaat 1620

gctttttgtt ttggagtggt agtaaaaagg gctcccagga taaggaggcc attcaggcct 1680

actctgagtc cctgatgtca ccagctgcca agggcacggt gctgcaagaa gccaagctat 1740

agggtcctga agacactata agccccccaa acctgatagc ttggtcagac cagccaccca 1800

gtccctacac cccgcttctt gaggactctc tcagcggaca gcccaccagt gccatggcct 1860

gagcccccag atgtcacacc tgtccgcacg cacggcacat ggatgcccac gcatgtgcaa 1920

aaacaactca gggaccaggg acagacctgc tgccaagtga gcctgatggg ccacaggtgt 1980

gctcttctaa atggcctgtg agccaggctg tgggaactct agctgctgtc agcccctcct 2040

gtaggagctg gccctgccca ggctcctgac ttccctcagg aagtctttct gtctttctcc 2100

atcagtctga aagccttagt tcccacagag gacggatctg tcactcctag gggctgggca 2160

tatgtcggcc tcttgtgcca aggccaggca agcagctcca ggtcctgacc agtttgcaca 2220

cacactctgg agctgtatct ggcgcttttt ctatcgtctc tgctatgtca ctgaattaac 2280

cactgtacgt ggcagaggtg gcaggcccct cagggtcctt atttttcagg catggggtca 2340

aagctagagg tatgggccgt ctacaccccc ccgccccccg gcatctagtg tacctcacca 2400

gagggtattc ggaggcccag catcctgcaa ccgacccctt ttttctactg gaagagaaat 2460

tttatcatct tttgaaagga agtcatgact gaagcaataa accttttcac tgattcaaca 2520

aaaaaaaaaa aaaa 2534

<210> 40
<211> 2759
<212> DNA
<213> Homo sapiens

<220>
<223>  SR-B1 mRNA

<400>  40
gctcaggccc cgcccctgcc gccggaatcc tgaagcccaa ggctgcccgg gggcggtccg      60

gcggcgccgg cgatggggca taaaaccact ggccacctgc cgggctgctc ctgcgtgcgc     120

tgccgtcccg gatccaccgt gcctctgcgg cctgcgtgcc cggagtcccc gcctgtgtcg     180

tctctgtcgc cgtccccgtc tcctgccagg cgcggagccc tgcgagccgc gggtgggccc     240

caggcgcgca gacatgggct gctccgccaa agcgcgctgg gctgccgggg cgctgggcgt     300

cgcggggcta ctgtgcgctg tgctgggcgc tgtcatgatc gtgatggtgc cgtcgctcat     360

caagcagcag gtccttaaga acgtgcgcat cgaccccagt agcctgtcct tcaacatgtg     420

gaaggagatc cctatcccct tctatctctc cgtctacttc tttgacgtca tgaaccccag     480

cgagatcctg aagggcgaga agccgcaggt gcgggagcgc gggccctacg tgtacaggga     540

gttcaggcac aaaagcaaca tcaccttcaa caacaacgac accgtgtcct tcctcgagta     600

ccgcaccttc cagttccagc cctccaagtc ccacggctcg gagagcgact acatcgtcat     660

gcccaacatc ctggtcttgg gtgcggcggt gatgatggag aataagccca tgaccctgaa     720

gctcatcatg accttggcat tcaccaccct cggcgaacgt gccttcatga accgcactgt     780

gggtgagatc atgtggggct acaaggaccc ccttgtgaat ctcatcaaca agtactttcc     840

aggcatgttc cccttcaagg acaagttcgg attatttgct gagctcaaca actccgactc     900

tgggctcttc acggtgttca cgggggtcca gaacatcagc aggatccacc tcgtggacaa     960

gtggaacggg ctgagcaagg ttgacttctg gcattccgat cagtgcaaca tgatcaatgg    1020

aacttctggg caaatgtggc cgcccttcat gactcctgag tcctcgctgg agttctacag    1080

cccggaggcc tgccgatcca tgaagctaat gtacaaggag tcaggggtgt ttgaaggcat    1140

ccccacctat cgcttcgtgg ctcccaaaac cctgtttgcc aacgggtcca tctacccacc    1200

caacgaaggc ttctgcccgt gcctggagtc tggaattcag aacgtcagca cctgcaggtt    1260

cagtgccccc ttgtttctct cccatcctca cttcctcaac gctgacccgg ttctggcaga    1320

agcggtgact ggcctgcacc ctaaccagga ggcacactcc ttgttcctgg acatccaccc    1380

ggtcacggga atccccatga actgctctgt gaaactgcag ctgagcctct acatgaaatc    1440

tgtcgcaggc attggacaaa ctgggaagat tgagcctgtg gtcctgccgc tgctctggtt    1500

tgcagagagc ggggccatgg agggggagac tcttcacaca ttctacactc agctggtgtt    1560

gatgcccaag gtgatgcact atgcccagta cgtcctcctg gcgctgggct gcgtcctgct    1620

gctggtccct gtcatctgcc aaatccggag ccaagagaaa tgctatttat tttggagtag    1680

tagtaaaaag ggctcaaagg ataaggaggc cattcaggcc tattctgaat ccctgatgac    1740

atcagctccc aagggctctg tgctgcagga agcaaaactg tagggtcctg aggacaccgt 1800

gagccagcca ggcctggccg ctgggcctga ccggcccccc agcccctaca ccccgcttct 1860

cccggactct cccagcggac agccccccag ccccacagcc tgagcctccc agctgccatg 1920

tgcctgttgc acacctgcac acacgccctg gcacacatac acacatgcgt gcaggcttgt 1980

gcagacactc agggatggag ctgctgctga agggacttgt agggagaggc tcgtcaacaa 2040

gcactgttct ggaaccttct ctccacgtgg cccacaggcc tgaccacagg ggctgtgggt 2100

cctgcgtccc cttcctcggg tgagcctggc ctgtcccgtt cagccgttgg gcccaggctt 2160

cctcccctcc aaggtgaaac actgcagtcc cggtgtggtg gctccccatg caggacgggc 2220

caggctggga gtgccgcctt cctgtgccaa attcagtggg gactcagtgc ccaggccctg 2280

gccacgagct ttggccttgg tctacctgcc aggccaggca aagcgccttt acacaggcct 2340

cggaaaacaa tggagtgagc acaagatgcc ctgtgcagct gcccgagggt ctccgcccac 2400

cccggccgga ctttgatccc cccgaagtct tcacaggcac tgcatcgggt tgtctggcgc 2460

ccttttcctc cagcctaaac tgacatcatc ctatggactg agccggccac tctctggccg 2520

aagtggccgc aggctgtgcc cccgagctgc ccccaccccc tcacagggtc cctcagatta 2580

taggtgccca ggctgaggtg aagaggcctg gggccctgcc cttccgggcg ctcctggacc 2640

ctggggcaaa cctgtgaccc ttttctactg gaatagaaat gagttttatc atctttgaaa 2700

aataattcac tcttgaagta ataaacgttt aaaaaaatgg gaaaaaaaaa aaaaaaaaa 2759

<210> 41
<211> 2761
<212> DNA
<213> Mus musculus

<220>
<223> DMPK mRNA

<400> 41
gaactggcca gagagaccca agggatagtc agggacgggc agacatgcag ctagggttct 60

ggggcctgga caggggcagc caggccctgt gacgggaaga ccccgagctc cggcccgggg 120

aggggccatg gtgttgcctg cccaacatgt cagccgaagt gcggctgagg cagctccagc 180

agctggtgct ggacccaggc ttcctgggac tggagcccct gctcgacctt ctcctgggcg 240

tccaccagga gctgggtgcc tctcacctag cccaggacaa gtatgtggcc gacttcttgc 300

agtgggtgga gcccattgca gcaaggctta aggaggtccg actgcagagg gatgattttg 360

agattttgaa ggtgatcggg cgtggggcgt tcagcgaggt agcggtggtg aagatgaaac 420

agacgggcca agtgtatgcc atgaagatta tgaataagtg ggacatgctg aagagaggcg 480

aggtgtcgtg cttccgggaa gaaagggatg tattagtgaa aggggaccgg cgctggatca 540

cacagctgca ctttgccttc caggatgaga actacctgta cctggtcatg gaatactacg 600

tgggcgggga cctgctaacg ctgctgagca agtttgggga gcggatcccc gccgagatgg 660

ctcgcttcta cctggccgag attgtcatgg ccatagactc cgtgcaccgg ctgggctacg 720

tgcacaggga catcaaacca gataacattc tgctggaccg atgtgggcac attcgcctgg 780

cagacttcgg ctcctgcctc aaactgcagc ctgatggaat ggtgaggtcg ctggtggctg 840

tgggcacccc ggactacctg tctcctgaga ttctgcaggc cgttggtgga gggcctgggg 900

caggcagcta cgggccagag tgtgactggt gggcactggg cgtgttcgcc tatgagatgt 960

tctatgggca gacccccttc tacgcggact ccacagccga gacatatgcc aagattgtgc 1020

actacaggga acacttgtcg ctgccgctgg cagacacagt tgtccccgag gaagctcagg 1080

acctcattcg tgggctgctg tgtcctgctg agataaggct aggtcgaggt ggggcaggtg 1140

atttccagaa acatcctttc ttctttggcc ttgattggga gggtctccga gacagtgtac 1200

ccccctttac accagacttc gagggtgcca cggacacatg caatttcgat gtggtggagg 1260

accggctcac tgccatggtg agcgggggcg gggagacgct gtcagacatg caggaagaca 1320

tgccccttgg ggtgcgcctg cccttcgtgg gctactccta ctgctgcatg gccttcagag 1380

acaatcaggt cccggacccc acccctatgg aactagaggc cctgcagttg cctgtgtcag 1440

acttgcaagg gcttgacttg cagcccccag tgtccccacc ggatcaagtg gctgaagagg 1500

ctgacctagt ggctgtccct gcccctgtgg ctgaggcaga gaccacggta acgctgcagc 1560

agctccagga agccctggaa gaagaggttc tcacccggca gagcctgagc cgcgagctgg 1620

aggccatccg gaccgccaac cagaacttct ccagccaact acaggaggcc gaggtccgaa 1680

accgagacct ggaggcgcat gttcggcagc tacaggaacg gatggagatg ctgcaggccc 1740

caggagccgc agccatcacg gggtccccca gtccccgggc cacggatcca ccttcccatc 1800

tagatggccc cccggccgtg gctgtgggcc agtgcccgct ggtggggcca ggccccatgc 1860

accgccgtca cctgctgctc cctgccagga tccctaggcc tggcctatcc gaggcgcgtt 1920

gcctgctcct gttcgccgct gctctggctg ctgccgccac actgggctgc actgggttgg 1980

tggcctatac cggcggtctc accccagtct ggtgtttccc gggagccacc ttcgccccct 2040

gaaccctaag actccaagcc atctttcatt taggcctcct aggaaggtcg agcgaccagg 2100

gagcgaccca aagcgtctct gtgcccatcg cgcccccccc ccccccccac cgctccgctc 2160

cacacttctg tgagcctggg tccccaccca gctccgctcc tgtgatccag gcctgccacc 2220

tggcggccgg ggagggagga acagggctcg tgcccagcac ccctggttcc tgcagagctg 2280

gtagccaccg ctgctgcagc agctgggcat tcgccgacct tgctttactc agccccgacg 2340

tggatgggca aactgctcag ctcatccgat ttcacttttt cactctccca gccatcagtt 2400

acaagccata agcatgagcc ccctatttcc agggacatcc cattcccata gtgatggatc 2460

agcaagacct ctgccagcac acacggagtc tttggcttcg gacagcctca ctcctggggg 2520

ttgctgcaac tccttccccg tgtacacgtc tgcactctaa caacggagcc acagctgcac 2580

tcccccctcc cccaaagcag tgtgggtatt tattgatctt gttatctgac tcactgacag 2640

actccgggac ccacgtttta gatgcattga gactcgacat tcctcggtat ttattgtctg 2700

tccccaccta cgacctccac tcccgaccct tgcgaataaa atacttctgg tctgccctaa 2760

a 2761

<210> 42
<211> 3243
<212> DNA
<213> Homo sapiens

<220>
<223> DMPK mRNA

<400> 42
gccacaagcc tccaccccag ctggtccccc acccaggctg cccagtttaa cattcctagt 60

cataggacct tgacttctga gaggcctgat tgtcatctgt aaataagggg taggactaaa 120

gcactcctcc tggaggactg agagatgggc tggaccggag cacttgagtc tgggatatgt 180

gaccatgcta cctttgtctc cctgtcctgt tccttccccc agccccaaat ccagggtttt 240

ccaaagtgtg gttcaagaac cacctgcatc tgaatctaga ggtactggat acaaccccac 300

gtctgggccg ttacccagga cattctacat gagaacgtgg gggtggggcc ctggctgcac 360

ctgaactgtc acctggagtc agggtggaag gtggaagaac tgggtcttat ttccttctcc 420

ccttgttctt tagggtctgt ccttctgcag actccgttac cccaccctaa ccatcctgca 480

cacccttgga gccctctggg ccaatgccct gtcccgcaaa gggcttctca ggcatctcac 540

ctctatggga gggcattttt ggcccccaga accttacacg gtgtttatgt ggggaagccc 600

ctgggaagca gacagtccta gggtgaagct gagaggcaga gagaagggga gacagacaga 660

gggtggggct ttcccccttg tctccagtgc cctttctggt gaccctcggt tcttttcccc 720

caccaccccc ccagcggagc ccatcgtggt gaggcttaag gaggtccgac tgcagaggga 780

cgacttcgag attctgaagg tgatcggacg cggggcgttc agcgaggtag cggtagtgaa 840

gatgaagcag acgggccagg tgtatgccat gaagatcatg aacaagtggg acatgctgaa 900

gaggggcgag gtgtcgtgct tccgtgagga gagggacgtg ttggtgaatg gggaccggcg 960

gtggatcacg cagctgcact tcgccttcca ggatgagaac tacctgtacc tggtcatgga 1020

gtattacgtg ggcggggacc tgctgacact gctgagcaag tttggggagc ggattccggc 1080

cgagatggcg cgcttctacc tggcggagat tgtcatggcc atagactcgg tgcaccggct 1140

tggctacgtg cacagggaca tcaaacccga caacatcctg ctggaccgct gtggccacat 1200

ccgcctggcc gacttcggct cttgcctcaa gctgcgggca gatggaacgg tgcggtcgct 1260

ggtggctgtg ggcaccccag actacctgtc ccccgagatc ctgcaggctg tgggcggtgg 1320

gcctgggaca ggcagctacg ggcccgagtg tgactggtgg gcgctgggtg tattcgccta 1380

tgaaatgttc tatgggcaga cgcccttcta cgcggattcc acggcggaga cctatggcaa 1440

gatcgtccac tacaaggagc acctctctct gccgctggtg gacgaagggg tccctgagga 1500

ggctcgagac ttcattcagc ggttgctgtg tcccccggag acacggctgg gccggggtgg 1560

agcaggcgac ttccggacac atcccttctt ctttggcctc gactgggatg gtctccggga 1620

cagcgtgccc ccctttacac cggatttcga aggtgccacc gacacatgca acttcgactt 1680

ggtggaggac gggctcactg ccatggtgag cgggggcggg gagacactgt cggacattcg 1740

ggaaggtgcg ccgctagggg tccacctgcc ttttgtgggc tactcctact cctgcatggc 1800

cctcagggac agtgaggtcc caggccccac acccatggaa ctggaggccg agcagctgct 1860

tgagccacac gtgcaagcgc ccagcctgga gccctcggtg tccccacagg atgaaacagc 1920

tgaagtggca gttccagcgg ctgtccctgc ggcagaggct gaggccgagg tgacgctgcg 1980

ggagctccag gaagccctgg aggaggaggt gctcacccgg cagagcctga gccgggagat 2040

ggaggccatc cgcacggaca accagaactt cgccagtcaa ctacgcgagg cagaggctcg 2100

gaaccgggac ctagaggcac acgtccggca gttgcaggag cggatggagt tgctgcaggc 2160

agagggagcc acagctgtca cgggggtccc cagtccccgg gccacggatc caccttccca 2220

tctagatggc cccccggccg tggctgtggg ccagtgcccg ctggtggggc caggccccat 2280

gcaccgccgc cacctgctgc tccctgccag ggtccctagg cctggcctat cggaggcgct 2340

ttccctgctc ctgttcgccg ttgttctgtc tcgtgccgcc gccctgggct gcattgggtt 2400

ggtggcccac gccggccaac tcaccgcagt ctggcgccgc ccaggagccg cccgcgctcc 2460

ctgaacccta gaactgtctt cgactccggg gccccgttgg aagactgagt gcccggggca 2520

cggcacagaa gccgcgccca ccgcctgcca gttcacaacc gctccgagcg tgggtctccg 2580

cccagctcca gtcctgtgat ccgggcccgc cccctagcgg ccggggaggg aggggccggg 2640

tccgcggccg gcgaacgggg ctcgaagggt ccttgtagcc gggaatgctg ctgctgctgc 2700

tgctgctgct gctgctgctg ctgctgctgc tgctgctgct gctgctgggg ggatcacaga 2760

ccatttcttt ctttcggcca ggctgaggcc ctgacgtgga tgggcaaact gcaggcctgg 2820

gaaggcagca agccgggccg tccgtgttcc atcctccacg cacccccacc tatcgttggt 2880

tcgcaaagtg caaagctttc ttgtgcatga cgccctgctc tggggagcgt ctggcgcgat 2940

ctctgcctgc ttactcggga aatttgcttt tgccaaaccc gctttttcgg ggatcccgcg 3000

cccccctcct cacttgcgct gctctcggag ccccagccgg ctccgcccgc ttcggcggtt 3060

tggatattta ttgacctcgt cctccgactc gctgacaggc tacaggaccc ccaacaaccc 3120

caatccacgt tttggatgca ctgagacccc gacattcctc ggtatttatt gtctgtcccc 3180

acctaggacc cccacccccg accctcgcga ataaaaggcc ctccatctgc ccaaagctct 3240

gga 3243

<210> 43
<211> 16
<212> DNA
<213> Artificial

<220>
<223> synthetic

<220>
<221> misc_feature
<222> (1)..(3)
<223> LNA

<220>
<221> misc_feature
<222> (14)..(16)
<223> LNA

<400> 43
acaataaata ccgagg 16

<210> 44
<211> 16
<212> RNA
<213> Artificial

<220>
<223> synthetic

<220>
<221> misc_feature
<222> (1)..(3)
<223> 2'-O-Me RNA

<220>
<221> misc_feature
<222> (14)..(16)
<223> 2'-O-Me RNA

<400> 44
ccucgguauu uauugu 16

<210> 45
<211> 16
<212> RNA
<213> Artificial

<220>
<223> synthetic

<220>
<221> misc_feature
<222> (1)..(4)
<223> 2'-O-Me RNA

<220>
<221>  misc_feature
<222>  (7)..(7)
<223>  2'-O-Me RNA

<220>
<221>  misc_feature
<222>  (9)..(11)
<223>  2'-O-Me RNA

<220>
<221>  misc_feature
<222>  (14)..(16)
<223>  2'-O-Me RNA

<400>  45
ccucgguauu uauugu                                                    16


<210>  46
<211>  2761
<212>  DNA
<213>  Mus musculus

<220>
<223>  DMPK mRNA

<400>  46
gaactggcca gagagaccca agggatagtc agggacgggc agacatgcag ctagggttct      60

ggggcctgga caggggcagc caggccctgt gacgggaaga ccccgagctc cggcccgggg     120

aggggccatg gtgttgcctg cccaacatgt cagccgaagt gcggctgagg cagctccagc     180

agctggtgct ggacccaggc ttcctgggac tggagcccct gctcgacctt ctcctgggcg     240

tccaccagga gctgggtgcc tctcacctag cccaggacaa gtatgtggcc gacttcttgc     300

agtgggtgga gcccattgca gcaaggctta aggaggtccg actgcagagg gatgattttg     360

agattttgaa ggtgatcggg cgtggggcgt tcagcgaggt agcggtggtg aagatgaaac     420

agacgggcca agtgtatgcc atgaagatta tgaataagtg ggacatgctg aagagaggcg     480

aggtgtcgtg cttccgggaa gaaagggatg tattagtgaa aggggaccgg cgctggatca     540

cacagctgca ctttgccttc caggatgaga actacctgta cctggtcatg gaatactacg     600

tgggcgggga cctgctaacg ctgctgagca agtttgggga gcggatcccc gccgagatgg     660

ctcgcttcta cctggccgag attgtcatgg ccatagactc cgtgcaccgg ctgggctacg     720

tgcacaggga catcaaacca gataacattc tgctggaccg atgtgggcac attcgcctgg     780

cagacttcgg ctcctgcctc aaactgcagc ctgatggaat ggtgaggtcg ctggtggctg     840

tgggcacccc ggactacctg tctcctgaga ttctgcaggc cgttggtgga gggcctgggg     900

caggcagcta cgggccagag tgtgactggt gggcactggg cgtgttcgcc tatgagatgt     960

tctatgggca gacccccttc tacgcggact ccacagccga gacatatgcc aagattgtgc    1020

actacaggga acacttgtcg ctgccgctgg cagacacagt tgtccccgag gaagctcagg 1080

acctcattcg tgggctgctg tgtcctgctg agataaggct aggtcgaggt ggggcaggtg 1140

atttccagaa acatcctttc ttctttggcc ttgattggga gggtctccga gacagtgtac 1200

ccccctttac accagacttc gagggtgcca cggacacatg caatttcgat gtggtggagg 1260

accggctcac tgccatggtg agcgggggcg gggagacgct gtcagacatg caggaagaca 1320

tgccccttgg ggtgcgcctg cccttcgtgg gctactccta ctgctgcatg gccttcagag 1380

acaatcaggt cccggacccc acccctatgg aactagaggc cctgcagttg cctgtgtcag 1440

acttgcaagg gcttgacttg cagcccccag tgtccccacc ggatcaagtg gctgaagagg 1500

ctgacctagt ggctgtccct gcccctgtgg ctgaggcaga gaccacggta acgctgcagc 1560

agctccagga agccctggaa gaagaggttc tcacccggca gagcctgagc cgcgagctgg 1620

aggccatccg gaccgccaac cagaacttct ccagccaact acaggaggcc gaggtccgaa 1680

accgagacct ggaggcgcat gttcggcagc tacaggaacg gatggagatg ctgcaggccc 1740

caggagccgc agccatcacg gggtccccca gtccccgggc cacggatcca ccttcccatc 1800

tagatggccc cccggccgtg gctgtgggcc agtgcccgct ggtggggcca ggccccatgc 1860

accgccgtca cctgctgctc cctgccagga tccctaggcc tggcctatcc gaggcgcgtt 1920

gcctgctcct gttcgccgct gctctggctg ctgccgccac actgggctgc actgggttgg 1980

tggcctatac cggcggtctc accccagtct ggtgtttccc gggagccacc ttcgccccct 2040

gaaccctaag actccaagcc atctttcatt taggcctcct aggaaggtcg agcgaccagg 2100

gagcgaccca aagcgtctct gtgcccatcg cgcccccccc cccccccac cgctccgctc 2160

cacacttctg tgagcctggg tccccaccca gctccgctcc tgtgatccag gcctgccacc 2220

tggcggccgg ggagggagga acagggctcg tgcccagcac ccctggttcc tgcagagctg 2280

gtagccaccg ctgctgcagc agctgggcat tcgccgacct tgctttactc agccccgacg 2340

tggatgggca aactgctcag ctcatccgat ttcacttttt cactctccca gccatcagtt 2400

acaagccata agcatgagcc ccctatttcc agggacatcc cattcccata gtgatggatc 2460

agcaagacct ctgccagcac acacggagtc tttggcttcg gacagcctca ctcctggggg 2520

ttgctgcaac tccttccccg tgtacacgtc tgcactctaa caacggagcc acagctgcac 2580

tcccccctcc cccaaagcag tgtgggtatt tattgatctt gttatctgac tcactgacag 2640

actccgggac ccacgtttta gatgcattga gactcgacat tcctcggtat ttattgtctg 2700

tccccaccta cgacctccac tcccgaccct tgcgaataaa atacttctgg tctgccctaa 2760

a 2761

<210> 47
<211> 17

<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (15)..(17)
<223>  2'-O-Me RNA

<400>  47
auccugauaa acauuaa                                                    17


<210>  48
<211>  17
<212>  RNA
<213>  Artificial

<220>
<223>  synthetic

<220>
<221>  misc_feature
<222>  (1)..(5)
<223>  2'-O-Me RNA

<220>
<221>  misc_feature
<222>  (8)..(8)
<223>  2'-O-Me RNA

<220>
<221>  misc_feature
<222>  (12)..(12)
<223>  2'-O-Me RNA

<220>
<221>  misc_feature
<222>  (14)..(17)
<223>  2'-O-Me RNA

<400>  48
auccugauaa acauuaa                                                    17


<210>  49
<211>  16
<212>  DNA
<213>  Artificial

<220>
<223>  synthetic

<220>
<221>  misc_feature
<222>  (1)..(3)

<223>  LNA

<220>
<221>  misc_feature
<222>  (14)..(16)
<223>  LNA

<400>  49
gcattctaat agcagc                                                        16


<210>  50
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  50
gcugcuauua gaaugc                                                        16


<210>  51
<211>  16
<212>  DNA
<213>  Artificial

<220>
<223>  synthetic

<220>
<223>  Combined DNA/RNA molecule: Synthetic oligonucleotide

<220>
<221>  misc_feature
<222>  (1)..(3)
<223>  2'-O-Me RNA

<220>
<221>  misc_feature
<222>  (4)..(4)
<223>  RNA

<220>
<221>  misc_feature
<222>  (5)..(6)
<223>  DNA

<220>
<221>  misc_feature
<222>  (7)..(7)
<223>  RNA

<220>
<221>  misc_feature
<222>  (8)..(9)
<223>  DNA

<220>
<221>  misc_feature
<222>  (10)..(13)
<223>  RNA

<220>
<221>  misc_feature
<222>  (14)..(16)
<223>  2'-O-Me RNA

<400>  51
gcugctatta gaaugc                                                        16


<210>  52
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  52
ccucgguauu uauugu                                                        16


<210>  53
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  53
ccucgguauu uauugu                                                        16

<210>  54
<211>  16
<212>  RNA
<213>  Artificial

<220>
<223>  synthetic

<220>
<221>  misc_feature
<222>  (1)..(4)
<223>  2'-O-Me RNA

<220>
<221>  misc_feature
<222>  (7)..(7)
<223>  2'-O-Me RNA

<220>
<221>  misc_feature
<222>  (9)..(11)
<223>  2'-O-Me RNA

<220>
<221>  misc_feature
<222>  (13)..(16)
<223>  2'-O-Me RNA

<400>  54
ccucgguauu uauugu                                                        16


<210>  55
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (5)..(6)
<223>  2'-O-Me RNA

<220>
<221>  misc_feature
<222>  (8)..(8)
<223>  2'-O-Me RNA

<220>
<221>  misc_feature
<222>  (12)..(12)
<223>  2'-O-Me RNA

<220>
<221>  misc_feature

<222>  (14)..(16)
<223>  2'-O-Me RNA

<400>  55
ccucgguauu uauugu                                                    16


<210>  56
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  56
ccucgguauu uauugu                                                    16


<210>  57
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  57
ccucgguauu uauugu                                                    16


<210>  58
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  58
ccucgguauu uauugu                                                        16


<210>  59
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  59
ccucgguauu uauugu                                                        16


<210>  60
<211>  16
<212>  DNA
<213>  Artificial

<220>
<223>  synthetic

<220>
<223>  Combined DNA/RNA molecule: Synthetic oligonucleotide

<220>
<221>  misc_feature
<222>  (1)..(3)
<223>  2'-O-Me RNA

<220>
<221>  misc_feature
<222>  (4)..(4)
<223>  DNA

<220>
<221>  misc_feature
<222>  (5)..(6)
<223>  RNA

<220>
<221>  misc_feature
<222>  (7)..(7)
<223>  DNA

<220>
<221>  misc_feature
<222>  (8)..(8)
<223>  RNA

<220>
<221>  misc_feature
<222>  (9)..(11)
<223>  DNA

<220>
<221>  misc_feature
<222>  (12)..(12)
<223>  RNA

<220>
<221>  misc_feature
<222>  (13)..(13)
<223>  DNA

<220>
<221>  misc_feature
<222>  (14)..(16)
<223>  2'-O-Me RNA

<400>  60
ccucggtatt tatugu                                                    16


<210>  61
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  61
ccucgguauu uauugu                                                    16


<210>  62
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  62
ccucgguauu uauugu                                                        16


<210>  63
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  63
ccucgguauu uauugu                                                        16


<210>  64
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  64
ccucgguauu uauugu                                                        16


<210>  65
<211>  16
<212>  RNA
<213>  Artificial

<220>
<223>  synthetic

<220>

<221>  misc_feature
<222>  (1)..(3)
<223>  2'-O-Me RNA

<220>
<221>  misc_feature
<222>  (14)..(16)
<223>  2'-O-Me RNA

<400>  65
ccucgguauu uauugu                                                       16


<210>  66
<211>  16
<212>  RNA
<213>  Artificial

<220>
<223>  synthetic

<220>
<221>  misc_feature
<222>  (1)..(3)
<223>  2'-O-Me RNA

<220>
<221>  misc_feature
<222>  (14)..(16)
<223>  2'-O-Me RNA

<400>  66
ccucgguauu uauugu                                                       16


<210>  67
<211>  17
<212>  DNA
<213>  Artificial

<220>
<223>  synthetic

<220>
<221>  misc_feature
<222>  (1)..(3)
<223>  LNA

<220>
<221>  misc_feature
<222>  (15)..(17)
<223>  LNA

<400>  67
ttaatgttta tcaggat                                                      17

## Description

### Technical Field

The present invention relates to a double-stranded nucleic acid complex, a composition comprising the same, and the like.

### Background Art

In recent years, an oligonucleotide has been drawing attention in the ongoing development of pharmaceuticals called nucleic acid medicines, and particularly, the development of a nucleic acid medicine utilizing the antisense method is actively pushed forward from the viewpoint of high selectivity on target genes, and low toxicity. The antisense method is a method in which an oligonucleotide complementary to a partial sequence of mRNA or miRNA transcribed from a target gene as a target sense strand (antisense oligonucleotide: herein often referred to as "ASO") is introduced into a cell so as to selectively modify or inhibit expression of a protein encoded by the target gene or the activity of miRNA.

As a nucleic acid utilizing the antisense method, the present inventors have developed a double-stranded nucleic acid complex (heteroduplex oligonucleotide, HDO) formed by annealing an antisense oligonucleotide and a complementary strand thereto (Patent Literature 1, Non-Patent Literatures 1 and 2).

The mechanism of action of the double-stranded nucleic acid complex described above is in part as follows without limitation thereto. When a double-stranded nucleic acid complex is introduced into a cell, the RNA region that is complementary to a portion of the antisense oligonucleotide in the complementary strand is cleaved by RNase H to release the antisense oligonucleotide, and then the antisense oligonucleotide can function, for example, to modify the activity or the function of a transcriptional product (see, *e.g.,* Patent Literature 2). This is called the "RNase H-dependent pathway". It is desirable that the nucleic acid moiety is not modified for cleavage by RNase H. Meanwhile, in a case where the nucleic acid moiety is not modified, the double-stranded nucleic acid complex may be degraded by a nuclease *in vivo* and may not produce sufficient activity.

### Citation List

### Patent Literature

Patent Literature 1: International Publication No. WO 2013/089283
Patent Literature 2: International Publication No. WO 2014/192310

### Non-Patent Literature

Non-Patent Literature 1: Nishina K, et al., "DNA/RNA heteroduplex oligonucleotide for highly efficient gene silencing", Nature Communication, 2015, 6:7969.
Non-Patent Literature 2: Asami Y, et al., "Drug delivery system of therapeutic oligonucleotides", Drug Discoveries & Therapeutics, 2016; 10(5):256-262.

### Summary of Invention

### Technical Problem

It is not sufficiently understood how a nucleic acid should be modified in order to suppress the degradation of a double-stranded nucleic acid complex by a nuclease *in vivo* while maintaining the activity of the double-stranded nucleic acid complex.

An object of the present invention is to provide a double-stranded nucleic acid complex that can maintain its activity and/or suppress its degradation *in vivo.*

### Solution to Problem

The present inventors found that a complex comprising a first nucleic acid strand and a second nucleic acid strand, in which said first nucleic acid strand is a gapmer, and said second nucleic acid strand comprises a sugar-unmodified central region (also herein referred to as "first exposed region") consisting of one sugar-unmodified ribonucleoside or two or three contiguous sugar-unmodified ribonucleosides linked by an internucleoside bond complementary to a part of said first nucleic acid strand, can maintain its activity and/or suppress its degradation *in vivo.*

The present inventors also found that the function of the complex described above may be further improved by including a protected region comprising a modified or unmodified pyrimidine base in the second nucleic acid strand, thereby completing the present invention.

The present invention is based on the findings described above and encompasses the following embodiments.
[1] A nucleic acid complex comprising a first nucleic acid strand and a second nucleic acid strand, wherein
   said first nucleic acid strand:
   (1) is capable of hybridizing to at least a part of a target transcriptional product;
   (2) has an antisense effect on the target transcriptional product; and
   (3) is a gapmer comprising: a central region comprising at least four contiguous deoxyribonucleosides; and a 5' wing region and a 3' wing region each comprising a non-natural nucleoside, on the 5' end side and the 3' end side of the central region, respectively,
      said second nucleic acid strand comprises at least one sugar-unmodified central region (first exposed region) consisting of one sugar-unmodified ribonucleoside or two or three contiguous sugar-unmodified ribonucleosides linked by an internucleoside bond, which is or are complementary to a part of said first nucleic acid strand, and
      said first nucleic acid strand is annealed to said second nucleic acid strand.
[2] The nucleic acid complex according to [1], wherein said first nucleic acid strand is from 13 to 20 base in length.
[3] The nucleic acid complex according to [1] or [2], wherein the second nucleic acid strand comprises:
   (1) said sugar-unmodified central region (first exposed region) and
   (2)(a) a deoxyribonucleoside and/or (b) a sugar-modified nucleoside,
   which are linked by a modified or unmodified internucleoside bond.
[4] The nucleic acid complex according to [3], wherein said second nucleic acid strand comprises a sugar-modified ribonucleoside.
[5] The nucleic acid complex according to any of [1] to [4], wherein said sugar-unmodified central region (first exposed region) consists of three contiguous sugar-unmodified ribonucleosides linked by an internucleoside bond.
[6] The nucleic acid complex according to any of [1] to [5], wherein said second nucleic acid strand comprises only one sugar-unmodified central region (first exposed region).
[7] The nucleic acid complex according to any of [1] to [6], wherein the sugar-unmodified ribonucleoside in said sugar-unmodified central region (first exposed region) is a natural ribonucleoside.
[8] The nucleic acid complex according to any of [1] to [7], wherein said sugar-unmodified central region (first exposed region) comprises at least one modified internucleoside bond.
[9] The nucleic acid complex according to any of [1] to [8], wherein said second nucleic acid strand comprises a sugar-unmodified terminal region comprising at least one sugar-unmodified ribonucleoside at the 5' end and/or the 3' end.
[10] The nucleic acid complex according to [9], wherein the sugar-unmodified ribonucleoside in said sugar-unmodified terminal region is a natural ribonucleoside.
[11] The nucleic acid complex according to any of [1] to [10], wherein the sugar-unmodified central region (first exposed region) of said second nucleic acid strand comprises at least one phosphorothioate bond chirally controlled in an Rp or Sp configuration.
[12] The nucleic acid complex according to [11], wherein said second nucleic acid strand does not comprise a sugar-unmodified region other than the sugar-unmodified terminal region and the sugar-unmodified central region (first exposed region).
[13] The nucleic acid complex according to any of [1] to [12], wherein the base in said sugar-unmodified central region (first exposed region) is a purine base.
[14] The nucleic acid complex according to any of [1] to [13], wherein said second nucleic acid strand is linked to a functional moiety having a function selected from a labeling function, a purifying function, and a function for delivery to a target.
[15] The nucleic acid complex according to any of [1] to [14], wherein said sugar-unmodified central region (first exposed region) is positioned to the 3' side relative to the center of said second nucleic acid strand.

The present invention further encompasses the following embodiments.
[1A] A nucleic acid complex comprising a first nucleic acid strand and a second nucleic acid strand, wherein
   said first nucleic acid strand:
      (1) is capable of hybridizing to at least a part of a target transcriptional product;
      (2) has an antisense effect on the target transcriptional product; and
      (3) is a gapmer comprising: a central region comprising at least four contiguous deoxyribonucleosides; and a 5' wing region and a 3' wing region each comprising a non-natural nucleoside, on the 5' end side and the 3' end side of the central region, respectively,
   said second nucleic acid strand comprises at least one first exposed region and at least one protected region,
   said first exposed region consists of one sugar-unmodified ribonucleoside or two or three contiguous sugar-unmodified ribonucleosides linked by an internucleoside bond which is or are complementary to a part of said first nucleic acid strand, and
   said protected region consists of one of (a) a deoxyribonucleoside; (b) a sugar-modified nucleoside; and/or (c) a nucleoside having a modified internucleoside bond on the 3' side, or two or more of said (a) to (c) linked by an internucleoside bond, and
   said first nucleic acid strand is annealed to said second nucleic acid strand.
[2A] The nucleic acid complex according to [1A], wherein said first nucleic acid strand is from 13 to 22 base in length.
[3A] The nucleic acid complex according to [1A] or [2A], wherein said first exposed region consists of three contiguous sugar-unmodified ribonucleosides linked by an internucleoside bond.
[4A] The nucleic acid complex according to any of [1A] to [3A], wherein the first exposed region comprises a nucleoside comprising a modified or unmodified purine base.
[5A] The nucleic acid complex according to any of [1A] to [4A], wherein said second nucleic acid strand comprises only one said first exposed region.
[6A] The nucleic acid complex according to any of [1A] to [4A], wherein said second nucleic acid strand comprises at least two first exposed regions.
[7A] The nucleic acid complex according to any of [1A] to [6A], wherein said second nucleic acid strand comprises at least two protected regions.
[8A] The nucleic acid complex according to any of [1A] to [7A], wherein the sugar-unmodified ribonucleoside in said first exposed region is a natural ribonucleoside.
[9A] The nucleic acid complex according to any of [1A] to [8A], further comprising a second exposed region consisting of four or more contiguous sugar-unmodified ribonucleosides linked by an internucleoside bond complementary to a part of said first nucleic acid strand, wherein the base of the sugar-unmodified ribonucleoside in said second exposed region comprises a modified or unmodified purine base.
[10A] The nucleic acid complex according to any of [1A] to [9A], wherein at least one of said protected region comprises: (a) a deoxyribonucleoside; (b) a sugar-modified nucleoside; and/or (c) a nucleoside having a modified internucleoside bond on the 3' side, each of (a) to (c) comprising a modified or unmodified pyrimidine base.
[11A] The nucleic acid complex according to [10A], wherein all of said protected region comprises: (a) a deoxyribonucleoside; (b) a sugar-modified nucleoside; and/or (c) a nucleoside having a modified internucleoside bond on the 3' side, each of (a) to (c) comprising a modified or unmodified pyrimidine base.
[12A] The nucleic acid complex according to any of [1A] to [11A], wherein at least one of said protected region consists of one of (a) a deoxyribonucleoside; (b) a sugar-modified nucleoside; and/or (c) a nucleoside having a modified internucleoside bond on the 3' side, each of (a) to (c) comprising a modified or unmodified pyrimidine base, or two or more of said (a) to (c) linked by an internucleoside bond.
[13A-1] The nucleic acid complex according to any of [1A] to [12A], wherein the sugar-modified nucleoside in said protected region is a nucleoside modified at the 2'-position of the sugar and/or a bridged nucleoside.
[13A-2] The nucleic acid complex according to any of [1A] to [12A] or [13A-1], wherein the sugar-modified nucleoside in said protected region is a nucleoside modified at the 2'-position of the sugar.
[13A-3] The nucleic acid complex according to any of [1A] to [12A] or [13A-1] to [13A-2], wherein the nucleoside having a modified internucleoside bond on the 3' side in the protected region is a nucleoside having a phosphorothioate bond, an internucleoside bond comprising a guanidine moiety substituted with one to four C₁ to C₆ alkyl groups, and/or an internucleoside bond comprising a cyclic guanidine moiety, on the 3' side.
[13A-4] The nucleic acid complex according to any of [1A] to [12A] or [13A-1] to [13A-3], wherein the nucleoside having a modified internucleoside bond on the 3' side in said protected region is a nucleoside having a phosphorothioate bond on the 3' side.
[13A] The nucleic acid complex according to any of [1A] to [12A] or [13A-1] to [13A-4], wherein the sugar-modified nucleoside in said protected region is 2'-O-methyl modified nucleoside and/or the nucleoside having a modified internucleoside bond on the 3' side is a nucleoside having a phosphorothioate bond on the 3' side.
[14A] The nucleic acid complex according to any of [1A] to [13A] or [13A-1] to [13A-4], wherein said first exposed region and/or the second exposed region comprise at least one modified internucleoside bond.
[15A] The nucleic acid complex according to any of [1A] to [14A] or [13A-1] to [13A-4], wherein said second nucleic acid strand comprises a sugar-unmodified terminal region comprising at least one sugar-unmodified ribonucleoside, at the 5' end and/or the 3' end.
[16A] The nucleic acid complex according to [15A], wherein the sugar-unmodified ribonucleoside in the sugar-unmodified terminal region is a natural ribonucleoside.
[17A] The nucleic acid complex according to any of [1A] to [16A] or [13A-1] to [13A-4], wherein at least one protected region in said second nucleic acid strand is linked by an internucleoside bond to said first exposed region and/or said second exposed region at the 5' end and the 3' end.
[18A] The nucleic acid complex according to any of [1A] to [17A] or [13A-1] to [13A-4], wherein said second nucleic acid strand does not comprise a sugar-unmodified region other than the first exposed region and the second exposed region.
[19A] The nucleic acid complex according to any of [1A] to [17A] or [13A-1] to [13A-4], wherein said second nucleic acid strand comprises a sugar-modified terminal region comprising at least one deoxyribonucleoside and/or a sugar-modified nucleoside at the 5' end and/or the 3' end.
[20A] The nucleic acid complex according to any of [1A] to [19A] or [13A-1] to [13A-4], wherein said second nucleic acid strand consists of: (1) at least one said first exposed region and/or at least one second exposed region; (2) at least one said protected region; and (3) said sugar-modified terminal regions at the 5' end and the 3' end, which are linked by a modified or unmodified internucleoside bond.
[21A] The nucleic acid complex according to any of [1A] to [20A] or [13A-1] to [13A-4], wherein the first exposed region and/or the second exposed region in said second nucleic acid strand comprise at least one phosphorothioate bond chirally controlled in an Rp or Sp configuration, an internucleoside bond comprising a guanidine moiety substituted with one to four C₁ to C₆ alkyl groups, and/or an internucleoside bond comprising a cyclic guanidine moiety.
[22A] The nucleic acid complex according to any of [1A] to [21A] or [13A-1] to [13A-4], wherein the protected region in said second nucleic acid strand comprises at least one phosphorothioate bond chirally controlled in an Rp or Sp configuration, an internucleoside bond comprising a guanidine moiety substituted with one to four C₁ to C₆ alkyl groups, and/or an internucleoside bond comprising a cyclic guanidine moiety.
[23A] The nucleic acid complex according to [21A] or [22A], wherein said internucleoside bond comprising a cyclic guanidine moiety is an internucleoside bond having a partial structure represented by the following Formula (II): 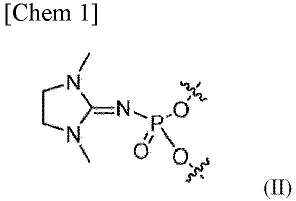
[24A] The nucleic acid complex according to any of [21A] to [23A], wherein said internucleoside bond comprising a guanidine moiety substituted with one to four C₁ to C₆ alkyl groups is an internucleoside bond having a partial structure represented by the following Formula (III): 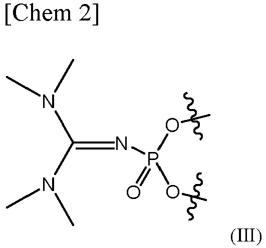
[25A] The nucleic acid complex according to any of [17A] or [19A] to [24A], wherein said second nucleic acid strand does not comprise a sugar-unmodified region other than the first exposed region, the second exposed region, and the sugar-unmodified terminal region.
[26A] The nucleic acid complex according to any of [17A] to [25A], wherein the at least one first exposed region, second exposed region, and/or protected region in said second nucleic acid strand comprises one or more mismatched bases.
[27A] The nucleic acid complex according to any of [17A] to [25A], wherein the first exposed region, second exposed region, and/or protected region in said second nucleic acid strand does not comprise a mismatched base.
[28A] The nucleic acid complex according to any of [1A] to [27A] or [13A-1] to [13A-4], wherein said second nucleic acid strand is linked to a functional moiety having a function selected from a labeling function, a purifying function, and a function for delivery to a target.
[29A] A pharmaceutical composition comprising the nucleic acid complex according to any one of [1A] to [28A] or [13A-1] to [13A-4] as an active ingredient.
[30A] A nucleic acid complex comprising a first nucleic acid strand and a second nucleic acid strand, wherein
   said first nucleic acid strand:
      (1) is capable of hybridizing to at least a part of a target transcriptional product;
      (2) has an antisense effect on the target transcriptional product; and
      (3) is a gapmer comprising: a central region comprising at least four contiguous deoxyribonucleosides; and a 5' wing region and a 3' wing region each comprising a non-natural nucleoside, on the 5' end side and the 3' end side of the central region, respectively,
   said second nucleic acid strand comprises at least one second exposed region and at least one protected region,
   said second exposed region consists of four or more contiguous sugar-unmodified ribonucleosides linked by an internucleoside bond complementary to a part of said first nucleic acid strand,
   said protected region consists of one of (a) a deoxyribonucleoside; (b) a sugar-modified nucleoside; and/or (c) a nucleoside having a modified internucleoside bond on the 3' side, or two or more of said (a) to (c) linked by an internucleoside bond, and
   said first nucleic acid strand is annealed to said second nucleic acid strand.
[31A] The nucleic acid complex according to [30A], wherein said first nucleic acid strand is from 13 to 22 base in length.
[32A] The nucleic acid complex according to [30A] or [31A], wherein said second nucleic acid strand comprises only one said second exposed region.
[33A] The nucleic acid complex according to any of [30A] to [32A], wherein said second nucleic acid strand comprises at least two second exposed regions.
[34A] The nucleic acid complex according to any of [30A] to [33A], which further comprises a first exposed region consisting of one sugar-unmodified ribonucleoside or two or three contiguous sugar-unmodified ribonucleosides linked by an internucleoside bond, which is or are complementary to a part of the first nucleic acid strand.

The present description encompasses the disclosures in Japanese Patent Application No. 2019-188042, which forms the basis of the priority of the present application.

### Advantageous Effects of Invention

The present invention provides a double-stranded nucleic acid complex having a novel structure. The nucleic acid complex of the present invention can have a high activity and/or its degradation can be suppressed *in vivo.*

### Brief Description of Drawings

[Figure 1] Figures 1A and 1B are each a schematic diagram showing an example of a specific embodiment of the nucleic acid complex according to the present invention, in which the second nucleic acid strand comprises a lipid.
[Figure 2] Figures 2A to 2C are each a schematic diagram showing an example of a specific embodiment of the nucleic acid complex according to the present invention, in which the second nucleic acid strand comprises a lipid and also comprises a complementary region and an overhang region.
[Figure 3] Figure 3 is a diagram showing an example of a general mechanism of the antisense method.
[Figure 4] Figure 4 is a diagram showing the structures of various bridged nucleic acids.
[Figure 5] Figure 5 is a diagram showing the structures of various natural nucleotides or non-natural nucleotides.
[Figure 6] Figure 6 is a schematic diagram of the structures of the nucleic acids used in Example 1. Chol represents cholesterol.
[Figure 7] Figure 7 shows the results of electrophoresis of ASO alone, a double-stranded complex (Chol-HDO) of ASO and Chol-cRNA (mMalat1), and a double-stranded complex of ASO and Chol-cRNA (mMalat1) full OMe (Chol-HDO with full OMe cRNA) after cleavage by RNase H and/or RNase A. In the RNase H-treated samples, "+" indicates the result when 10 U of Ribonuclease H was reacted with 5 µL of 10 µM nucleic acid, and "++" indicates the result when 10 U of Ribonuclease H was reacted with 5 µL of 20 µM nucleic acid.
[Figure 8] Figure 8 is a graph showing the densities of the undegraded bands based on the electrophoresis results of Figure 7 in terms of the relative intensity level (%) with respect to the untreated sample.
[Figure 9] Figure 9 is a graph showing the densities of the undegraded bands in terms of the relative intensity level (%) as in Figure 8 when a cleavage test with RNase H and/or RNase A was performed on double-stranded nucleic acid agents each comprising three contiguous sugar-unmodified ribonucleosides that are complementary to a part of the gap region in the first nucleic acid strand, in which the positions of the three contiguous sugar-unmodified ribonucleosides were changed.
[Figure 10] Figure 10 shows relative RNA expression levels when cells are treated with various double-stranded nucleic acid complexes, provided that the expression level of Malat1/Actb (β-actin) in PBS treatment is 1.

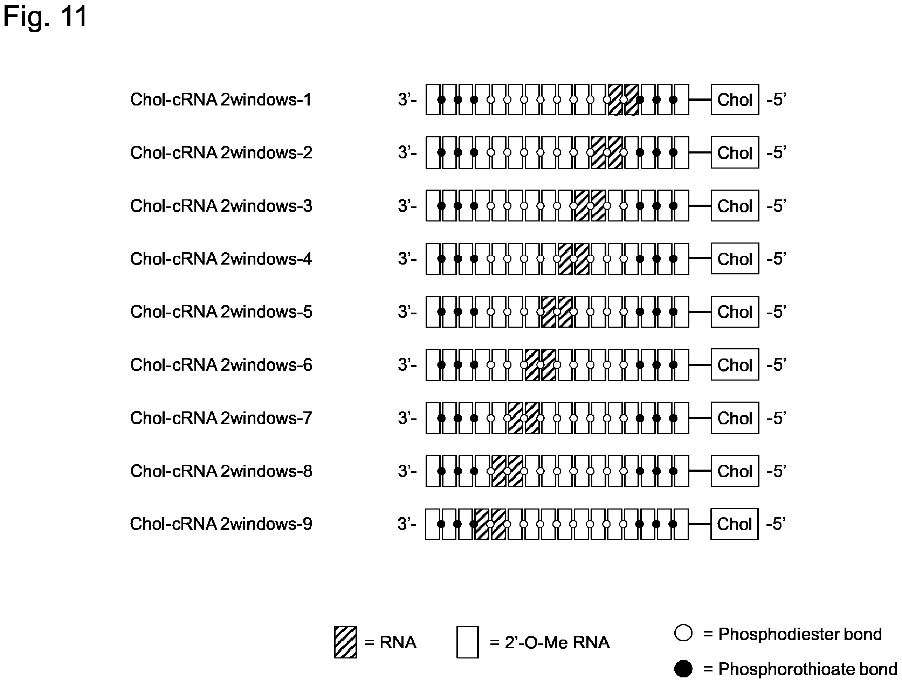

[Figure 11] Figure 11 is a schematic diagram of the structures of the nucleic acids used in Example 3. Chol represents cholesterol.

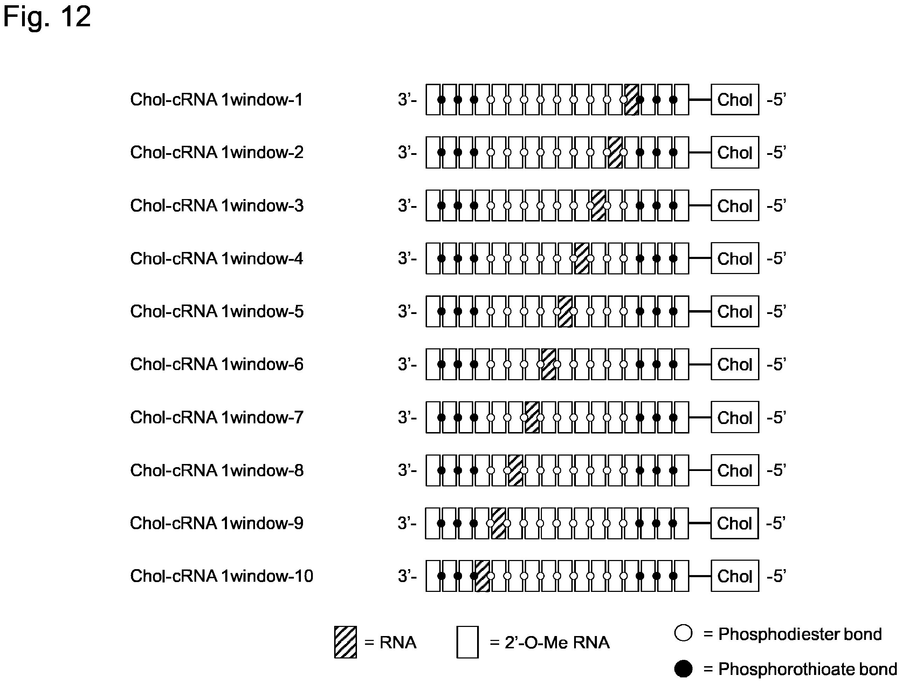

[Figure 12] Figure 12 is a schematic diagram of the structures of the nucleic acids used in Example 3. Chol represents cholesterol.
[Figure 13] Figure 13 shows the results of electrophoresis after cleavage with RNase A was performed on double-stranded nucleic acid agents each comprising one or two contiguous sugar-unmodified ribonucleosides that are complementary to a part of the gap region in the first nucleic acid strand in which the position of the sugar-unmodified ribonucleosides was changed.
[Figure 14] Figure 14 is a graph showing the densities of the undegraded bands in terms of the relative intensity level (%) as in Figure 8 after cleavage with RNase A was performed on double-stranded nucleic acid agents each comprising one or two contiguous RNAs that are complementary to a part of the gap region in the first nucleic acid strand.
[Figure 15] Figure 15 is a schematic diagram of the structures of the nucleic acids used in Example 4. Chol represents cholesterol. Figure 15A shows a double-stranded nucleic acid complex Chol-HDO (Default) comprising ASO (mDMPK) and Chol-cRNA (Default). Figure 15B shows a double-stranded nucleic acid complex Chol-HDO (CU OMe) comprising ASO (mDMPK) and Chol-cRNA (CU OMe).
[Figure 16] Figure 16 is a diagram showing the effects of double-stranded nucleic acid complexes Chol-HDO (Default) and Chol-cRNA (CU OMe) and PBS as a negative control for inhibiting the expression of the Dmpk gene in gastrocnemius (GC), triceps brachii (TB), tibialis anterior (TA), musculi dorsi proprii (Back), quadriceps (Quadriceps), and heart muscle (Heart). Relative RNA expression levels are shown, provided that the expression level of Dmpk/Actb (β-actin) is 1.
[Figure 17] Figure 17 is a diagram showing the effects of double-stranded nucleic acid complexes Chol-HDO (Default) and Chol-cRNA (CU OMe) and PBS as a negative control for inhibiting the expression of the Dmpk gene in kidney and liver. Relative RNA expression levels are shown, provided that the expression level of Dmpk/Actb (β-actin) is 1.
[Figure 18] Figure 18 is a schematic diagram of the structures of the nucleic acids used in Example 5. Chol represents cholesterol. Figure 18A shows a double-stranded nucleic acid complex Chol-HDO (Default) comprising ASO (hSOD1) and Chol-cRNA (Default). Figure 18B shows a double-stranded nucleic acid complex Chol-HDO (CU OMe) comprising ASO (hSOD1) and Chol-cRNA (CU OMe).
[Figure 19] Figure 19 is a diagram showing the effects of double-stranded nucleic acid complexes Chol-HDO (Default) and Chol-cRNA (CU OMe) and PBS as a negative control for inhibiting the expression of the SOD1 gene in musculi dorsi proprii (Back), quadriceps (Quadriceps), heart muscle (Heart), and diaphragm (Diaphragm). Relative RNA expression levels are shown, provided that the expression level of SOD1/Actb (β-actin) is 1.
[Figure 20] Figure 20 is a schematic diagram of the structures of the nucleic acids used in Example 6. Chol represents cholesterol. Figure 20A shows a double-stranded nucleic acid complex Chol-HDO (Default) comprising ASO (Malat1) and Chol-cRNA (Default). Figure 20B shows a double-stranded nucleic acid complex Chol-HDO (CT DNA) comprising ASO (Malat1) and Chol-cRNA (CT DNA).
[Figure 21] Figure 21 is a diagram showing the effects of double-stranded nucleic acid complexes Chol-HDO (Default) and Chol-cRNA (CU OMe) and PBS as a negative control for inhibiting the expression of the Malat1 gene in the cervical spinal cord. Relative RNA expression levels are shown, provided that the expression level of Malat1/Actb (β-actin) is 1.
[Figure 22A] Figure 22A shows the sequences, chemical modifications, and structures of the oligonucleotides used in Example 7.
[Figure 22B] Figure 22B shows the sequences, chemical modifications, and structures of the oligonucleotides used in Example 7.
[Figure 23] Figure 23 shows the results of stability examined by mixing a double-stranded nucleic acid complex comprising a first nucleic acid strand comprising an antisense oligonucleotide and a complementary strand (second nucleic acid strand) comprising modified ribonucleosides with mouse serum for the indicated time (0 hr, 24 hr) and performing electrophoresis.
[Figure 24] Figure 24 is a graph showing the concentrations of the double-stranded bands after 24 hr based on the electrophoresis results of Figure 23 in terms of the relative intensity level (%) with respect to the double-stranded band after 0 hr.
[Figure 25] Figure 25 shows the results of stability examined by mixing a double-stranded nucleic acid complex comprising a first nucleic acid strand comprising an antisense oligonucleotide and a complementary strand (second nucleic acid strand) comprising modified ribonucleosides with human serum for the indicated time (0 hr, 2 hr) and performing electrophoresis.
[Figure 26] Figure 26 is a graph showing the concentrations of the double-stranded bands after 2 hr based on the electrophoresis results of Figure 25 in terms of the relative intensity level (%) with respect to the double-stranded band after 0 hr.

### Description of Embodiments

### <Nucleic Acid Complex>

In an aspect, the present invention relates to a nucleic acid complex comprising a first nucleic acid strand and a second nucleic acid strand, in which the first nucleic acid strand is annealed to the second nucleic acid strand. The first nucleic acid strand (1) is capable of hybridizing to at least a part of a target transcriptional product; (2) has an antisense effect on the target transcriptional product; and (3) is a gapmer. The nucleic acid complex of the present invention and the nucleic acid strands constituting the same are described in detail below.

The first nucleic acid strand may be a nucleotide strand comprising or consisting of an antisense oligonucleotide region with respect to a target transcriptional product. An "antisense oligonucleotide" or an "antisense nucleic acid" refers to a single-stranded oligonucleotide that comprises a base sequence that is capable of hybridizing (namely being complementary) to at least a part of a target transcriptional product (mainly a transcriptional product of a target gene), and is able to produce an antisense effect on a target transcriptional product. In the present invention, the antisense oligonucleotide region in the first nucleic acid strand can produce an antisense effect on a target transcriptional product. The target region of a target transcriptional product can be: at least 8 base in length, for example, from 10 to 35 base in length, from 12 to 25 base in length, from 13 to 20 base in length, from 14 to 19 base in length, or from 15 to 18 base in length; or from 13 to 22 base in length, from 16 to 22 base in length, or from 16 to 20 base in length.

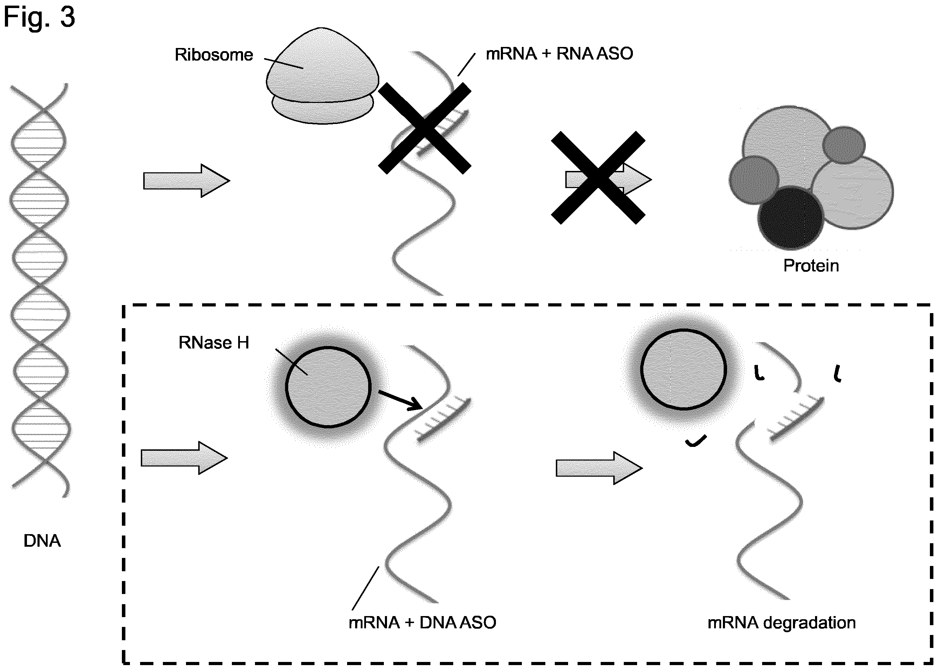

The "antisense effect" means the modulation of expression of a target transcriptional product, which results from hybridization of the target transcriptional product (RNA sense strand) with a strand (e.g., DNA strand) that is complementary to a partial sequence of a transcriptional product or the like and is designed to produce an antisense effect. The modulation of expression of a target transcriptional product comprises inhibition or reduction of expression of a target gene or the level (expression level) of a target transcriptional product, or in a certain example inhibition of translation or splicing function modification effect such as exon skipping, or degradation of a transcriptional product (see Figure 3). For example, in the case of inhibition of translation, when an oligonucleotide comprising RNA is introduced into a cell as an antisense oligonucleotide (ASO), the ASO binds to a transcriptional product (mRNA) of the target gene to form a partial double strand. This partial double strand serves as a cover to prevent translation by a ribosome, and thus the expression of a protein encoded by the target gene is inhibited at the translational level (Figure 3, x mark outside the dashed line box). Meanwhile, when an oligonucleotide comprising DNA is introduced into a cell as ASO, a partial DNA-RNA heteroduplex is formed. This heteroduplex structure is recognized by RNase H, and as a result mRNA of the target gene is degraded, and consequently, the expression of a protein encoded by the target gene is inhibited at the expression level (Figure 3, inside the dashed line box). This is called the "RNase H- dependent pathway". Further, in a certain example, an antisense effect may also be brought about by targeting an intron of an mRNA precursor. An antisense effect can also be brought about by targeting miRNA, and in this case, the function of the miRNAs is inhibited, and expression of a gene whose expression is normally regulated by the miRNA may be increased. In an embodiment, the modulation of expression of a target transcriptional product may be reduction of the amount of the target transcriptional product.

The antisense oligonucleotide region in the first nucleic acid strand comprises a base sequence that can hybridize to at least a part of a target transcriptional product (for example, any target region). The target region may comprise a 3' UTR, a 5' UTR, an exon, an intron, a coding region, a translation initiation region, a translation termination region, or any other nucleic acid region.

Although there is no particular restriction on the "target gene" whose expression is modulated (for example, inhibited, altered, or modified) by an antisense effect, examples thereof include genes whose expression increases in a variety of diseases. A target transcriptional product includes an mRNA transcribed from the genomic DNA encoding the target gene, and also includes mRNAs without base modification, an unprocessed mRNA precursor, or the like. A "target transcriptional product" may include not only an mRNA, but also a non-coding RNA (ncRNA) such as a miRNA. Further, in general, a "transcriptional product" may be any RNA synthesized by a DNA-dependent RNA polymerase. In an embodiment, a "target transcriptional product" may be, for example, the gene of scavenger receptor B1 (herein often referred to as "SR-B1"), myotonic dystrophy protein kinase (herein often referred to as "DMPK"), transthyretin (herein often referred to as "TTR"), apolipoprotein B (herein often referred to as "ApoB"), or metastasis associated lung adenocarcinoma transcript 1 (herein often referred to as "Malat1"), for example, non-coding RNA or mRNA thereof. The base sequence of murine Malat1 non-coding RNA is shown in SEQ ID NO: 1, and the base sequence of human Malat1 non-coding RNA is shown in SEQ ID NO: 2. The base sequence of murine SR-B1 mRNA is shown in SEQ ID NO: 39, and the base sequence of human SR-B1 mRNA is shown in SEQ ID NO: 40. The base sequence of murine DMPK mRNA is shown in SEQ ID NO: 41, and the base sequence of human DMPK mRNA is shown in SEQ ID NO: 42. Note that the base sequence of mRNA is replaced with the base sequence of DNA in SEQ ID NOs: 1-2, and 39 to 42. Base sequence information of these genes and transcriptional products is available from a known database such as the NCBI (U.S. National Center for Biotechnology Information) database (e.g., GenBank, Trace Archive, Sequence Read Archive, BioSample, BioProject). The target region of a target transcriptional product may comprise, for example, the base sequence of position 1317 to 1332 of SEQ ID NO: 1 in the case of murine Malat-1 non-coding RNA.

The term "nucleic acid" or "nucleic acid molecule" used herein may refer to a monomer of a nucleotide or a nucleoside, or may mean an oligonucleotide consisting of a plurality of monomers. The term "nucleic acid strand" or "strand" is also used herein to refer to an oligonucleotide. A nucleic acid strand can be produced as a full-length strand, or a partial strand by a chemical synthesis method (for example, with an automated synthesis apparatus) or by an enzymatic process (for example, but not limited to, by a polymerase, ligase, or a restriction reaction).

The term "nucleobase" or "base" used herein means a base component (heterocyclic moiety) constituting a nucleic acid, and primarily adenine guanine, cytosine, thymine, and uracil are known.

The term "complementary" used herein means a relationship in which nucleobases can form so-called Watson-Crick base pairs (natural type base pair), or non-Watson-Crick base pairs (such as Hoogsteen type base pairs) via hydrogen bonds. In the present invention, it is not necessarily required that the antisense oligonucleotide region in the first nucleic acid strand is completely complementary to at least a part of a target transcriptional product *(e.g.,* the transcriptional product of a target gene), but it is permitted if the base sequence has a complementarity of at least 70%, preferably at least 80%, and more preferably at least 90% *(e.g.,* 95%, 96%, 97%, 98%, or 99% or more). An antisense oligonucleotide region in the first nucleic acid strand can hybridize to a target transcriptional product, when the base sequence is complementary (typically, when the base sequence is complementary to at least a part of the base sequence of the target transcriptional product). Similarly, it is not necessarily required that the complementary region in the second nucleic acid strand is completely complementary to at least a part of the first nucleic acid strand, but it is permitted if the base sequence has a complementarity of at least 70%, preferably at least 80%, and more preferably at least 90% *(e.g.,* 95%, 96%, 97%, 98%, or 99% or more). When the base sequence of the complementary region in the second nucleic acid strand is complementary to at least a part of the first nucleic acid strand, the region can be annealed thereto. The complementarity of a base sequence can be determined using a BLAST program or the like. One skilled in the art can readily determine the conditions (temperature, salt concentration, etc.) under which two strands can be annealed or hybridized to each other, taking into account the complementarity between the strands. Further, one skilled in the art can readily design an antisense nucleic acid that is complementary to the target transcriptional product based on, for example, information on the base sequence of a target gene.

Hybridization conditions may be a variety of stringent conditions, such as a low-stringent condition and a high-stringent condition. A low-stringent condition may be a condition with a relatively low temperature and a high salt concentration, such as 30°C, 2 × SSC, and 0.1% SDS. A high-stringent condition may be a condition with a relatively high temperature and a low salt concentration, such as 65°C, 0.1 × SSC, and 0.1% SDS. The stringency of hybridization can be adjusted by varying the conditions, such as temperature and salt concentration. Here, 1 × SSC contains 150 mM of sodium chloride and 15 mM of sodium citrate.

The antisense oligonucleotide region in the first nucleic acid strand may be usually, but not particularly limited to, at least 8 base in length, at least 9 base in length, at least 10 base in length, at least 11 base in length, at least 12 base in length, or at least 13 base in length. The antisense oligonucleotide region in the first nucleic acid strand may be 35 base in length or less, 30 base in length or less, 25 base in length or less, 24 base in length or less, 23 base in length or less, 22 base in length or less, 21 base in length or less, 20 base in length or less, 19 base in length or less, 18 base in length or less, 17 base in length or less, or 16 base in length or less. The antisense oligonucleotide region in the first nucleic acid strand may be, for example: from 8 to 35 base in length, from 9 to 30 base in length, from 10 to 25 base in length, from 10 to 20 base in length, from 11 to 18 base in length, or 12 to 16 base in length; or from 13 to 22 base in length, from 16 to 22 base in length, or from 16 to 20 base in length.

Although there is no particular restriction, the first nucleic acid strand may be at least 9 base in length, at least 10 base in length, at least 11 base in length, at least 12 base in length, or at least 13 base in length. The first nucleic acid strand may be 50 base in length or less, 45 base in length or less, 40 base in length or less, 35 base in length or less, 30 base in length or less, 28 base in length or less, 26 base in length or less, 24 base in length or less, 22 base in length or less, 20 base in length or less, 18 base in length or less, or 16 base in length or less. The first nucleic acid strand may be, for example: from 9 to 50 base in length, from 10 to 40 base in length, from 11 to 35 base in length, from 12 to 30 base in length, or from 13 to 20 base in length; or from 13 to 20 base in length, from 16 to 22 base in length, or from 16 to 20 base in length.

The complementary region in the second nucleic acid strand may be usually, but not particularly limited to, at least 8 base in length, at least 9 base in length, at least 10 base in length, at least 11 base in length, at least 12 base in length, or at least 13 base in length. The complementary region in the second nucleic acid strand may be 35 base in length or less, 30 base in length or less, 25 base in length or less, 24 base in length or less, 23 base in length or less, 22 base in length or less, 21 base in length or less, 20 base in length or less, 19 base in length or less, 18 base in length or less, 17 base in length or less, or 16 base in length or less. In an embodiment, the complementary region in the second nucleic acid strand is from 9 to 35 base in length, from 9 to 30 base in length, from 10 to 25 base in length, from 10 to 20 base in length, from 11 to 18 base in length, or from 12 to 16 base in length. In an embodiment, the complementary region in the second nucleic acid strand is from 13 to 22 base in length, from 16 to 22 base in length, or from 16 to 20 base in length. In an embodiment, the complementary region in the second nucleic acid strand is from 8 to 35 base in length, from 8 to 30 base in length, from 8 to 25 base in length, from 8 to 20 base in length, from 8 to 16 base in length, from 8 to 12 base in length, or from 8 to 10 base in length.

The second nucleic acid strand may be, but not particularly limited to, at least 5 base in length, at least 6 base in length, at least 7 base in length, at least 8 base in length, at least 9 base in length, at least 10 base in length, at least 11 base in length, at least 12 base in length, or at least 13 base in length. The second nucleic acid strand may be 50 base in length or less, 45 base in length or less, 40 base in length or less, 35 base in length or less, 30 base in length or less, 28 base in length or less, 26 base in length or less, 24 base in length or less, 22 base in length or less, 20 base in length or less, 18 base in length or less, 16 base in length or less, 14 base in length or less, 12 base in length or less, 10 base in length or less, or 9 base in length or less. The second nucleic acid strand may be, for example: from 9 to 50 base in length, from 10 to 40 base in length, from 11 to 35 base in length, from 12 to 30 base in length, or from 13 to 20 base in length; from 13 to 22 base in length, from 16 to 22 base in length, or from 16 to 20 base in length; or from 5 to 14 base in length, from 6 to 12 base in length, from 6 to 10 base in length, from 7 to 10 base in length, from 8 to 10 base in length, from 8 to 9 base in length, or from 7 to 9 base in length. The length is generally selected especially according to the balance between the strength of the antisense effect and the specificity of the nucleic acid strand with respect to the target among other factors, such as cost, and synthesis yield.

The second nucleic acid strand comprises or consists of a complementary region that is complementary to at least a part of the first nucleic acid strand.

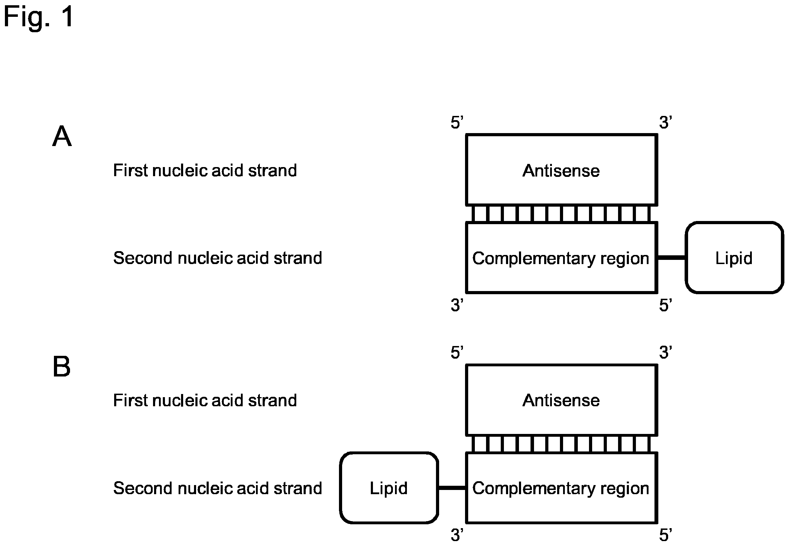

In an embodiment, the complementary region in the second nucleic acid strand may be complementary to at least a part of the antisense oligonucleotide region in the first nucleic acid strand. The complementary region in the second nucleic acid strand may be complementary to all of the antisense oligonucleotide regions in the first nucleic acid strand. The complementary region in the second nucleic acid strand may be complementary also to a part of the first nucleic acid strand other than the antisense oligonucleotide region, in addition to the antisense oligonucleotide region. As an example of this embodiment, there is a heteroduplex oligonucleotide (HDO) as disclosed in International Publication No. WO 2013/089283, Nishina K., et al. Nature Communication, 2015, 6:7969, or Asami Y., et al. Drug Discoveries & Therapeutics, 2016; 10(5):256-262 (Figures 1A and 1B).

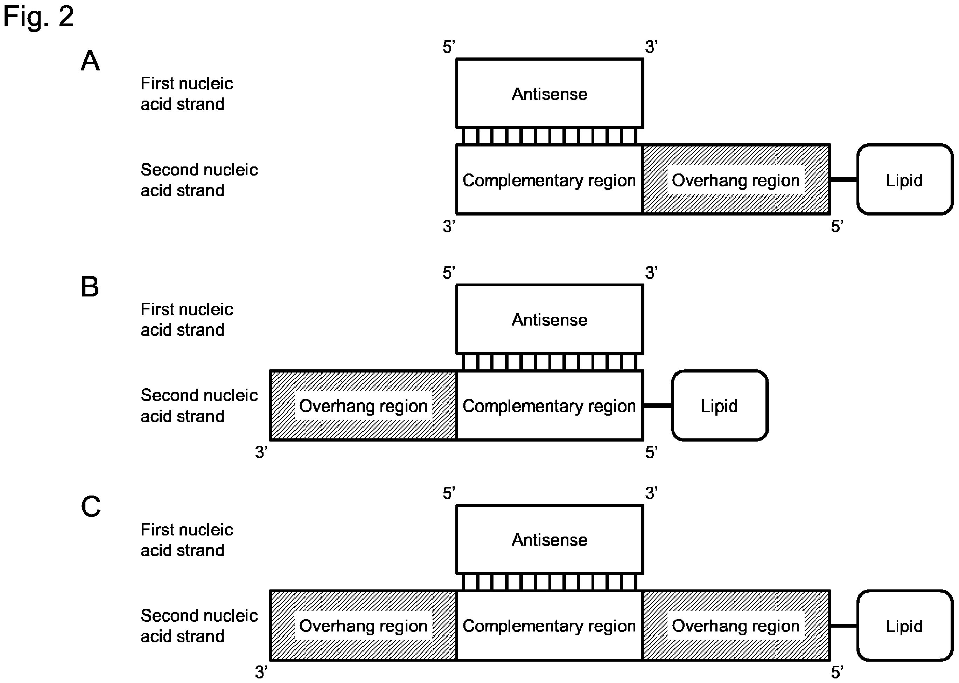

In a further embodiment, the second nucleic acid strand may further comprise at least one overhang region located on either or both of the 5' end side and the 3' end side of the complementary region. An example of this embodiment is described in International Publication No. WO 2018/062510. An "overhang region" refers to a region adjacent to the complementary region, where the 5' end of the second nucleic acid strand extends beyond the 3' end of the first nucleic acid strand, and/or the 3' end of the second nucleic acid strand extends beyond the 5' end of the first nucleic acid strand, when the first nucleic acid strand and the second nucleic acid strand anneal to form a double-stranded structure. In other words, it means nucleotide region(s) in the second nucleic acid strand, protruded from the double-stranded structure. The overhang region in the second nucleic acid strand may be located on the 5' end side of the complementary region (Figure 2A), or on the 3' end side (Figure 2B). The overhang regions in the second nucleic acid strand may be located on the 5' end side and the 3' end side of the complementary region (Figure 2C).

In general, a "nucleoside" is a combination of a base and a sugar. The nucleobase moiety (also known as a base) of a nucleoside is usually a heterocyclic base moiety. A "nucleotide" further comprises a phosphate group covalently bonded to the sugar moiety of the nucleoside. In a nucleoside comprising a pentofuranosyl sugar, a phosphate group can be linked to the 2', 3', or 5' hydroxyl portion of the sugar. An oligonucleotide is formed by contiguous nucleosides linked to each other by a covalent bond, forming a linear polymer oligonucleotide. Inside the oligonucleotide structure, it is generally considered that a phosphate group generally forms an internucleoside bond in the oligonucleotide.

A nucleic acid strand can comprise a natural nucleotide and/or a non-natural nucleotide. A "natural nucleotide" includes a deoxyribonucleotide found in DNA and a ribonucleotide found in RNA. The "deoxyribonucleotide" and "ribonucleotide" may be also referred to as "DNA nucleotide" and "RNA nucleotide", respectively.

Similarly, a "natural nucleoside" includes a deoxyribonucleoside found in DNA and a ribonucleoside found in RNA. The "deoxyribonucleoside" and "ribonucleoside" may be also referred to as "DNA nucleoside" and "RNA nucleoside," respectively.

A "non-natural nucleotide" refers to any nucleotide other than a natural nucleotide and includes a modified nucleotide or a nucleotide mimic. Similarly, a "non-natural nucleoside" refers to any nucleoside other than natural nucleoside, and includes a modified nucleoside, or a nucleoside mimic. A "modified nucleotide" means a nucleotide comprising one or more of a modified sugar moiety, a modified internucleoside bond, and a modified nucleobase. A "modified nucleoside" means a nucleoside having a modified sugar moiety and/or a modified nucleobase. A nucleic acid strand comprising a non-natural oligonucleotide is in many cases more preferable than a natural type, because of such desirable characteristics as enhanced cellular uptake, enhanced affinity for a nucleic acid target, increased stability in the presence of a nuclease, or increased inhibitory activity.

The term "modified internucleoside bond" refers to an internucleoside bond having a substitution or any change from a naturally occurring internucleoside bond (i.e., phosphodiester bond). The modified internucleoside bond includes an internucleoside bond comprising a phosphorus atom, and an internucleoside bond not comprising a phosphorus atom. Examples of a typical phosphorus-containing internucleoside bond include, but are not limited to, a phosphodiester bond, a phosphorothioate bond, a phosphorodithioate bond, a phosphotriester bond (a methylphosphotriester bond or an ethylphosphotriester bond disclosed in U.S. Patent No. 5,955,599), an alkylphosphonate bond *(e.g.,* a methylphosphonate bond disclosed in U.S. Patent Nos. 5,264,423 and 5,286,717 or a methoxypropylphosphonate bond disclosed in International Publication No. WO 2015/168172), an alkylthiophosphonate bond, a methylthiophosphonate bond, a boranophosphonate bond, an internucleoside bond comprising a cyclic guanidine moiety *(e.g.,* a substructure represented by the following formula (II): 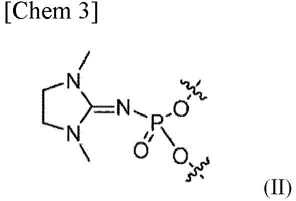 ), an internucleoside bond comprising a guanidine moiety substituted with one to four C₁ to C₆ alkyl groups *(e.g.,* a tetramethylguanidine (TMG) moiety) *(e.g.,* a substructure represented by the following formula (III): 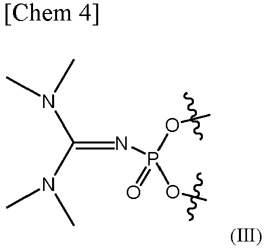 ), an internucleoside bond and a phosphoramidate bond used for a self-neutralizing nucleic acid (ZON) disclosed in International Publication No. WO 2016/081600. A phosphorothioate bond refers to an internucleoside bond in which a non-bridging oxygen atom of a phosphodiester bond is substituted with a sulfur atom. A method for preparing a phosphorus-containing bond or a phosphorus-free bond is well known. A modified internucleoside bond is preferably a bond whose nuclease resistance is higher than a naturally occurring internucleoside bond.

In a case where an internucleoside bond has a chiral center, the internucleoside bond may be chirally controlled. The expression "chirally controlled" means that the bond is present as a single diastereomer with respect to the chiral center, for example, chiral-linked phosphorus. A chirally controlled internucleoside bond may be completely chirally pure or may have high chiral purity, for example, a chiral purity of 90%de, 95%de, 98%de, 99%de, 99.5%de, 99.8%de, or 99.9%de or more. The term "chiral purity" herein refers to a proportion of one diastereomer in a mixture of diastereomers and is expressed as diastereomeric excess (%de) which is defined as (diastereomers of interest - other diastereomers) / (all diastereomers) × 100 (%).

For example, an internucleoside bond may be a phosphorothioate bond chirally controlled in an Rp or Sp configuration, an internucleoside bond *(e.g.,* a partial structure represented by Formula (III)) comprising a guanidine moiety substituted with one to four C₁ to C₆ alkyl groups *(e.g.,* tetramethylguanidine (TMG) moiety; see, *e.g.,* Alexander A. Lomzov et al., Biochem Biophys Res Commun., 2019, 513(4), 807-811), and/or an internucleoside bond *(e.g.,* a partial structure represented by Formula (II)) comprising a cyclic guanidine moiety. A method for preparing a chirally controlled internucleoside bond is known, and a phosphorothioate bond chirally controlled in an Rp or Sp configuration can be synthesized according to the methods described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, and Yohei Nukaga et al., J. Org. Chem. 2012, 77, 7913-7922. A phosphorothioate bond chirally controlled in an Rp or Sp configuration is also known, and it is known to have effects as described in Naoki Iwamoto et al., Nat. Biotechnol, 2017, 35(9), 845-851 and Anastasia Khvorova et al., Nat. Biotechnol., 2017, 35(3), 238-248. For example, in an embodiment, a phosphorothioate bond chirally controlled in an Sp configuration is more stable than that in an Rp configuration, and/or ASO chirally controlled in an Sp configuration promotes target RNA cleavage by RNase HI and brings a more sustained response *in vivo.* A method for preparing an internucleoside bond comprising a guanidine moiety substituted with one to four C₁ to C₆ alkyl groups *(e.g.,* TMG moiety) is known, and an internucleoside bond can be synthesized according to, for example, the method described in Alexander A. Lomzov et al., Biochem Biophys Res Commun., 2019, 513(4), 807-811.

The term "nucleobase" or "base" herein encompasses any modified or unmodified nucleobase (base) unless otherwise specified. Therefore, unless otherwise specified, the purine base may be either a modified or unmodified purine base. Unless otherwise specified, the pyrimidine base may be either a modified or unmodified pyrimidine base.

A "modified nucleobase" or a "modified base" means any nucleobase other than adenine, cytosine guanine, thymine, or uracil. An "unmodified nucleobase" or an "unmodified base" (natural nucleobase) means adenine (A) and guanine (G), which are purine bases, as well as thymine (T), cytosine (C), and uracil (U), which are pyrimidine bases. Examples of a modified nucleobase include, but are not limited to, 5-methylcytosine, 5-fluorocytosine, 5-bromocytosine, 5-iodocytosine, or N4-methylcytosine; N6-methyladenine or 8-bromoadenine; 2-thio-thymine; and N2-methylguanine or 8-bromoguanine. A modified nucleobase is preferably 5-methylcytosine.

The term "modified sugar" refers to a sugar having a substitution and/or any change from a natural sugar moiety (i.e., a sugar moiety found in DNA(2'-H) or RNA(2'-OH)). The term "sugar-modified" refers to substitution and/or any change from a natural sugar moiety. A nucleic acid strand may, in some cases, comprise one or more modified nucleoside including a modified sugar. The term "sugar-modified nucleoside" refers to a nucleoside having a modified sugar moiety. Such a sugar-modified nucleoside can confer beneficial biological properties, such as enhanced nuclease stability, an increased binding affinity, or the like to a nucleic acid strand. In a certain embodiment, a nucleoside comprises a chemically modified ribofuranose ring moiety. Examples of a chemically modified ribofuranose ring include, but are not limited to, the addition of a substituent (including 5' or 2' substituent), formation of a bicyclic nucleic acid (bridged nucleic acid, BNA) by forming a bridge between non-geminal ring atoms, and substitution of a ribosyl ring oxygen atom with S, N(R), or C(R1)(R2) (wherein R, R1 and R2 each independently represents H, a C₁ to C₁₂ alkyl, or a protective group), and a combination thereof.

Examples of a sugar-modified nucleoside include, but not limited to, a nucleoside having a substituent, such as 5'-vinyl, 5'-methyl (R or S), 5'-allyl (R or S), 4'-S, 2'-F (2'-fluoro group), 2'-OCH₃ (2'-OMe group, or 2'-O-methyl group), and 2'-O(CH₂)₂OCH₃. A substituent at the 2' position may be selected from allyl, amino, azide, thio, -O-allyl, -O-C₁-C₁₀ alkyl, -OCF₃, -O(CH₂)₂SCH₃, -O(CH₂)₂-O-N(Rm)(Rn), and -O-CH₂-C(=O)-N(Rm)(Rn), wherein each Rm and Rn is independently H or a substituted or unsubstituted C₁-C₁₀ alkyl. A "2'-modified sugar" means a furanosyl sugar modified at the 2' position. A nucleoside comprising a 2'-modified sugar may be referred to as "nucleoside modified at the 2' position of the sugar".

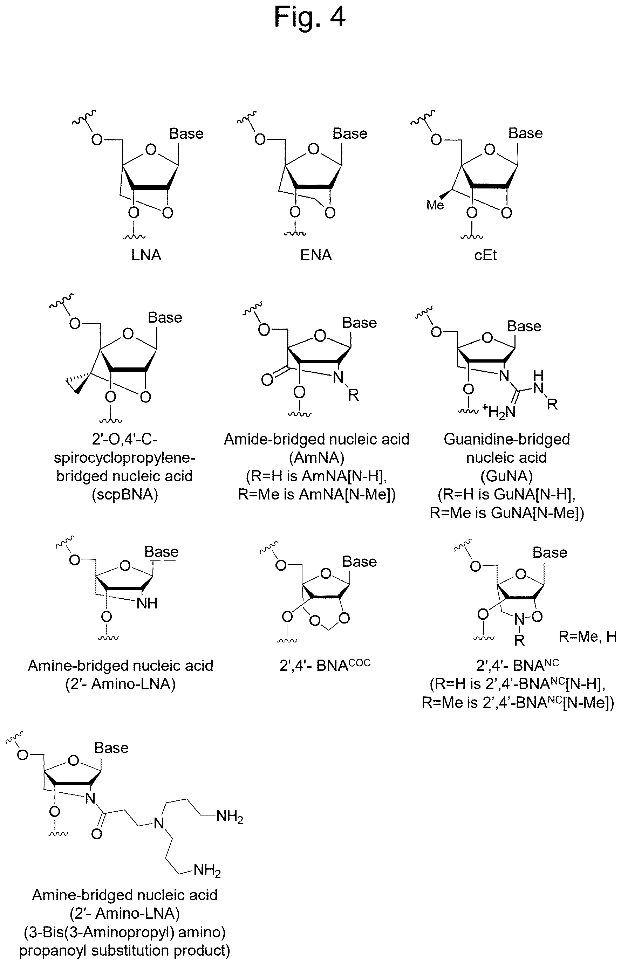

The term "bicyclic nucleoside" refers to a modified nucleoside comprising a bicyclic sugar moiety. A nucleic acid comprising a bicyclic sugar moiety is generally referred to as bridged nucleic acid (BNA). A nucleoside comprising a bicyclic sugar moiety may be referred to as "bridged nucleoside" or "BNA nucleoside". Figure 4 exemplifies some of the bridged nucleic acids.

A bicyclic sugar may be a sugar in which the carbon atom at the 2' position and the carbon atom at the 4' position are bridged via two or more atoms. Examples of a bicyclic sugar are known to those skilled in the art. A subgroup of nucleic acid comprising a bicyclic sugar (BNA) or BNA nucleoside may be described as having a carbon atom at the 2' position and a carbon atom at the 4' position bridged by 4'-(CH₂)ₚ-O-2', 4'-(CH₂)ₚ-CH₂-2', 4'-(CH₂)ₚ-S-2', 4'-(CH₂)ₚ-OCO-2', or 4'-(CH₂)ₙ-N(R₃)-O-(CH₂)ₘ-2' [wherein p, m, and n respectively represent integers of 1 to 4, 0 to 2, and 1 to 3; and R₃ represents a hydrogen atom, an alkyl group, an alkenyl group, a cycloalkyl group, an aryl group, an aralkyl group, an acyl group, a sulfonyl group, or a unit substituent (such as a fluorescently or chemiluminescently labeled molecule, a functional group having nucleic acid cleavage activity, and intracellular or intranuclear localization signal peptide)]. Further, regarding the BNA or BNA nucleoside according to a specific embodiment, in an OR₂ substituent on the carbon atom at the 3' position and an OR₁ substituent on the carbon atom at the 5' position, R₁ and R₂ are typically a hydrogen atom, but they may be the same or different, and further may be a protecting group for a hydroxyl group for nucleic acid synthesis, an alkyl group, an alkenyl group, a cycloalkyl group, an aryl group, an aralkyl group, an acyl group, a sulfonyl group, a silyl group, a phosphate group, a phosphate group protected by a protecting group for nucleic acid synthesis, or -P(R₄)R₅ [wherein R₄ and R₅ may be the same or different, and respectively may be a hydroxyl group, a hydroxyl group protected by a protecting group for nucleic acid synthesis, a mercapto group, a mercapto group protected by a protecting group for nucleic acid synthesis, an amino group, an alkoxy group having 1 to 5 carbon atoms, an alkylthio group having 1 to 5 carbon atoms, a cyanoalkoxy group having 1 to 6 carbon atoms, or an amino group substituted with an alkyl group having 1 to 5 carbon atoms]. Non-restrictive examples of such BNA include methyleneoxy(4'-CH₂-O-2') BNA (LNA (Locked Nucleic Acid^{®}, also known as 2',4'-BNA), (e.g., α-L-methyleneoxy(4'-CH₂-O-2') BNA, or β-D-methyleneoxy(4'-CH₂-O-2') BNA, ethyleneoxy(4'-(CH₂)₂-O-2') BNA (also known as ENA), β-D-thio(4'-CH₂-S-2') BNA, aminooxy(4'-CH₂-O-N(R₃)-2') BNA, oxyamino(4'-CH₂-N(R₃)-O-2') BNA (also known as 2',4'-BNA^{NC}; R=H is 2',4'-BNA^{NC}[N-H], R=Me is 2',4'-BNA^{NC}[N-Me]), 2',4'-BNA^{coc}, 3'-amino-2',4'-BNA, 5'-methyl BNA, (4'-CH(CH₃)-O-2') BNA (also known as cEt BNA), (4'-CH(CH₂OCH₃)-O-2') BNA (also known as cMOE BNA), amide BNA, (4'-C(O)-N(R)-2') BNA (R=H, Me) (also known as AmNA; R=H is AmNA[N-H], R=Me is AmNA[N-Me])), guanidine BNA (also known as GuNA *(e.g.,* R=H is GuNA[N-H], R=Me is GuNA[N-Me] in Figure 4)), amine BNA (also known as 2'-Amino-LNA) (e.g., 3-(Bis(3-aminopropyl)amino)propanoyl substitution product), 2'-O,4'-C-spirocyclopropylene-bridged nucleic acid (also known as scpBNA), and other BNAs known to those skilled in the art. Non-restrictive examples of such BNA nucleoside include methyleneoxy(4'-CH₂-O-2') BNA nucleoside (LNA nucleoside, also known as 2',4'-BNA nucleoside), (e.g., α-L-methyleneoxy(4'-CH₂-O-2') BNA nucleoside, or β-D-methyleneoxy(4'-CH₂-O-2') BNA nucleoside), ethyleneoxy(4'-(CH₂)₂-O-2') BNA nucleoside (also known as ENA nucleoside), β-D-thio(4'-CH₂-S-2') BNA nucleoside, aminooxy(4'-CH₂-O-N(R₃)-2') BNA nucleoside, oxyamino(4'-CH₂-N(R₃)-O-2') BNA nucleoside (also known as 2',4'-BNA^{NC} nucleoside; R=H is 2',4'-BNA^{NC}[N-H] nucleoside, R=Me is 2',4'-BNA^{NC}[N-Me] nucleoside), 2',4'-BNA^{coc} nucleoside, 3'-amino-2',4'-BNA nucleoside, 5'-methyl BNA nucleoside, (4'-CH(CH₃)-O-2') BNA nucleoside (also known as cEt nucleoside), (4'-CH(CH₂OCH₃)-O-2') BNA nucleoside (also known as cMOE nucleoside), amide BNA nucleoside, (4'-C(O)-N(R)-2') BNA nucleoside (R=H, Me) (also known as AmNA nucleoside; R=H is AmNA[N-H] nucleoside, R=Me is AmNA[N-Me] nucleoside)), guanidine BNA nucleoside (also known as GuNA nucleoside (e.g. R=H is GuNA[N-H] nucleoside, R=Me is GuNA[N-Me] nucleoside in Figure 4)), amine BNA nucleoside (also known as 2'-Amino-LNA nucleoside) (e.g., 3-(Bis(3-aminopropyl)amino)propanoyl substituted nucleoside), 2'-0,4'-C-spirocyclopropylene-bridged nucleoside (also known as scpBNA nucleoside), and other BNA nucleosides known to those skilled in the art.

The "cationic nucleoside" herein is a modified nucleoside existing in the cationic form as compared to the neutral form *(e.g.,* neutral form of ribonucleoside) at a certain pH *(e.g.,* human physiological pH (about 7.4) or pH of a delivery site (e.g., organelles, cells, tissues, organs, organisms)). The cationic nucleoside may comprise one or more cationic modified groups at any position of a nucleoside. In an embodiment, the cationic nucleoside is 2'-Amino-LNA nucleoside (e.g., 3-(Bis(3-aminopropyl) amino) propanoyl-substituted nucleoside), aminoalkyl-modified nucleoside (e.g., 2'-O-methyl and 4'-CH₂CH₂CH₂NH₂-substituted nucleoside), GuNA nucleoside (e.g. R=H is GuNA[N-H] nucleoside, R=Me is GuNA[N-Me] nucleoside in Figure 3), or the like.

A bicyclic nucleoside having a methyleneoxy (4'-CH₂-O-2') bridge may be referred to as LNA nucleoside.

The method for preparing a modified sugar is well known to those skilled in the art. In a nucleotide having a modified sugar moiety, the nucleobase moiety (natural one, modified one, or a combination thereof) may be maintained for hybridization with an appropriate nucleic acid target.

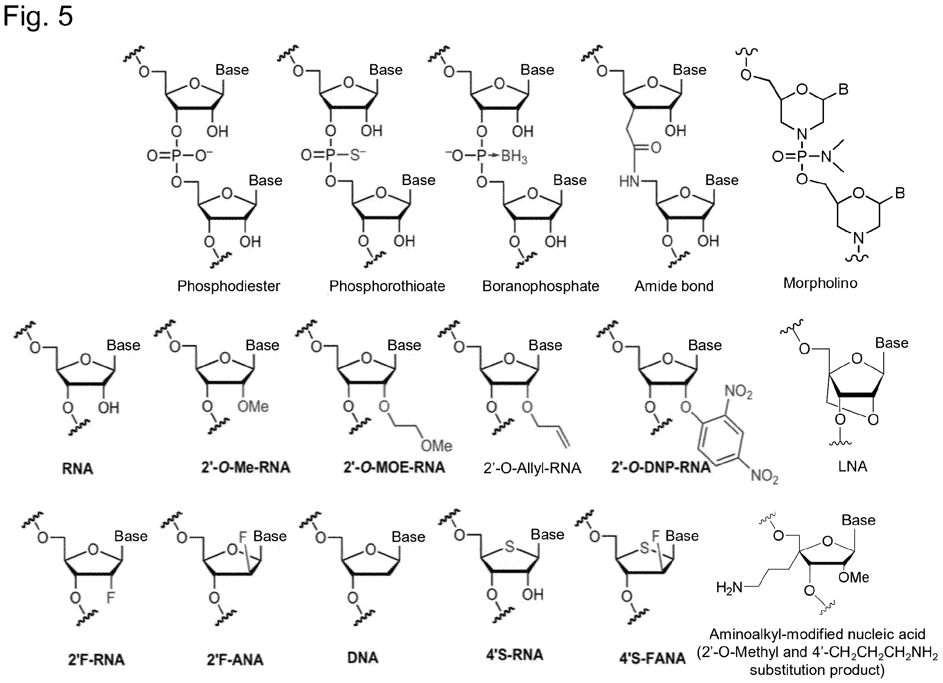

The "nucleoside mimic" includes a structure used for replacing a sugar, or a sugar and a base, and, but not mandatorily, a bond at one or more positions of an oligomeric compound. The term "oligomeric compound" means a polymer of linked monomeric subunits capable of hybridizing to at least a region of a nucleic acid molecule. Examples of a nucleoside mimic include, for example, morpholino, cyclohexenyl, cyclohexyl, tetrahydropyranyl, bicyclic or tricyclic sugar mimic, *e.g.,* a nucleoside mimic having a non-furanose sugar unit. The "nucleotide mimic" includes a structure used for replacing a nucleoside and a bond at one or more positions of an oligomeric compound. Examples of a nucleotide mimic include a peptide nucleic acid, and a morpholino nucleic acid (a morpholino linked by -N(H)-C(=O)-O- or other non-phosphodiester bonds). A peptide nucleic acid (PNA) is a nucleotide mimic having a main chain in which N-(2-aminoethyl) glycine in place of a sugar is linked by an amide bond. An example of the structure of a morpholino nucleic acid is shown in Figure 5. A "mimic" refers to a group that replaces one or more of a sugar, a nucleobase, and an internucleoside bond. In general, a mimic is used in place of a sugar or a combination of sugar and an internucleoside bond, and a nucleobase is maintained for hybridization to a selected target.

In general, modifications can be performed such that nucleotides in the same strand can independently undergo different modifications. In addition, to confer resistance to enzymatic cleavage, the same nucleotide may have a modified internucleoside bond *(e.g.,* phosphorothioate bond), and also have a modified sugar *(e.g.,* 2'-O-methyl-modified sugar, or bicyclic sugar). Further, the same nucleotide can have a modified nucleobase (e.g., 5-methylcytosine) and further have a modified sugar (e.g., 2'-O-methyl-modified sugar, or bicyclic sugar).

The number, type, and position of a non-natural nucleotide in a nucleic acid strand can influence the antisense effect or the like provided by a nucleic acid complex. Selection of a modification may vary depending on the sequence of a target gene or the like, but one skilled in the art can determine a suitable embodiment by referring to the description of literature related to the antisense method *(e.g.,* WO 2007/143315, WO 2008/043753, and WO 2008/049085). Furthermore, when the antisense effect of the nucleic acid complex after the modification is measured, if the measurement value thus obtained is not significantly lower than the measurement value of the nucleic acid complex before the modification (for example, in a case where the measurement value obtained after the modification are 70% or more, 80% or more, or 90% or more of the measurement value of the nucleic acid complex before the modification), a relevant modification can be evaluated.

Measurement of an antisense effect may be performed, for example, by administering a test nucleic acid compound to a subject (e.g., mouse), and measuring the expression level of a target gene whose expression is modulated by the antisense effect provided by the test nucleic acid compound, or the level (amount) of the target transcriptional product (for example, the amount of mRNA, or the amount of RNA such as microRNA, the amount of cDNA, or the amount of protein), for example, several days after the administration (for example, after 2 to 7 days).

For example, in a case where the expression level of a target gene or the level of a target transcriptional product measured is reduced by at least 10%, at least 20%, at least 25%, at least 30%, or at least 40% as compared to the negative control (e.g., vehicle administration), it is demonstrated that the test nucleic acid compound can produce an antisense effect (e.g., reduction of target transcriptional product amount).

The first nucleic acid strand herein is a gapmer. In other words, the antisense oligonucleotide region in the first nucleic acid strand is an antisense oligonucleotide region of a gapmer type (a gapmer type antisense oligonucleotide region). A "gapmer type" refers to a nucleoside configuration consisting of a central region (DNA gap region) comprising at least four contiguous deoxyribonucleosides, and regions (5' wing region, and 3' wing region) which comprise non-natural nucleosides and are located respectively on the 5' end side and the 3' end side of the central region. A gapmer in which a non-natural nucleoside is constituted by a bridged nucleoside is specifically referred to as "BNA/DNA gapmer". The length of the DNA gap region may be: from 13 to 22 base in length, from 16 to 22 base in length, or from 16 to 20 base in length; or from 4 to 20 base in length, from 5 to 18 base in length, from 6 to 16 base in length, from 7 to 14 base in length, or from 8 to 12 base in length. In addition, the central region of a gapmer may comprise or consist of a natural nucleoside, an unmodified sugar in which the sugar portion of nucleic acid is not modified (and a modified or unmodified internucleoside bond), for example, it comprises a natural nucleoside which is from 13 to 22 base in length, from 16 to 22 base in length, or from 16 to 20 base in length, or from 4 to 20 base in length, from 5 to 18 base in length, from 6 to 16 base in length, from 7 to 14 base in length, or from 8 to 12 base in length or an unmodified sugar. The central region may comprise or consist of, for example, natural nucleosides linked by a modified internucleoside bond such as a phosphorothioate bond.

The lengths of the 5' wing region and the 3' wing region may be usually from 1 to 10 base in length, from 1 to 7 base in length, from 2 to 5 base in length, or from 2 to 3 base in length, independently. The 5' wing region and the 3' wing region may comprise at least one kind of non-natural nucleoside and may further comprise a natural nucleoside. The gapmer type antisense oligonucleotide region may have a nucleoside configuration of BNA/DNA gapmer type including a 5' wing region comprising two or three bridged nucleosides, a 3' wing region comprising two or three bridged nucleosides, and a DNA gap region therebetween. A bridged nucleoside may comprise a modified nucleobase (e.g., 5-methylcytosine). In addition, the gapmer may be "LNA/DNA gapmer" in which a bridged nucleoside is constituted by an LNA nucleoside. The 5' wing region and the 3' wing region may be, for example, non-natural nucleosides linked by a modified internucleoside bond such as a phosphorothioate bond, for example, 2'-O-methyl modified nucleosides. In an embodiment, nucleosides in the first nucleic acid strand may comprise or consist of deoxyribonucleosides; for example, 70% or more, 80% or more, 90% or more, or 95% or more of nucleosides in the first nucleic acid strand are deoxyribonucleosides.

The internucleoside bond in the first nucleic acid strand may be a naturally occurring internucleoside bond and/or a modified internucleoside bond.

At least one, at least two, or at least three internucleoside bonds from the 5' end of the first nucleic acid strand may be modified internucleoside bonds. At least one, at least two, or at least three internucleoside bonds from the 3' end of the first nucleic acid strand may be modified internucleoside bonds. For example, two internucleoside bonds from an end of a nucleic acid strand refers to an internucleoside bond closest to the end of the nucleic acid strand, and an internucleoside bond positioned next thereto in the direction opposite to the end of the nucleic acid strand. Modified internucleoside bond(s) at the terminal region of a nucleic acid strand are preferred because they can reduce or inhibit undesired degradation of the nucleic acid strand.

Modified internucleoside bond(s) may be at least 70%, at least 80%, at least 85%, at least 90%, at least 93%, at least 95%, at least 98%, or 100% of the internucleoside bonds of the antisense oligonucleotide region in the first nucleic acid strand. A modified internucleoside bond may be a phosphorothioate bond.

The nucleoside in the first nucleic acid strand may be a natural nucleoside (including deoxyribonucleoside, ribonucleoside, or both) and/or a non-natural nucleoside.

The second nucleic acid strand comprises at least one, for example, 1 to 5, 1 to 4, 1 to 3, 1 to 2, or one sugar-unmodified central region (first exposed region) complementary to a part of the first nucleic acid strand *(e.g.,* a part of the central region of the first nucleic acid strand), or at least two thereof. The sugar-unmodified central region is mainly herein referred to as "first exposed region".

The first exposed region may be completely complementary to a part of the first nucleic acid strand, or may have one or more mismatched bases. For example, the first exposed region may have 1 to 3, 1 to 2, or one mismatched base (non-complementary base) with respect to a part of the first nucleic acid strand.

The first exposed region consists of one sugar-unmodified ribonucleoside or two or three contiguous sugar-unmodified ribonucleosides linked by an internucleoside bond. The "sugar-unmodified ribonucleoside" herein refers to a ribonucleoside in which the sugar moiety of the natural ribonucleoside does not comprise a sugar modification such as a substitution at the 2' position and/or any change. The first exposed region may comprise at least one modified base and/or modified internucleoside bond.

In an embodiment, sugar-unmodified ribonucleosides in the first exposed region include a natural ribonucleoside; for example, all of sugar-unmodified ribonucleosides are natural ribonucleosides, or some of them, for example, one or two sugar-unmodified ribonucleosides are natural ribonucleosides.

In an embodiment, the second nucleic acid strand comprise at least one, for example, 1 to 10, 1 to 5, 1 to 4, 1 to 3, 1 to 2, or one, at least two, for example, 2 to 10, 2 to 5, 3 to 4, 2 to 3, or 2, or at least three, for example, 3 to 10, 3 to 5, 3 to 4, or three first exposed regions.

In an embodiment, the first exposed region consists of two or three contiguous sugar-unmodified ribonucleosides linked by an internucleoside bond. In an embodiment, the first exposed region consists of three contiguous sugar-unmodified ribonucleosides linked by an internucleoside bond. This embodiment may have an effect of maintaining higher RNase H cleavage activity than that of the first exposed region consisting of one sugar-unmodified ribonucleoside or two contiguous sugar-unmodified ribonucleosides. The central region of this embodiment may have an effect of maintaining/improving the activity in the RNase H-dependent and/or independent pathway as compared to the central region consisting of sugar-unmodified ribonucleosides.

In an embodiment, the first exposed region comprises a nucleoside comprising a modified or unmodified purine base. This embodiment may have an effect of suppressing RNase A cleavage activity at a low level while maintaining RNase H cleavage activity as compared to the first exposed region which does not comprise a nucleoside comprising a purine base (e.g., consisting of a nucleoside comprising a pyrimidine base). This embodiment may have an effect of maintaining/improving the activity in the RNase H-dependent and/or independent pathway as compared to the first exposed region which does not comprise a nucleoside comprising a purine base (e.g., consisting of a nucleoside comprising a pyrimidine base).

In an embodiment, the second nucleic acid strand comprises at least one, for example, 1 to 10, 1 to 5, 1 to 4, 1 to 3, 1 to 2, or one, at least two, for example, 2 to 10, 2 to 5, 3 to 4, 2 to 3, or 2, or at least three, for example, 3 to 10, 3 to 5, 3 to 4, or three protected regions.

The "protected region" herein refers to a region resistant to cleavage by a nuclease. Here, the nuclease is not limited. An example of a nuclease is RNase A or RNase H. The protected region may be, for example, a region resistant to RNase A, a region resistant to RNase H, or a region resistant to both RNase A and RNase H. In addition, resistance to cleavage by a nuclease is not necessarily complete resistance, and it is sufficient that the region has the same base sequence and increased resistance than the region comprising unmodified ribonucleosides.

The protected region consists of one of (a) a deoxyribonucleoside; (b) a sugar-modified nucleoside; and/or (c) a nucleoside having a modified internucleoside bond on the 3' side, or two or more of said (a) to (c) linked by an internucleoside bond. The protected region may comprise at least one modified base and/or modified internucleoside bond. A deoxyribonucleoside and/or sugar-modified nucleoside in the protected region is preferably a deoxyribonucleoside, a nucleoside modified at the 2'-position of the sugar (e.g., nucleoside comprising a 2'-O-methyl group), bridged nucleoside (e.g., LNA nucleoside), and/or a cationic nucleoside, more preferably a deoxyribonucleoside, a nucleoside modified at the 2'-position of the sugar (e.g., nucleoside comprising a 2'-O-methyl group), and/or a bridged nucleoside (e.g., LNA nucleoside), still more preferably a deoxyribonucleoside and/or a nucleoside modified at the 2'-position of the sugar (e.g., nucleoside comprising a 2'-O-methyl group), and particularly preferably a deoxyribonucleoside and/or a nucleoside comprising a 2'-O-methyl group. A nucleoside having a modified internucleoside bond on the 3' side in the protected region is preferably a deoxyribonucleoside, ribonucleoside, a nucleoside modified at the 2'-position of the sugar (e.g., nucleoside comprising a 2'-O-methyl group), a bridged nucleoside (e.g., LNA nucleoside), and/or a cationic nucleoside, more preferably a deoxyribonucleoside, a ribonucleoside, a nucleoside modified at the 2'-position of the sugar *(e.g.,* nucleoside comprising a 2'-O-methyl group), and/or a bridged nucleoside *(e.g.,* LNA nucleoside), still more preferably a deoxyribonucleoside, ribonucleoside, and/or a nucleoside modified at the 2'-position of the sugar (e.g., nucleoside comprising 2'-O-methyl group), yet more preferably a deoxyribonucleoside, ribonucleoside, and/or a nucleoside comprising a 2'-O-methyl group, and particularly preferably a ribonucleoside. A nucleoside having a modified internucleoside bond on the 3' side in the protected region is preferably a nucleoside having a phosphorothioate bond on the 3' side, a phosphorothioate bond, an internucleoside bond comprising a guanidine moiety substituted with one to four C₁ to C₆ alkyl groups (preferably a TMG moiety) (preferably a partial structure represented by Formula (III)), and/or an internucleoside bond comprising a cyclic guanidine moiety (preferably a partial structure represented by Formula (II)), and more preferably a nucleoside having a phosphorothioate bond on the 3' side. A modified internucleoside bond on the 3' side may be chirally controlled in an Rp or Sp configuration. The nucleoside having a modified internucleoside bond on the 3' side herein refers to a nucleoside having a modified internucleoside bond on at least the 3' side, which may optionally have a modified internucleoside bond on the 5' side as well.

The protected region may be completely complementary to a part of the first nucleic acid strand *(e.g.,* a part of the central region of the first nucleic acid strand), or may have one or more mismatched bases. For example, the protected region may have 1 to 3, 1 to 2, or one mismatched base (non-complementary base) with respect to a part of the first nucleic acid strand.

In an embodiment, the protected region comprises: (a) a deoxyribonucleoside; (b) a sugar-modified nucleoside; and/or (c) a nucleoside having a modified internucleoside bond on the 3' side, each comprising a pyrimidine base. Here, the pyrimidine base may be either modified or unmodified. For example, at least one protected region or all protected regions may comprise: (a) a deoxyribonucleoside; (b) a sugar-modified nucleoside; and/or (c) a nucleoside having a modified internucleoside bond on the 3' side, each comprising a modified or unmodified pyrimidine base. This embodiment may have an effect of suppressing RNase A cleavage activity on the second nucleic acid strand at a low level as compared to when a sugar-unmodified region *(e.g.,* a first exposed region or a second exposed region) comprises a nucleoside comprising a pyrimidine base. This embodiment may have an effect of maintaining/improving the activity in the RNase H-dependent and/or independent pathway as compared to when a sugar-unmodified region *(e.g.,* a first exposed region or a second exposed region) comprises a nucleoside comprising a pyrimidine base.

In an embodiment, a sugar-modified nucleoside in the protected region is a sugar-modified ribonucleoside.

In an embodiment, the second nucleic acid strand consists of a first exposed region and a protected region. In a further embodiment, a first exposed region and a protected region are arranged alternately in the second nucleic acid strand. In a further embodiment, at least one protected region or all protected regions comprise: (a) a deoxyribonucleoside; (b) a sugar-modified nucleoside; and/or (c) a nucleoside having a modified internucleoside bond on the 3' side, each comprising a modified or unmodified pyrimidine base.

In an aspect, the second nucleic acid strand comprises at least one, for example, 1 to 5, 1 to 4, 1 to 3, 1 to 2, or one, or at least two second exposed regions complementary to a part of the first nucleic acid strand.

The second exposed region consists of four or more contiguous sugar-unmodified ribonucleosides linked by an internucleoside bond, and the bases of sugar-unmodified ribonucleosides in the second exposed region comprise a purine base. Here, the purine base may be either modified or unmodified. The number of sugar-unmodified ribonucleosides constituting the second exposed region may be, for example, from 4 to 10, from 4 to 9, from 4 to 8, from 4 to 7, from 4 to 6, from 4 to 5, or 4. The second exposed region may comprise at least one modified base and/or modified internucleoside bond.

The second exposed region may be completely complementary to a part of the first nucleic acid strand *(e.g.,* a part of the central region of the first nucleic acid strand), or may have one or more mismatched bases. For example, the second exposed region may have 1 to 3, 1 to 2, or one mismatched base (non-complementary base) with respect to a part of the first nucleic acid strand.

In an embodiment, sugar-unmodified ribonucleosides in the second exposed region comprise a natural ribonucleoside; for example, all sugar-unmodified ribonucleosides are natural ribonucleosides, or some of them, for example, one or two sugar-unmodified ribonucleosides are natural ribonucleosides.

In an embodiment, the second nucleic acid strand comprises at least one, for example, 1 to 10, 1 to 5, 1 to 4, 1 to 3, 1 to 2, or one, at least two, for example, 2 to 10, 2 to 5, 3 to 4, 2 to 3, or two, or at least three, for example, 3 to 10, 3 to 5, 3 to 4, or three second exposed regions.

In an embodiment, the second nucleic acid strand comprises at least one, for example, 1 to 10, 1 to 6, 1 to 5, 1 to 4, 1 to 3, 1 to 2, or one, at least two, for example, 2 to 10, 2 to 5, 3 to 4, 2 to 3, or two, or at least three, for example, 3 to 10, 3 to 5, 3 to 4, or three first exposed regions and/or second exposed regions in total.

In an embodiment, the second nucleic acid strand consists of a first exposed region, a second exposed region, and a protected region. In a further embodiment, a first exposed region or a second exposed region, and a protected region are arranged alternately in the second nucleic acid strand. In a further embodiment, at least one protected region or all protected regions comprise: (a) a deoxyribonucleoside; (b) a sugar-modified nucleoside; and/or (c) a nucleoside having a modified internucleoside bond on the 3' side, each comprising a modified or unmodified pyrimidine base.

In an embodiment, the second nucleic acid strand consists of a second exposed region and a protected region. In a further embodiment, a second exposed region and a protected region are arranged alternately in the second nucleic acid strand. In a further embodiment, at least one protected region or all protected regions comprise: (a) a deoxyribonucleoside; (b) a sugar-modified nucleoside; and/or (c) a nucleoside having a modified internucleoside bond on the 3' side, each comprising a modified or unmodified pyrimidine base.

In an embodiment, at least one protected region in the second nucleic acid strand is linked by an internucleoside bond to the first exposed region and/or the second exposed region at the 5' end and the 3' end.

In an embodiment, the first exposed region and/or the second exposed region comprise at least one, for example, one or two modified internucleoside bonds, for example, a phosphorothioate bond such as a phosphorothioate bond chirally controlled in an Rp or Sp configuration, an internucleoside bond *(e.g.,* a partial structure represented by Formula (III)) comprising a guanidine moiety substituted with one to four C₁ to C₆ alkyl groups *(e.g.,* a TMG moiety), and/or an internucleoside bond *(e.g.,* a partial structure represented by Formula (II)) comprising a cyclic guanidine moiety.

In an embodiment, the second nucleic acid strand comprises or consists of: (1) aforementioned first exposed region; and/or (2) aforementioned second exposed region; and (3)(a) a deoxyribonucleoside and/or (b) a sugar-modified nucleoside such as a sugar-modified ribonucleoside, which are linked by a modified or unmodified internucleoside bond.

In an embodiment, the second nucleic acid strand further comprises the following sugar-unmodified terminal region at the 5' end and/or the 3' end. In an embodiment, the lengths of the sugar-unmodified terminal region at the 5' end and/or the 3' end are usually from 1 to 10 base in length, from 1 to 7 base in length, from 2 to 5 base in length, or from 2 to 3 base in length, independently. In an embodiment, the second nucleic acid strand comprises or consists of aforementioned first exposed region and/or the second exposed region and a sugar-modified ribonucleoside (and optionally a sugar-unmodified terminal region), which are linked by a modified or unmodified internucleoside bond.

In an embodiment, the second nucleic acid strand comprises a sugar-unmodified terminal region comprising at least one, for example, 1 to 6, 1 to 5, 1 to 4, 1 to 3, 1 to 2, or one or at least two sugar-unmodified ribonucleosides at the 5' end and/or the 3' end. The more sugar modifications, the more toxic the nucleic acid complex tends to be. Therefore, by making the terminal region sugar-unmodified, the number of sugar-modified nucleic acids in the second nucleic acid strand can be reduced, resulting in reduced toxicity. The sugar-unmodified terminal region may comprise a modified base and/or a modified internucleoside bond. In an embodiment, the sugar-unmodified terminal region is complementary to a portion of the first nucleic acid strand *(e.g.,* the 5' wing region, the central region, the 3' wing region, or a portion thereof of the first nucleic acid strand). The sugar-unmodified terminal region may be completely complementary to a portion of the first nucleic acid strand, or may have one or more mismatched bases. For example, the second exposed region may have 1 to 3, 1 to 2, or one mismatched base (non-complementary base) with respect to a part of the first nucleic acid strand. In an embodiment, sugar-unmodified ribonucleosides in the sugar-unmodified terminal region comprise a natural ribonucleoside; for example, all sugar-unmodified ribonucleosides are natural ribonucleosides, or some of them, for example, 1 to 5, 1 to 4, 1 to 3, or one or two sugar-unmodified ribonucleosides are natural ribonucleosides. In an embodiment, the sugar-unmodified terminal region comprises a nucleoside comprising a modified or unmodified purine base and/or a modified or unmodified pyrimidine base. In an embodiment, the sugar-unmodified terminal region comprises a nucleoside comprising a modified or unmodified purine base.

In an embodiment, the second nucleic acid strand consists of: (1) at least one first exposed region and/or at least one second exposed region; (2) at least one protected region; and (3) sugar-unmodified terminal regions at the 5' end and the 3' end, which are linked by a modified or unmodified internucleoside bond. In an embodiment, the second nucleic acid strand consists of: (1) 1 to 10 first exposed regions and/or 1 to 10 second exposed regions; (2) 1 to 10 protected regions; and (3) sugar-unmodified terminal regions at the 5' end and the 3' end, which are linked by a modified or unmodified internucleoside bond. In an embodiment, the second nucleic acid strand consists of: (1) 1 to 5 first exposed regions and/or 1 to 5 second exposed regions; (2) 1 to 5 protected regions; and (3) sugar-unmodified terminal regions at the 5' end and the 3' end, which are linked by a modified or unmodified internucleoside bond. In an embodiment, the second nucleic acid strand consists of: (1) 1 to 3 first exposed regions and/or 1 to 3 second exposed regions; (2) 1 to 3 protected regions; and (3) sugar-unmodified terminal regions at the 5' end and the 3' end, which are linked by a modified or unmodified internucleoside bond. In an embodiment, the second nucleic acid strand consists of: (1) 2 to 10 first exposed regions and/or second exposed regions; (2) 2 to 10 protected regions; and (3) sugar-unmodified terminal regions at the 5' end and the 3' end, which are linked by a modified or unmodified internucleoside bond. In an embodiment, the second nucleic acid strand consists of: (1) 2 to 6 first exposed regions and/or second exposed regions; (2) 2 to 6 protected regions; and (3) sugar-unmodified terminal regions at the 5' end and the 3' end, which are linked by a modified or unmodified internucleoside bond. In an embodiment, the second nucleic acid strand consists of: (1) 2 to 5 first exposed regions and/or second exposed regions; (2) 2 to 5 protected regions; and (3) sugar-unmodified terminal regions at the 5' end and the 3' end, which are linked by a modified or unmodified internucleoside bond. In an embodiment, the second nucleic acid strand consists of: (1) 2 to 4 first exposed regions and/or second exposed regions; (2) 2 to 4 protected regions; and (3) sugar-unmodified terminal regions at the 5' end and the 3' end, which are linked by a modified or unmodified internucleoside bond. In an embodiment, the second nucleic acid strand consists of: (1) 2 to 3 first exposed regions and/or second exposed regions; (2) 2 to 3 protected regions; and (3) sugar-unmodified terminal regions at the 5' end and the 3' end, which are linked by a modified or unmodified internucleoside bond. In an embodiment, the second nucleic acid strand consists of: (1) 3 to 10 first exposed regions and/or second exposed regions; (2) 3 to 10 protected regions; and (3) sugar-unmodified terminal regions at the 5' end and the 3' end, which are linked by a modified or unmodified internucleoside bond. In an embodiment, the second nucleic acid strand consists of: (1) 3 to 6 first exposed regions and/or second exposed regions; (2) 3 to 6 protected regions; and (3) sugar-unmodified terminal regions at the 5' end and the 3' end, which are linked by a modified or unmodified internucleoside bond. In an embodiment, the second nucleic acid strand consists of: (1) 3 to 5 first exposed regions and/or second exposed regions; (2) 3 to 5 protected regions; and (3) sugar-unmodified terminal regions at the 5' end and the 3' end, which are linked by a modified or unmodified internucleoside bond. In an embodiment, the second nucleic acid strand consists of: (1) 3 to 4 first exposed regions and/or second exposed regions; (2) 3 to 4 protected regions; and (3) sugar-unmodified terminal regions at the 5' end and the 3' end, which are linked by a modified or unmodified internucleoside bond. In this embodiment, (a) a first exposed region or a second exposed region and (b) a protected region are arranged alternately in the second nucleic acid strand.

In an embodiment, the second nucleic acid strand comprises a sugar-modified terminal region comprising at least one deoxyribonucleoside, sugar-modified nucleoside, and/or nucleoside having a modified internucleoside bond on the 3' side, at the 5' end and/or the 3' end. In an embodiment, the second nucleic acid strand comprises a sugar-modified terminal region comprising at least one deoxyribonucleoside and/or sugar-modified nucleoside at the 5' end and/or the 3' end.

In an embodiment, the sugar-modified terminal region is complementary to a part of the first nucleic acid strand *(e.g.,* the 5' wing region, the central region, the 3' wing region, or part thereof of the first nucleic acid strand). The sugar-modified terminal region may be completely complementary to a part of the first nucleic acid strand, or may have one or more mismatched bases. For example, the second exposed region may have 1 to 3, 1 to 2, or one mismatched base (non-complementary base) with respect to a part of the first nucleic acid strand. In an embodiment, the sugar-modified terminal region comprises a deoxyribonucleoside, a sugar-modified nucleoside, and/or a nucleoside having a modified internucleoside bond on the 3' side, each comprising a pyrimidine base. Here, the pyrimidine base may be either modified or unmodified. In an embodiment, the sugar-modified terminal region comprises a deoxyribonucleoside, a sugar-modified nucleoside, and/or a nucleoside having a modified internucleoside bond on the 3' side, each comprising a purine base. In an embodiment, the sugar-modified terminal region comprises a deoxyribonucleoside and/or sugar-modified nucleoside comprising a purine base.

Here, the purine base mentioned may be either modified or unmodified.

In an embodiment, the second nucleic acid strand comprises or consists of aforementioned first exposed region, second exposed region, the protected region, and/or the sugar-modified terminal region, which are linked by a modified or unmodified internucleoside bond. In an embodiment, the lengths of the sugar-modified terminal region at the 5' end and/or the 3' end are usually from 1 to 10 base in length, from 1 to 7 base in length, from 2 to 5 base in length, or from 2 to 3 base in length, independently.

In an embodiment, in the sugar-modified terminal region, all nucleosides are deoxyribonucleosides, sugar-modified nucleosides, and/or nucleosides having a modified internucleoside bond on the 3' side (preferably, all nucleosides are deoxyribonucleosides and/or sugar-modified nucleosides), or some of them, for example, one, 1 to 2, 1 to 3, 1 to 4, 1 to 5, 1 to 6, or 1 to 7 nucleosides are deoxyribonucleosides, sugar-modified nucleosides, and/or nucleosides having a modified internucleoside bond on the 3' side (preferably, all nucleosides are deoxyribonucleosides and/or sugar-modified nucleosides). In an embodiment, nucleosides in the sugar-modified terminal region comprise deoxyribonucleosides, sugar-modified nucleosides, and/or nucleosides having a modified internucleoside bond on the 3' side at any proportions (preferably, all nucleosides are deoxyribonucleosides and/or sugar-modified nucleosides). For example, 10% or more, 30% or more, 50% or more, 70% or more, 80% or more, 90% or more thereof are deoxyribonucleosides, sugar-modified nucleosides, and/or nucleosides having a modified internucleoside bond on the 3' side (preferably, all nucleosides are deoxyribonucleosides and/or sugar-modified nucleosides). A deoxyribonucleoside and/or sugar-modified nucleoside is preferably a deoxyribonucleoside, a nucleoside modified at the 2'-position of the sugar *(e.g.,* nucleoside comprising a 2'-O-methyl group), bridged nucleoside *(e.g.,* LNA nucleoside), and/or a cationic nucleoside, more preferably a deoxyribonucleoside, a nucleoside modified at the 2'-position of the sugar (e.g., nucleoside comprising a 2'-O-methyl group), and/or a bridged nucleoside (e.g., LNA nucleoside), still more preferably a deoxyribonucleoside, a nucleoside comprising a 2'-O-methyl group, and/or LNA nucleoside, and yet more preferably a deoxyribonucleoside and/or LNA nucleoside, and particularly preferably LNA nucleoside. A nucleoside having a modified internucleoside bond on the 3' side is preferably a nucleoside having a phosphorothioate bond on the 3' side.

In an embodiment, the second nucleic acid strand consists of: (1) at least one first exposed region and/or at least one second exposed region; (2) at least one protected region; and (3) sugar-modified terminal regions at the 5' end and the 3' end, which are linked by a modified or unmodified internucleoside bond. In an embodiment, the second nucleic acid strand consists of: (1) 1 to 10 first exposed regions and/or 1 to 10 second exposed regions; (2) 1 to 10 protected regions; and (3) sugar-modified terminal regions at the 5' end and the 3' end, which are linked by a modified or unmodified internucleoside bond. In an embodiment, the second nucleic acid strand consists of: (1) 1 to 5 first exposed regions and/or 1 to 5 second exposed regions; (2) 1 to 5 protected regions; and (3) sugar-modified terminal regions at the 5' end and the 3' end, which are linked by a modified or unmodified internucleoside bond. In an embodiment, the second nucleic acid strand consists of: (1) 1 to 4 first exposed regions and/or 1 to 4 second exposed regions; (2) 1 to 4 protected regions; and (3) sugar-modified terminal regions at the 5' end and the 3' end, which are linked by a modified or unmodified internucleoside bond. In an embodiment, the second nucleic acid strand consists of: (1) 1 to 3 first exposed regions and/or 1 to 3 second exposed regions; (2) 1 to 3 protected regions; and (3) sugar-modified terminal regions at the 5' end and the 3' end, which are linked by a modified or unmodified internucleoside bond. In an embodiment, the second nucleic acid strand consists of: (1) 2 to 5 first exposed regions and/or 1 to 5 second exposed regions; (2) 2 to 5 protected regions; and (3) sugar-modified terminal regions at the 5' end and the 3' end, which are linked by a modified or unmodified internucleoside bond. In an embodiment, the second nucleic acid strand consists of: (1) 2 to 4 first exposed regions and/or 1 to 4 second exposed regions; (2) 2 to 4 protected regions; and (3) sugar-modified terminal regions at the 5' end and the 3' end, which are linked by a modified or unmodified internucleoside bond. In an embodiment, the second nucleic acid strand consists of: (1) 2 to 3 first exposed regions and/or 1 to 3 second exposed regions; (2) 2 to 3 protected regions; and (3) sugar-modified terminal regions at the 5' end and the 3' end, which are linked by a modified or unmodified internucleoside bond. In an embodiment, the second nucleic acid strand consists of: (1) 3 to 5 first exposed regions and/or 1 to 5 second exposed regions; (2) 3 to 5 protected regions; and (3) sugar-modified terminal regions at the 5' end and the 3' end, which are linked by a modified or unmodified internucleoside bond. In an embodiment, the second nucleic acid strand consists of: (1) 3 to 4 first exposed regions and/or 1 to 4 second exposed regions; (2) 3 to 4 protected regions; and (3) sugar-modified terminal regions at the 5' end and the 3' end, which are linked by a modified or unmodified internucleoside bond. In an embodiment, the second nucleic acid strand consists of: (1) 2 to 10 first exposed regions and/or second exposed regions; (2) 2 to 10 protected regions; and (3) sugar-unmodified terminal regions at the 5' end and the 3' end, which are linked by a modified or unmodified internucleoside bond. In an embodiment, the second nucleic acid strand consists of: (1) 2 to 6 first exposed regions and/or second exposed regions; (2) 2 to 6 protected regions; and (3) sugar-unmodified terminal regions at the 5' end and the 3' end, which are linked by a modified or unmodified internucleoside bond. In an embodiment, the second nucleic acid strand consists of: (1) 2 to 5 first exposed regions and/or second exposed regions; (2) 2 to 5 protected regions; and (3) sugar-unmodified terminal regions at the 5' end and the 3' end, which are linked by a modified or unmodified internucleoside bond. In an embodiment, the second nucleic acid strand consists of: (1) 2 to 4 first exposed regions and/or second exposed regions; (2) 2 to 4 protected regions; and (3) sugar-unmodified terminal regions at the 5' end and the 3' end, which are linked by a modified or unmodified internucleoside bond. In an embodiment, the second nucleic acid strand consists of: (1) 2 to 3 first exposed regions and/or second exposed regions; (2) 2 to 3 protected regions; and (3) sugar-unmodified terminal regions at the 5' end and the 3' end, which are linked by a modified or unmodified internucleoside bond. In an embodiment, the second nucleic acid strand consists of: (1) 3 to 10 first exposed regions and/or second exposed regions; (2) 3 to 10 protected regions; and (3) sugar-unmodified terminal regions at the 5' end and the 3' end, which are linked by a modified or unmodified internucleoside bond. In an embodiment, the second nucleic acid strand consists of: (1) 3 to 6 first exposed regions and/or second exposed regions; (2) 3 to 6 protected regions; and (3) sugar-unmodified terminal regions at the 5' end and the 3' end, which are linked by a modified or unmodified internucleoside bond. In an embodiment, the second nucleic acid strand consists of: (1) 3 to 5 first exposed regions and/or second exposed regions; (2) 3 to 5 protected regions; and (3) sugar-unmodified terminal regions at the 5' end and the 3' end, which are linked by a modified or unmodified internucleoside bond. In an embodiment, the second nucleic acid strand consists of: (1) 3 to 4 first exposed regions and/or second exposed regions; (2) 3 to 4 protected regions; and (3) sugar-unmodified terminal regions at the 5' end and the 3' end, which are linked by a modified or unmodified internucleoside bond. In this embodiment, (a) a first exposed region or a second exposed region and (b) a protected region are arranged alternately in the second nucleic acid strand.

In an embodiment, a modified internucleoside bond in the first nucleic acid strand and/or the second nucleic acid strand comprises a non-negatively charged (neutral or cationic) internucleoside bond existing in the neutral form or the cationic form at a certain pH *(e.g.,* human physiological pH (about 7.4) or pH of a delivery site *(e.g.,* organelles, cells, tissues, organs, organisms)) as compared to when the modified internucleoside bond is in the anionic form *(e.g.,* -O-P(O)(O⁻)-O- (anionic form of natural phosphate bond) or -O-P(O)(S⁻)-O-((anionic form of phosphorothioate bond)). In an embodiment, a modified internucleoside bond in the first nucleic acid strand and/or the second nucleic acid strand comprises a neutral internucleoside bond. In an embodiment, a modified internucleoside bond in the first nucleic acid strand and/or the second nucleic acid strand comprises a cationic internucleoside bond. In an embodiment, a non-negatively charged internucleoside bond *(e.g.,* a neutral internucleoside bond) in the neutral form does not have a moiety with pKa less than 8, less than 9, less than 10, less than 11, less than 12, less than, less than 13, or less than 14. In an embodiment, a non-negatively charged internucleoside bond is, for example, a methylphosphonate bond disclosed in U.S. Patent Nos. 5,264,423 and 5,286,717, a methylphosphotriester bond and an ethylphosphotriester bond disclosed in U.S. Patent No. 5,955,599, a methoxypropylphosphonate bond disclosed in International Publication No. WO 2015/168172, an internucleoside bond used for a self-neutralizing nucleic acid (ZON) disclosed in International Publication No. WO 2016/081600, or the like. In an embodiment, a non-negatively charged internucleoside bond comprises a triazole moiety or an alkyne moiety. In an embodiment, a non-negatively charged internucleoside bond comprises a cyclic guanidine moiety and/or a guanidine moiety substituted with one to four C₁ to C₆ alkyl groups (preferably, a TMG moiety). In an embodiment, a modified internucleoside bond comprising a cyclic guanidine moiety has a partial structure represented by Formula (II). In an embodiment, the guanidine moiety substituted with one to four C₁ to C₆ alkyl groups has a partial structure represented by Formula (III). In an embodiment, a neutral internucleoside bond comprising a cyclic guanidine moiety and/or a guanidine moiety substituted with one to four C₁ to C₆ alkyl groups is chirally controlled. In an embodiment, the present disclosure relates to a composition comprising an oligonucleotide comprising at least one neutral internucleoside bond and at least one phosphorothioate internucleoside bond. Without wishing to be bound by any particular theory, at least in some cases, a neutral internucleotide bond can improve properties and/or activity as compared to comparable nucleic acids that do not comprise a neutral internucleotide bond; for example, it can improve delivery, resistance to exonuclease and endonuclease, cellular uptake, endosome escape, and/or nuclear uptake.

In an embodiment, the first nucleic acid strand and/or the second nucleic acid strand may respectively comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, or 50 or more modified internucleoside bonds. In an embodiment, the first nucleic acid strand and/or the second nucleic acid strand may respectively comprise modified internucleoside bonds at a proportion of at least 5%, at least 10%, at least 20%, at least 30%, at least 40%, at least 50%, at least 60%, at least 70%, at least 80%, or at least 90% or more.

In an embodiment, a first exposed region, a second exposed region, a protected region, a sugar-unmodified terminal region, and/or a sugar-modified terminal region of the second nucleic acid strand may respectively comprise one, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, or 1 to 22 modified internucleoside bonds. In an embodiment, a first exposed region, a second exposed region, a protected region, a sugar-unmodified terminal region, and/or a sugar-modified terminal region of the second nucleic acid strand may respectively comprise modified internucleoside bonds at a proportion of at least 5%, at least 10%, at least 20%, at least 30%, at least 40%, at least 50%, at least 60%, at least 70%, at least 80%, or at least 90% or more.

In an embodiment, the first nucleic acid strand and/or the second nucleic acid strand may respectively comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, or 50 or more chirally controlled internucleoside bonds. In an embodiment, the first nucleic acid strand and/or the second nucleic acid strand may respectively comprise chirally controlled internucleoside bonds at a proportion of at least 5%, at least 10%, at least 20%, at least 30%, at least 40%, at least 50%, at least 60%, at least 70%, at least 80%, or at least 90% or more.

In an embodiment, a first exposed region, a second exposed region, a protected region, a sugar-unmodified terminal region, and/or a sugar-modified terminal region of the second nucleic acid strand may respectively comprise one, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, or 1 to 22 chirally controlled internucleoside bonds. In an embodiment, a first exposed region, a second exposed region, a protected region, a sugar-unmodified terminal region, and/or a sugar-modified terminal region of the second nucleic acid strand may respectively comprise chirally controlled internucleoside bonds at a proportion of at least 5%, at least 10%, at least 20%, at least 30%, at least 40%, at least 50%, at least 60%, at least 70%, at least 80%, or at least 90% or more.

In an embodiment, the first nucleic acid strand and/or the second nucleic acid strand may respectively comprise 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, or 50 or more non-negatively charged internucleoside bonds (preferably, neutral internucleoside bonds). In an embodiment, the first nucleic acid strand and/or the second nucleic acid strand may respectively comprise non-negatively charged internucleoside bonds at a proportion of at least 5%, at least 10%, at least 20%, at least 30%, at least 40%, at least 50%, at least 60%, at least 70%, at least 80%, or at least 90% or more.

In an embodiment, a first exposed region, a second exposed region, a protected region, a sugar-unmodified terminal region, and/or a sugar-modified terminal region of the second nucleic acid strand may respectively comprise one, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, or 1 to 22 non-negatively charged internucleoside bonds (preferably, neutral internucleoside bonds). In an embodiment, a first exposed region, a second exposed region, a protected region, a sugar-unmodified terminal region, and/or a sugar-modified terminal region of the second nucleic acid strand may independently comprise non-negatively charged internucleoside bonds at a proportion of at least 5%, at least 10%, at least 20%, at least 30%, at least 40%, at least 50%, at least 60%, at least 70%, at least 80%, or at least 90% or more.

In an embodiment, any of the aforedescribed regions (a first exposed region, a protected region, a second exposed region, a sugar-unmodified terminal region, and/or a sugar-modified terminal region) in the second nucleic acid strand comprises at least one, for example, one or two, modified internucleoside bond, for example, a phosphorothioate bond, a phosphorothioate bond chirally controlled in an Rp or Sp configuration, an internucleoside bond *(e.g.,* a partial structure represented by Formula (III)) comprising a guanidine moiety substituted with one to four C₁ to C₆ alkyl groups *(e.g.,* a TMG moiety), an internucleoside bond *(e.g.,* a partial structure represented by Formula (III)) comprising a guanidine moiety chirally controlled in an Rp or Sp configuration which is substituted with one to four C₁ to C₆ alkyl groups *(e.g.,* a TMG moiety), an internucleoside bond *(e.g.,* a partial structure represented by Formula (II)) comprising a cyclic guanidine moiety, and/or a internucleoside bond *(e.g.,* a partial structure represented by Formula (II)) comprising a cyclic guanidine moiety chirally controlled in an Rp or Sp configuration inside the region or between two adjacent regions.

At least one, at least two, or at least three internucleoside bonds from the 5' end of the second nucleic acid strand may be modified internucleoside bonds. At least one, at least two, or at least three internucleoside bonds from the 3' end of the second nucleic acid strand may be a modified internucleoside bond such as a phosphorothioate bond, an internucleoside bond *(e.g.,* a partial structure represented by Formula (III)) comprising a guanidine moiety substituted with one to four C₁ to C₆ alkyl groups *(e.g.,* a TMG moiety), and/or an internucleoside bond *(e.g.,* a partial structure represented by Formula (II)) comprising a cyclic guanidine moiety. A modified internucleoside bond may be chirally controlled in Rp or Sp configuration.

The second nucleic acid strand may not comprise a sugar-unmodified region other than a first exposed region and/or a second exposed region (and, if present, a sugar-unmodified terminal region). In other words, the sugar of a nucleic acid in a region other than a first exposed region and/or a second exposed region (and, if present, a sugar-unmodified terminal region) is a modified sugar such as a 2'-modified sugar.

In an embodiment, a base in a region other than a first exposed region and/or a second exposed region (and, if present, a sugar-unmodified terminal region) comprises a modified and/or unmodified pyrimidine base. For example, all bases are modified and/or unmodified pyrimidine bases, or some of them, for example, 1 to 20, 1 to 15, 1 to 10, 1 to 5, 1 to 2, or one base is a modified and/or unmodified pyrimidine base. A modified and/or unmodified pyrimidine base is preferably cytosine, uracil, thymine, 5-methylcytosine, 5-fluorocytosine, 5-bromocytosine, 5-iodocytosine, N4-methylcytosine, and/or 2-thio-thymine, more preferably cytosine, uracil, thymine, and/or 5-methylcytosine, and particularly preferably cytosine.

In an embodiment, a base in a region other than a first exposed region and/or a second exposed region (and, if present, a sugar-unmodified terminal region) comprises a modified and/or unmodified pyrimidine base at any proportion. For example, a modified and/or unmodified pyrimidine base is 10% or more, 30% or more, 50% or more, 70% or more, 80% or more, or 90% or more. A modified and/or unmodified pyrimidine base is preferably cytosine, uracil, thymine, 5-methylcytosine, 5-fluorocytosine, 5-bromocytosine, 5-iodocytosine, N4-methylcytosine, and/or 2-thio-thymine, more preferably cytosine, uracil, thymine, and/or 5-methylcytosine, and particularly preferably cytosine.

In an embodiment, a base in a first exposed region comprises a purine base; for example, all bases are modified and/or unmodified purine bases, or some of them, for example, 1 or 2 bases are modified and/or unmodified purine bases. Since RNase A cleaves a phosphodiester bond on the 3' side of a modified and/or unmodified pyrimidine base, when a base in a first exposed region is a modified and/or unmodified pyrimidine base, this part can be cleaved by RNase A. Therefore, when a base in a first exposed region includes a modified and/or unmodified purine base, undesirable degradation by RNase A can be reduced. A base in a first exposed region does not comprise C, UC, or UU, which are modified and/or unmodified pyrimidine bases, and for example, does not comprise any of the three sequences.

In an embodiment, a base in a first exposed region comprises a modified or unmodified purine base. For example, all bases are modified and/or unmodified purine bases, or some of them, for example, 1 to 3, 1 to 2, or one base is a modified and/or unmodified purine base. In an embodiment, a base in a second exposed region comprises a modified and/or unmodified purine base. For example, all bases are modified and/or unmodified purine bases, or some of them, for example, 1 to 7, 1 to 6, 1 to 5, 1 to 4, 1 to 3, 1 to 2, or one base is a modified and/or unmodified purine base. In an embodiment, bases in a first exposed region and/or a second exposed region comprise modified and/or unmodified purine bases at any proportion. For example, a modified and/or unmodified pyrimidine base is 10% or more, 30% or more, 50% or more, 70% or more, 80% or more, or 90% or more. A modified and/or unmodified purine base is preferably adenine, guanine, N6-methyladenine, 8-bromoadenine, N2-methylguanine and/or 8-bromoadenine, and more preferably adenine and/or guanine.

In an embodiment, the second nucleic acid strand comprises a sugar-modified nucleoside and/or deoxyribonucleoside comprising a modified and/or unmodified pyrimidine base. For example, all bases are modified and/or unmodified pyrimidine bases, or some of them, for example, 1 to 20, 1 to 15, 1 to 10, 1 to 5, 1 to 2, or one base is a modified and/or unmodified pyrimidine base. In an embodiment, the second nucleic acid strand comprises a sugar-modified nucleoside and/or deoxyribonucleoside comprising a modified and/or unmodified pyrimidine base at any proportion. For example, 10% or more, 30% or more, 50% or more, 70% or more, 80% or more, or 90% or more of the second nucleic acid strand are pyrimidine bases. A pyrimidine base subject to sugar modification is preferably cytosine, uracil, thymine, 5-methylcytosine, 5-fluorocytosine, 5-bromocytosine, 5-iodocytosine, N4-methylcytosine, and/or 2-thio-thymine, more preferably cytosine, uracil, thymine, and/or 5-methylcytosine, and particularly preferably cytosine.

In an embodiment, a protected region in the second nucleic acid strand comprises a modified and/or unmodified pyrimidine base. For example, all bases are modified and/or unmodified pyrimidine bases, or some of them, for example, 1 to 20, 1 to 15, 1 to 10, 1 to 5, 1 to 2, or one base is a modified and/or unmodified pyrimidine base. In an embodiment, a protected region in the second nucleic acid strand comprises a modified and/or unmodified pyrimidine base. For example, 10% or more, 30% or more, 50% or more, 70% or more, 80% or more, or 90% or more of the second nucleic acid strand are pyrimidine bases. A pyrimidine with sugar modification is preferably cytosine, uracil, thymine, 5-methylcytosine, 5-fluorocytosine, 5-bromocytosine, 5-iodocytosine, N4-methylcytosine, and/or 2-thio-thymine, more preferably cytosine, uracil, thymine, and/or 5-methylcytosine, and particularly preferably cytosine.

The positions of a first exposed region and/or a second exposed region in the second nucleic acid strand are not limited. In an embodiment, at least one, for example, all of the first exposed regions and/or second exposed regions are located on the 3' side relative to the center of the second nucleic acid strand. Here, the phrase that "first exposed regions and/or second exposed regions are located on the 3' side relative to the center of the second nucleic acid strand" encompasses that the center, the 5' end, or the 3' end of a first exposed region and/or a second exposed region is located on the 3' side of the center of the second nucleic acid strand.

In an embodiment, a first exposed region and/or a second exposed region (and, if present, a sugar-unmodified terminal region) can be cleaved by RNase H. It is possible to determine whether or not it is cleaved by RNase H, *e.g.,* by reacting a nucleic acid strand with RNase H, performing electrophoresis, and measuring the density of uncleaved band. The details are as described in the Examples. The finding that a first exposed region consisting of one sugar-unmodified ribonucleoside or two or more contiguous sugar-unmodified ribonucleosides linked by an internucleoside bond can be cleaved by RNase H was surprising considering the common technical knowledge that the RNase H cleavage activity requires at least four contiguous natural ribonucleosides (see, *e.g.,* Figure 3 in FEBS J 2008; 276(6): 1494-505). In an embodiment, a first exposed region and/or a second exposed region (and, if present, a sugar-unmodified terminal region) can be active in the RNase H-dependent and/or independent pathway.

In an embodiment, a first nucleic acid strand and/or a second nucleic acid strand may comprise a cationic nucleoside in its entirety or in part. In an embodiment, a cationic nucleoside is a nucleoside including 2'-Amino-LNA nucleoside (e.g., 3-(Bis(3-aminopropyl) amino) propanoyl-substituted nucleoside), aminoalkyl-modified nucleoside *(e.g.,* 2'-O-methyl and 4'-CH₂CH₂CH₂NH₂-substituted nucleoside), GuNA nucleoside (e.g., R=H is GuNA[N-H] nucleoside, R=Me is GuNA[N-Me] nucleoside in Figure 4), or the like.

In an embodiment, a functional moiety may be bound to the second nucleic acid strand. The binding between the second nucleic acid strand and a functional moiety may be a direct binding or an indirect binding mediated by another substance. However, in one embodiment, it is preferable that the functional moiety is directly conjugated to the second nucleic acid strand via a covalent bond, an ionic bond, a hydrogen bond, or the like, and from the viewpoint that a more stable binding can be obtained, a covalent bond is more preferable.

There is no particular restriction on the structure of a "functional moiety" in one embodiment, and the double-stranded nucleic acid complex to which the moiety is bound is provided with a desired function. Examples of the desired function include a labeling function, a purifying function, and a function for delivery to a target. Examples of a moiety to confer a labeling function include a compound such as a fluorescent protein or luciferase. Examples of a moiety to confer a purifying function include a compound such as biotin, avidin, His-tag peptide, GST-tag peptide, or FLAG-tag peptide. In an embodiment, from the viewpoint of delivering the first nucleic acid strand to the target site with high specificity and efficiency and inhibiting the expression of the target gene very effectively by the nucleic acid, it is preferable that a molecule having the activity of delivering a double-stranded nucleic acid complex to the target site in an embodiment is conjugated as a functional moiety to the second nucleic acid strand. Examples of a moiety to confer a function for delivery to a target include a lipid, an antibody, an aptamer, and a ligand for a specific receptor. In an embodiment, the first nucleic acid strand and/or the second nucleic acid strand (preferably, the second nucleic acid strand) is conjugated to a functional moiety.

In an embodiment, the first nucleic acid strand and/or the second nucleic acid strand is conjugated to a lipid. Examples of the lipid include, but are not limited to: tocopherol, cholesterol, fatty acid, phospholipid and analogs thereof; folic acid, vitamin C, vitamin B1, vitamin B2; estradiol, androstane, and analogs thereof; steroid and analogs thereof; ligands for LDLR, SRBI or LRP1/2; FK-506, and cyclosporine; lipids disclosed in PCT/JP2019/12077, PCT/JP2019/10392, and PCT/JP2020/035117.

An "analog" herein refers to a compound having an similar structure having the same or a similar basic backbone and a similar nature. An analog includes, for example, a biosynthesis intermediate, a metabolite, and a compound having a substituent. A skilled in the art can determine whether a compound is an analog of another compound.

A tocopherol can be selected from the group consisting of α-tocopherol, β-tocopherol, γ-tocopherol, and δ-tocopherol. Examples of an analog of a tocopherol include various unsaturated analogs of tocopherols, such as α-tocotrienol, β-tocotrienol, γ-tocotrienol, and δ-tocotrienol. Tocopherol is preferably α-tocopherol.

A cholesterol analog refers to various cholesterol metabolites, which are alcohols having a sterol backbone, and analogs thereof. Examples thereof include, but not limited to, cholestanol, lanosterol, cerebrosterol, dehydrocholesterol, and coprostanol.

A lipid may be linked to the 5' end or the 3' end, or to both the ends of the first nucleic acid strand and/or the second nucleic acid strand (preferably, the second nucleic acid strand). Alternatively, a lipid may be linked to an interior nucleotide in the first nucleic acid strand and/or the second nucleic acid strand (preferably, the second nucleic acid strand). The first nucleic acid strand and/or the second nucleic acid strand (preferably, the second nucleic acid strand) may comprise two or more lipids, which may be linked to a plurality of positions in the second nucleic acid strand and/or may be linked as a group to one position in the first nucleic acid strand and/or the second nucleic acid strand (preferably, the second nucleic acid strand). Lipids may be linked one each to the 5' end and the 3' end (preferably, the 5' end) of the first nucleic acid strand and/or the second nucleic acid strand (preferably, the second nucleic acid strand).

The binding between the first nucleic acid strand and/or the second nucleic acid strand (preferably, the second nucleic acid strand) and a lipid may be a direct binding or an indirect binding mediated by another substance. However, in a specific embodiment, it is preferable that the lipid is directly conjugated to the first nucleic acid strand and/or the second nucleic acid strand (preferably, the second nucleic acid strand) via a covalent bond, an ionic bond, a hydrogen bond, or the like, and considering that a more stable binding can be obtained, a covalent bond is more preferable.

The lipid may also be bound to the first nucleic acid strand and/or the second nucleic acid strand (preferably, the second nucleic acid strand) via a cleavable linking group (linker). A "cleavable linking group (linker)" means a linking group that is cleaved under physiological conditions, for example, inside a cell, or inside an animal body (e.g., inside a human body). In a certain embodiment, a cleavable linker is selectively cleaved by an endogenous enzyme, such as a nuclease. Examples of a cleavable linker include an amide, an ester, one or both esters of a phosphodiester, a phosphate ester, a carbamate, and a disulfide bond, as well as a natural DNA linker.

The lipid may also be bound to the first nucleic acid strand and/or the second nucleic acid strand (preferably, the second nucleic acid strand) via an uncleavable linker. An "uncleavable linker" means a linking group that is not cleaved under physiological conditions, for example, inside a cell, or inside an animal body (e.g., inside a human body). Examples of an uncleavable linker include a linker consisting of a phosphorothioate bond, and modified or unmodified deoxyribonucleosides, or modified or unmodified ribonucleosides linked by a phosphorothioate bond. When the linker is a nucleic acid or an oligonucleotide, such as DNA, its chain length is not particularly limited, and may be from 2 to 20 base in length, from 3 to 10 base in length, or from 4 to 6 base in length.

Specific examples of the linker include a linker represented by the following Formula (I). 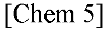 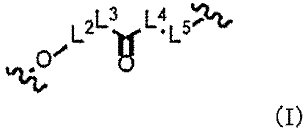 (wherein: L² represents a substituted or unsubstituted C₁ to C₁₂ alkylene group *(e.g.,* propylene, hexylene, or dodecylene), a substituted or unsubstituted C₃ to C₈ cycloalkylene group (*e.g.*, cyclohexylene), -(CH₂)₂-O-(CH₂)₂-O-(CH₂)₂-O-(CH₂)₃-, -(CH₂)₂-O-(CH₂)₂-O-(CH₂)₂-O-(CH₂)₂-O-(CH₂)₃-, or CH(CH₂-OH)-CH₂-O-(CH₂)₂-O-(CH₂)₂-O-(CH₂)₂-O-(CH₂)₃-, L³ represents -NH- or a bond, L⁴ represents a substituted or unsubstituted C₁ to C₁₂ alkylene group (e.g., ethylene, pentylene, heptylene, or undecylene), a substituted or unsubstituted C₃ to C₈ cycloalkylene group (e.g., cyclohexylene), -(CH₂)₂-[O-(CH₂)₂₁.-, or a bond where m represents an integer of 1 to 25, L⁵ represents -NH-(C=O)-, -(C=O)-, or a bond (where the substitution is preferably made by a halogen atom).

In an embodiment, for a linker represented by Formula (I), L² is an unsubstituted C₃ to C₆ alkylene group (e.g., propylene, hexylene), -(CH₂)₂-O-(CH₂)₂-O-(CH₂)₂-O-(CH₂)₃-, or -(CH₂)₂-O-(CH₂)₂-O-(CH₂)₂-O-(CH₂)₂-O-(CH₂)₃-, and L³ is -NH-, and L⁴ and L⁵ are each a bond.

The binding position and the type of binding of the functional moiety in the first nucleic acid strand and/or the second nucleic acid strand (preferably, the second nucleic acid strand) are as described above in connection with the binding between a lipid and the first nucleic acid strand and/or the second nucleic acid strand (preferably, the second nucleic acid strand).

One skilled in the art can produce the first nucleic acid strand and the second nucleic acid strand constituting a nucleic acid complex by appropriately selecting known methods. For example, a nucleic acid can be produced by designing the base sequence of each nucleic acid based on the information about the base sequence of a target transcriptional product (or, in some examples, the base sequence of a target gene), synthesizing the nucleic acid using a commercial automatic nucleic acid synthesizer (such as product of Applied Biosystems, Inc., or product of Beckman Coulter, Inc.), and then purifying the obtained oligonucleotides using a reversed phase column or the like. The nucleic acid produced by this method is mixed in an appropriate buffer solution and denatured at about 90°C to 98°C for several minutes (e.g., 5 min), and then the nucleic acid is annealed at about 30°C to 70°C for about 1 to 8 hours, so that a nucleic acid complex can be produced. Production of an annealed nucleic acid complex is not limited to such time and temperature protocols. Conditions suitable for promoting annealing of strands are well known in the art. A nucleic acid complex to which a functional moiety is conjugated can be produced by using a nucleic acid species to which a functional moiety has been conjugated in advance, and performing the synthesis, purification, and annealing as described above, or by linking a functional moiety to a nucleic acid afterwards. A large number of methods for linking a functional moiety to a nucleic acid are well known in the art. Alternatively, a nucleic acid strand is available on demand from a manufacturer (e.g., GeneDesign Inc.) by specifying a base sequence and a modification site or type.

### (Composition)

In an aspect, the present invention relates to a composition for inhibiting the expression of a target gene or a target transcriptional product by an antisense effect, which comprises the aforedescribed nucleic acid complex. This composition may be a pharmaceutical composition. This composition may be for treating or preventing a disease associated with increased expression of a target gene, such as a neurological disease, a central nervous system disease, a metabolic disease, a tumor, or an infectious disease, in a subject.

A subject may be any animal, including human. However, there is no specific limitation on animal other than human, and various livestock, poultry, pets, and laboratory animals can be a subject.

The present composition may be formulated by a known pharmaceutical production method. For example, the present composition can be used perorally or parenterally in a form of capsule, tablet, pill, liquid formulation, dispersant, granule, microgranule, film coating agent, pellet, troche, sublingual formulation, peptizer, buccal tablet, paste, syrup, suspending agent, elixir, emulsion formulation, coating agent, ointment, plaster, cataplasm, transdermal patch, lotion, inhalant, aerosol, eye drop, injection, or suppository.

Regarding the formulation of these formulations, a pharmaceutically acceptable carrier or solvent or a carrier or solvent acceptable for foods and beverages can be appropriately incorporated. Specific examples of such a carrier or solvent include sterile water, saline, a vegetable oil, a base, an emulsifier, a suspending agent, a surfactant, a pH regulator, a stabilizer, a flavoring agent, a flavor, an excipient, a vehicle, a preservative, a binder, a diluent, an isotonizing agent, a sedative, a bulking agent, a disintegrant, a buffer, a coating agent, a lubricant, a colorant, a sweetener, a thickener, a corrigent, a solubilizer, and other additives.

The dose of the present composition may be selected appropriately according to the age, body weight, symptoms, and health status of a subject, the dosage form, and the like. The dose of the present composition may be, for example, in terms of nucleic acid complex, from 0.0000001 mg/kg/day to 1,000,000 mg/kg/day, from 0.00001 mg/kg/day to 10,000 mg/kg/day, or from 0.001 mg/kg/day to 500 mg/kg/day. The composition may be of a single dose or multiple doses. In the case of multiple doses, it can be administered daily or at appropriate time intervals (e.g., at intervals of 1 day, 2 days, 3 days, 1 week, 2 weeks, or 1 month), for example, 2 to 20 times. A single dose of the aforedescribed nucleic acid complex can be, for example, 0.001 mg/kg or more, 0.005 mg/kg or more, 0.01 mg/kg or more, 0.025 mg/kg or more, 0.1 mg/kg or more, 0.5 mg/kg or more, 1 mg/kg or more, 2.5 mg/kg or more, 0.5 mg/kg or more, 1.0 mg/kg or more, 2.0 mg/kg or more, 3.0 mg/kg or more, 4.0 mg/kg or more, 5 mg/kg or more, 10 mg/kg or more, 20 mg/kg or more, 30 mg/kg or more, 40 mg/kg or more, 50 mg/kg or more, 75 mg/kg or more, 100 mg/kg or more, 150 mg/kg or more, 200 mg/kg or more, 300 mg/kg or more, 400 mg/kg or more, or 500 mg/kg or more. For example, any dose included in a range of from 0.001mg/kg to 500 mg/kg *(e.g.,* 0.001 mg/kg, 0.01 mg/kg, 0.1 mg/kg, 1 mg/kg, 5 mg/kg, 10 mg/kg, 50 mg/kg, 100 mg/kg, or 200 mg/kg) can be appropriately selected.

The nucleic acid complex of the present invention may be administered at a dose of from 0.01 to 10 mg/kg (e.g., about 6.25 mg/kg) four times at a frequency of twice a week. In addition, the nucleic acid complex may be administered at a dose of from 0.05 to 30 mg/kg *(e.g.,* about 25 mg/kg) two to four times at a frequency of once or twice a week, for example, two times at a frequency of twice a week. By adopting such an administration regimen (divided administration), toxicity can be lowered and the load on a subject can be reduced as compared to a single administration of a higher dose. In an embodiment, subcutaneous administration of the nucleic acid complex of the present invention can lower toxicity and reduce the load on a subject as compared to intravenous administration thereof.

In a specific embodiment, the nucleic acid complex of the present invention has excellent solubility in water, Japanese Pharmacopoeia 2nd dissolution test fluid, or Japanese Pharmacopoeia 2nd disintegration test fluid, excellent pharmacokinetics (e.g., serum drug half-life, intracerebral transfer, metabolic stability, CYP inhibition), low toxicity *(e.g.,* excellent as a drug in terms of acute toxicity, chronic toxicity, genetic toxicity, reproductive toxicity, cardiotoxicity, drug interaction, carcinogenicity, phototoxicity), and excellent properties as a pharmaceutical product such as having fewer side effects (e.g., inhibition of sedation, avoiding layered necrosis).

There is no specific limitation on the preferable administration mode of the composition herein. The administration mode may be peroral administration or parenteral administration. Specific examples of parenteral administration include intravenous administration, intra-arterial administration, intraperitoneal administration, subcutaneous administration, intradermal administration, tracheal/bronchial administration, rectal administration, intrathecal administration, intraventricular administration, nasal administration, and intramuscular administration, as well as administration by transfusion. Administration may be performed by intramuscular injection, intravenous instillation, or implantable continuous subcutaneous administration. Since a subcutaneous administration can be carried out by self-injection by a patient on its own, it is suitable. In the case of intravenous administration, the amount of nucleic acid complex contained in a single dose of the composition, i.e., a single dose of nucleic acid complex can be, for example, 0.001 mg/kg or more, 0.005 mg/kg or more, 0.01 mg/kg or more, 0.025 mg/kg or more, 0.1 mg/kg or more, 0.5 mg/kg or more, 1 mg/kg or more, 2.5 mg/kg or more, 5 mg/kg or more, 10 mg/kg or more, 20 mg/kg or more, 30 mg/kg or more, 40mg/kg or more, 50 mg/kg or more, 75 mg/kg or more, 100 mg/kg or more, 150 mg/kg or more, 200 mg/kg or more, 300 mg/kg or more, 400mg/kg or more, or 500 mg/kg or more. For example, any amount in a range of from 0.001 mg/kg to 500 mg/kg *(e.g.,* 0.001 mg/kg, 0.01 mg/kg, 0.1 mg/kg, 1 mg/kg, 5 mg/kg, 10 mg/kg, 50 mg/kg, 100 mg/kg, or 200 mg/kg) can be selected as appropriate.

In one aspect, provided is a method of treating and/or preventing a disease comprising administering the above nucleic acid complex or composition to a subject.

### Examples

Hereinafter, the present invention is described more specifically with reference to Examples. However, the scope of the present invention is not limited to these Examples.

### [Example 1: RNase H/A cleavage activity in the double-stranded nucleic acid complex comprising second nucleic acid strand comprising three contiguous sugar-unmodified ribonucleosides]

A double-stranded nucleic acid complex comprising a first nucleic acid strand comprising an antisense oligonucleotide and a cholesterol-conjugated complementary strand (second nucleic acid strand) comprising three contiguous sugar-unmodified ribonucleosides was treated with RNase A or RNase H, and its cleavage efficiency was examined. The second nucleic acid strand and cholesterol are conjugated by the same 5' end structure as chol#1-cRNA(DNA)(mMalat1) shown in International Publication No. WO 2018/056442.

### (Preparation of nucleic acid agents)

A 16-mer single-stranded LNA/DNA gapmer (ASO(mMalat1)1) (first nucleic acid strand) targeting malat1 non-coding RNA was prepared. This LNA/DNA gapmer is an oligonucleotide of 16 base in length comprising three LNA nucleosides at the 5' end and three LNA nucleosides at the 3' end and 10 DNA nucleosides therebetween. This LNA/DNA gapmer has a base sequence complementary to positions 1317 to 1332 of murine malat1 non-coding RNA (SEQ ID NO: 1).

A complementary RNA strand having a base sequence complementary to this ASO and having a cholesterol covalently linked at the 5' end (Chol-cRNA(mMalatl)) (second nucleic acid strand) was prepared. The second strand is an oligonucleotide of 16 base in length comprising three 2'-O-methyl-modified ribonucleosides at both ends, seven 2'-O-methyl-modified ribonucleosides therebetween, and three contiguous sugar-unmodified ribonucleosides at a variety of positions. A complementary strand comprising three 2'-O-methyl-modified ribonucleosides at both ends and 10 sugar-unmodified ribonucleosides therebetween (Chol-cRNA) and a complementary strand in which all 16 nucleic acid moieties are 2'-O-methyl-modified ribonucleosides (Chol-cRNA full OMe) were prepared as controls.

A double-stranded nucleic acid agent was prepared by annealing the aforedescribed ASO with the second nucleic acid strand. Specifically, these were dissolved in PBS, mixed in equal molar volumes, the solution was heated at 98°C for 5 minutes, then cooled to 37°C, and held for 1 hour, whereby the nucleic acid strand was annealed. Thus, the double-stranded nucleic acid agents were prepared.

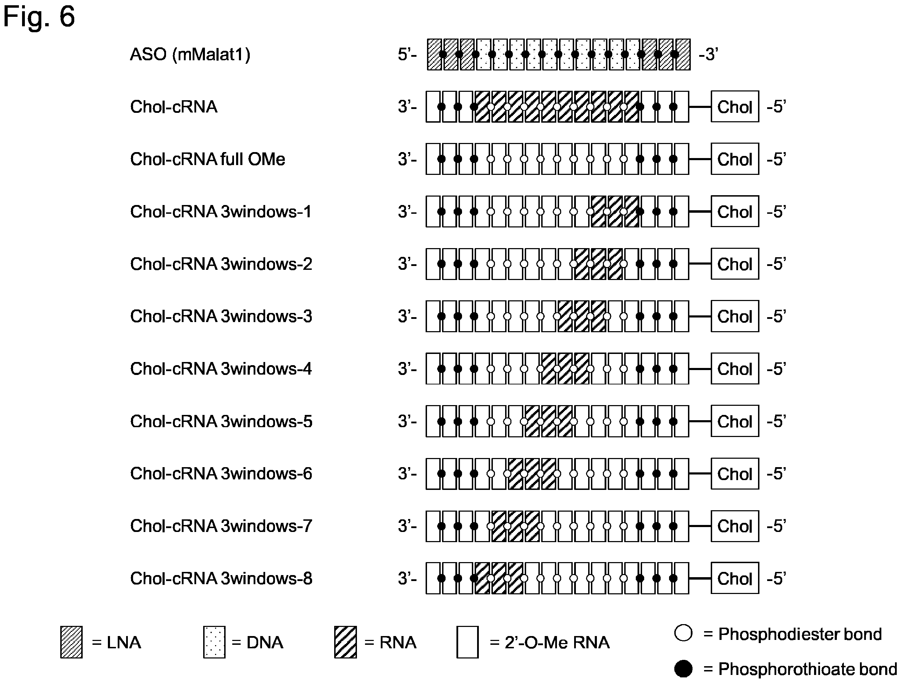

The sequences, chemical modifications, and structures of the oligonucleotides used in Example 1 are shown in Table 1 and Figure 6. All oligonucleotides were produced by Gene Design, Inc. (Osaka, Japan).

**[Table 1]**

| Oligonucleotide name | Sequence (5'-3') | SEQ ID NO: |
|---|---|---|
| ASO (mMalat1) | 5(L)*T(L)*A(L)*g*t*t*c*a*c*t*g*a*a*T(L)*G(L)*5(L) | 3 |
| Chol-cRNA full OMe | Chol-G*C*A*UUCAGUGAAC*U*A*G | 4 |
| Chol-cRNA | Chol-G*C*A*UUCAGUGAAC*U*A*G | 5 |
| Chol-cRNA 3windows-1 | Chol-G*C*A*UUCAGUGAAC*U*A*G | 6 |
| Chol-cRNA 3windows-2 | Chol-G*C*A*UUCAGUGAAC*U*A*G | 7 |
| Chol-cRNA 3windows-3 | Chol-G*C*A*UUCAGUGAAC*U*A*G | 8 |
| Chol-cRNA 3windows-4 | Chol-G'C'A'UUCAGUGAAC'U'A'G | 9 |
| Chol-cRNA 3windows-5 | Chol-G*C*A*UUCAGUGAAC*U*A*G | 10 |
| Chol-cRNA 3windows-6 | Chol-G*C*A*UUCAGUGAAC*U*A*G | 11 |
| Chol-cRNA 3windows-7 | Chol-G*C*A*UUCAGUGAAC*U*A*G | 12 |
| Chol-cRNA 3windows-8 | Chol-G*C*A*UUCAGUGAAC*U*A*G | 13 |

| | | |
|---|---|---|
| Upper case (L): LNA (5(L) represents 5-methylcytosine LNA) Lower case: DNA Upper case: RNA Upper case (underlined): 2'-O-Me RNA *: Phosphorothioate bond Chol: Cholesterol | | |

### (Cleavage experiment)

Five microliters (5 µL) of 10 µM double-stranded nucleic acid annealed as described above was prepared.

The following enzymes were prepared.
Ribonuclease H (RNase H) (60 U/µL: TAKARA BIO INC)
RNase A (7000 U/ml (100 mg/ml): Qiagen)

The following buffers were prepared for RNase H (each at the final concentration).
60 mM Tris-HCl (pH 7.8)
60 mM KCl
2.5 mMMgCl₂
2 mM TCEP

After buffer preparation, 5 U or 10 U of Ribonuclease H was reacted with 5 µL of 10 µM nucleic acid.

For RNase A, Dulbecco's phosphate buffered saline (free of Ca and Mg) (NACALAI TESQUE, INC.) was used. In the buffer, 0.035 U of RNase A was reacted with 5 µL of 10 µM nucleic acid.

Under the aforedescribed reaction conditions, the enzyme was reacted by PCR at 37°C for 20 minutes using a PCR machine (LifeECO Thermal Cycler). Thereafter, the enzymatic reaction was stopped in liquid nitrogen.

### (Electrophoresis/Analysis)

20% acrylamide gel (1 × TBE) was prepared. The enzyme reaction product was mixed with 6 × Gel Loading Dye, Purple (New England Biolab) and electrophoresed on the gel at 150 V for 2 hours. ASO alone was electrophoresed simultaneously as a control. Then, a solution was prepared by diluting a GelRed (× 10000) aqueous solution (Biotium) to a concentration of 1/10000 with 1 × TBE. The gel was impregnated with the solution for 10 minutes. The gel was then photographed with the ChemiDo Touch Imaging System (BioRad). The band density was analyzed with Image Lab Version 5.2 build 14 (BioRad).

### (Results)

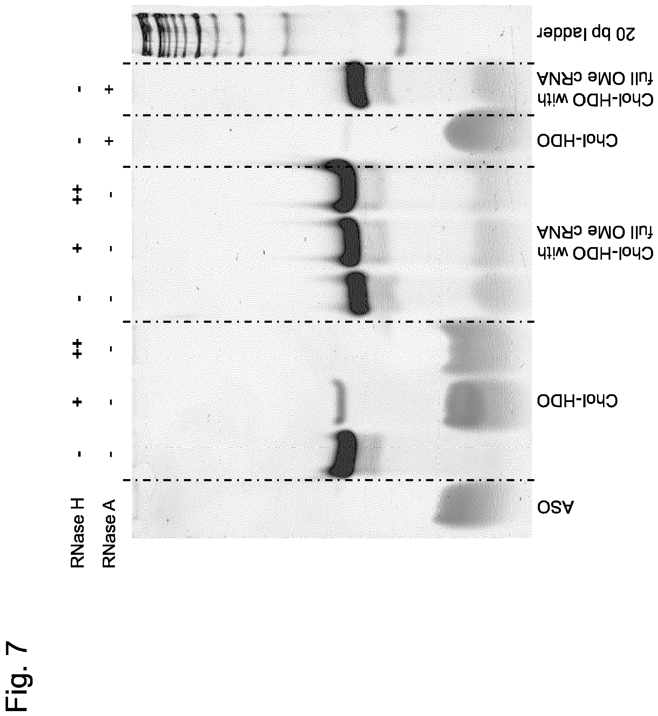

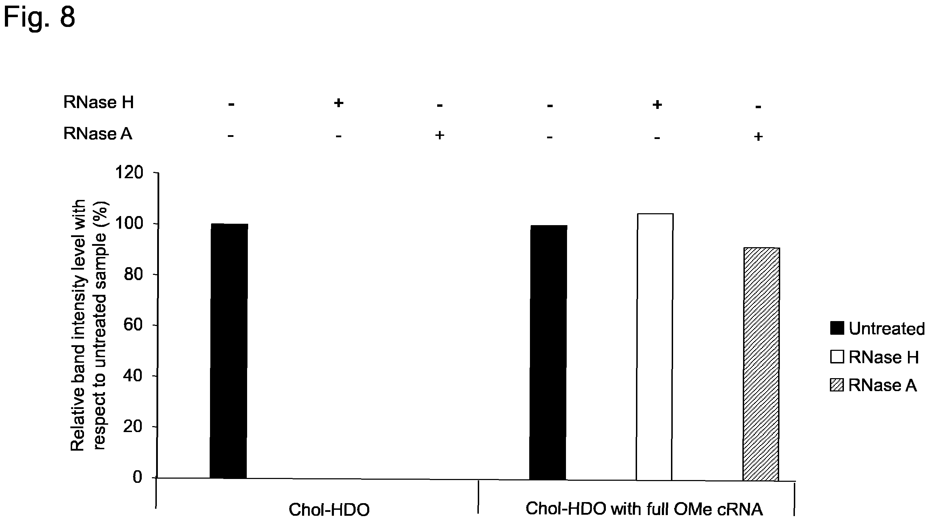

Figure 7 shows the results of electrophoresis of ASO alone, a double-stranded complex of ASO and Chol-cRNA (mMalat1), and a double-stranded complex of ASO and Chol-cRNA (mMalat1) full OMe (Chol-HDO with full OMe cRNA) after cleavage by RNase H and/or RNase A. In addition, Figure 8 is a graph showing the densities of the uncleaved bands based on the electrophoresis results in terms of the relative intensity level (%) with respect to the untreated band. As shown in Figures 7 and 8, in the double-stranded complex in which all 10 nucleic acids in the second nucleic acid strand that were complementary to the gap region of the first nucleic acid strand were sugar-unmodified ribonucleosides (Chol-HDO), the complementary strand was cleaved when either RNase H or RNase A was used. In contrast, in the double-stranded complex in which all 10 nucleic acids in the second nucleic acid strand that were complementary to the gap region of the first nucleic acid strand were 2'-O-methyl-modified ribonucleosides (Chol-HDO with full OMe cRNA), the complementary strand was not cleaved when either RNase H or RNase A was used. These results suggest that Chol-HDO can be reduced in activity due to *in vivo* degradation, whereas Chol-HDO with full OMe cRNA has excessively high resistance to degradation and therefore RNase H-dependent pathway-based activity may be reduced.

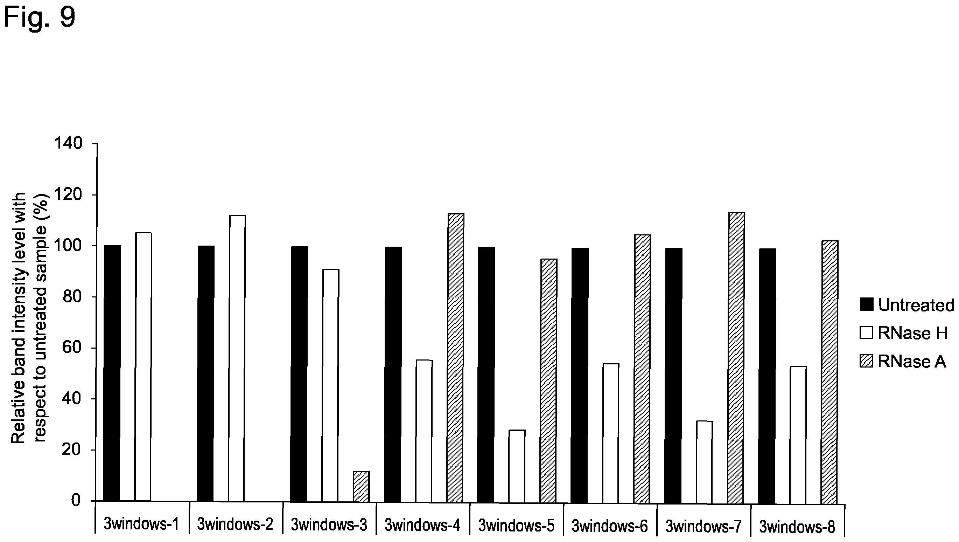

Figure 9 is a graph showing the densities of the uncleaved bands in terms of the relative intensity level (%) with respect to the untreated band in a similar manner when a similar test was performed on double-stranded nucleic acid agents each comprising three contiguous sugar-unmodified ribonucleosides that are complementary to a part of the gap region of the first nucleic acid strand in which the positions of the three contiguous sugar-unmodified ribonucleosides were changed. Note that 3windows-1 to -8 indicate the results for the complexes of each of Chol-cRNA 3windows-1 to -8 shown in Table 1 and ASO. In the complex comprising a second nucleic acid strand comprising three contiguous sugar-unmodified ribonucleosides, when there were three contiguous sugar-unmodified ribonucleosides on the 3' side of the second nucleic acid strand having a low C/U content (3windows-4 to -8), RNase A resistance was high, and when there were three contiguous sugar-unmodified ribonucleosides on the 5' side thereof having a high C/U content (3windows-1 to -3), RNase A resistance was low. From these results, it is considered that RNase A resistance correlates with whether or not the three contiguous sugar-unmodified ribonucleosides comprise C/U, and this result is also consistent with the fact that RNase A cleaves the PO bond on the 3' side of C/U, which is a pyrimidine base. On the other hand, in the case of RNase H, cleavage was likely to occur when there were three contiguous sugar-unmodified ribonucleosides on the 3' side of the second nucleic acid strand (3windows-4 to -8). These results suggest that it is possible to design nucleic acids that maintain activity by the RNase H-dependent pathway while reducing degradation by RNase A.

### [Example 2: Gene inhibition effect in cells]

A double-stranded nucleic acid complex comprising a first nucleic acid strand comprising an antisense oligonucleotide and a cholesterol-conjugated complementary strand (second nucleic acid strand) was introduced into cells to examine its gene inhibition effect.

### (Preparation of nucleic acid agents)

Nucleic acids corresponding to those of Example 1 were prepared.

### (Cell experiment)

Neuro2a cells used herein were purchased from the American Type Culture Collection (ATC). Neuro2a was prepared in a 24-well plate and grown to 60% to 80% confluency. After the growth, the aforedescribed nucleic acids were each introduced at 5/10/20 nM with 1.5 µL of RNAimax (Thermo Fisher Scientific) per well according to the manufacturer's protocol and cells were collected after 24 hours. Then, RNA was extracted with ISOGEN (NIPPON GENE CO., LTD.) according to the manufacturer's protocol. cDNA was synthesized using the RNA by using PrimeScript RT Master Mix (TAKARA-Bio) according to the manufacturer's protocol. Quantitative RT-PCR was performed according to a conventional method using LightCycler 480 Probes Master (Roche Life Science). The primers and probes used in quantitative RT-PCR are as follows.

**[Table 2]**

| Probe name or Primer name | Sequence (5'-3') | SEQ ID NO: |
|---|---|---|
| mMalat1-Probe | FAM-TGTTGGCACGACACCTTCAGGGACT-TAMRA | 14 |
| mMalat1-Forward | TGGGTTAGAGAAGGCGTGTACTG | 15 |
| mMalat1-Reverse | TCAGCGGCAACTGGGAAA | 16 |
| mActb-Probe | FAM-CACACCCGCCACCAGTTCGCCATG-TAMRA | 17 |
| mActb-Forward | CGCGAGCACAGCTTCTTTG | 18 |
| mActb-Reverse | ATGCCGGAGCCGTTGTC | 19 |

Probes Master, cDNA, primers, and probes were mixed, and PCR was performed at 95°C for 10 minutes for 1 cycle, 95°C for 10 seconds/60°C for 30 seconds/72°C for 1 second for 45 cycles, and 40°C for 30 seconds for 1 cycle.

### (Results)

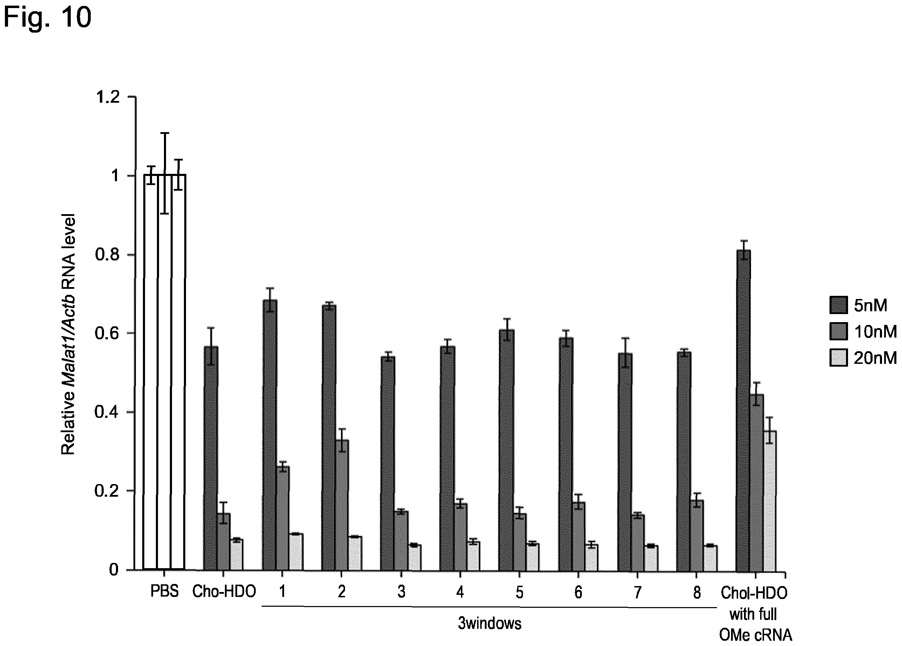

Figure 10 shows relative RNA expression levels when cells are treated with various double-stranded nucleic acid complexes, provided that the expression level of Malat1/Actb (β-actin) in PBS treatment is 1. As described above, Chol-HDO indicates the result for the double-stranded complex of ASO and Chol-cRNA (mMalat1), Chol-HDO with full OMe cRNA indicates the result for the double-stranded complex of ASO and Chol-cRNA (mMalat1) full OMe, 3windows-1 to -8 indicate the results for the complexes of each of Chol-cRNA 3windows-1 to -8 shown in Table 1 and ASO.

In the double-stranded complex in which all 10 nucleic acids in the second nucleic acid strand that were complementary to the gap region of the first nucleic acid strand were sugar-unmodified ribonucleosides (Chol-HDO), the antisense effect was high. In contrast, in the double-stranded complex in which all 10 nucleic acids in the second nucleic acid strand that were complementary to the gap region of the first nucleic acid strand were 2'-O-methyl-modified ribonucleosides (Chol-HDO with full OMe cRNA), the antisense effect was low. In the complex comprising a second nucleic acid strand comprising three contiguous sugar-unmodified ribonucleosides, when there were three contiguous sugar-unmodified ribonucleosides on the 5' side of the second nucleic acid strand (3windows-1 and -2), the gene inhibition effect was lower than that when there were three contiguous sugar-unmodified ribonucleosides on the 3' side thereof (3windows-3 to -8). These results are largely consistent with the results of the activity of cleavage by RNase A/H in Example 1.

It is considered that the antisense effect observed with Chol-HDO with full OMe cRNA is a cleavage-independent effect, and the difference in the antisense effect between 3windows-1 and -2 and 3windows-3 to -8 is derived from the activity dependent on RNase H cleavage.

### [Example 3: RNase H/A cleavage activity in a double-stranded nucleic acid complex comprising second nucleic acid strand comprising one or two contiguous sugar-unmodified ribonucleosides]

A double-stranded nucleic acid complex comprising a first nucleic acid strand comprising an antisense oligonucleotide and a cholesterol-conjugated complementary strand (second nucleic acid strand) comprising one or two contiguous sugar-unmodified ribonucleosides was treated with RNase A or RNase H, and its cleavage efficiency was examined.

### (Preparation of nucleic acid agents)

ASO and complementary strands comprising one or two contiguous sugar-unmodified ribonucleosides were prepared based on Example 1. These were dissolved in PBS, mixed in equal molar volumes, the solution was heated at 98°C for 5 minutes, then cooled to 37°C, and held for 1 hour, whereby the nucleic acid strand was annealed. Thus, the double-stranded nucleic acid agents were prepared. A complementary strand comprising three 2'-O-methyl-modified ribonucleosides at both ends and 10 sugar-unmodified ribonucleosides therebetween (Chol-cRNA) and a complementary strand in which all 16 nucleic acid moieties are 2'-O-methyl-modified ribonucleosides (Chol-cRNA full OMe) were prepared as controls.

The sequences of ASO, Chol-cRNA, and Chol-cRNA full OMe are as shown in Table 1, and the names and sequences of the other second strands used in this Example are shown below.

**[Table 3]**

| Oligonucleotide name | Sequence (5'-3') | SEQ ID NO: |
|---|---|---|
| Chol-cRNA 2windows-1 | Chol-G*C*A*UUCAGUGAAC*U*A*G | 20 |
| Chol-cRNA 2windows-2 | Chol-G*C*A*UUCAGUGAAC*U*A*G | 21 |
| Chol-cRNA 2windows-3 | Chol-G*C*A*UUCAGUGAAC*U*A*G | 22 |
| Chol-cRNA 2windows-4 | Chol-G*C*A*UUCAGUGAAC*U*A*G | 23 |
| Chol-cRNA 2windows-5 | Chol-G*C*A*UUCAGUGAAC*U*A*G | 24 |
| Chol-cRNA 2windows-6 | Chol-G*C*A*UUCAGUGAAC*U*A*G | 25 |
| Chol-cRNA 2windows-7 | Chol-G*C*A*UUCAGUGAAC*U*A*G | 26 |
| Chol-cRNA 2windows-8 | Chol-G*C*A*UUCAGUGAAC*U*A*G | 27 |
| Chol-cRNA 2windows-9 | Chol-G*C*A*UUCAGUGAAC*U*A*G | 28 |
| Chol-cRNA 1window-1 | Chol-G*C*A*UUCAGUGAAC*U*A*G | 29 |
| Chol-cRNA 1window-2 | Chol-G*C*A*UUCAGUGAAC*U*A*G | 30 |
| Chol-cRNA 1window-3 | Chol-G*C*A*UUCAGUGAAC*U*A*G | 31 |
| Chol-cRNA 1window-4 | Chol-G*C*A*UUCAGUGAAC*U*A*G | 32 |
| Chol-cRNA 1window-5 | Chol-G*C*A*UUCAGUGAAC*U*A*G | 33 |
| Chol-cRNA 1window-6 | Chol-G*C*A*UUCAGUGAAC*U*A*G | 34 |
| Chol-cRNA 1window-7 | Chol-G*C*A*UUCAGUGAAC*U*A*G | 35 |
| Chol-cRNA 1window-8 | Chol-G*C*A*UUCAGUGAAC*U*A*G | 36 |
| Chol-cRNA 1window-9 | Chol-G*C*A*UUCAGUGAAC*U*A*G | 37 |
| Chol-cRNA 1window-10 | Chol-G*C*A*UUCAGUGAAC*U*A*G | 38 |

| | | |
|---|---|---|
| Upper case: RNA Upper case (underlined): 2'-O-Me RNA *: Phosphorothioate bond Chol: Cholesterol | | |

Cleavage with RNase A or H, and subsequent electrophoresis and analysis were carried out in accordance with Example 1.

### (Results)

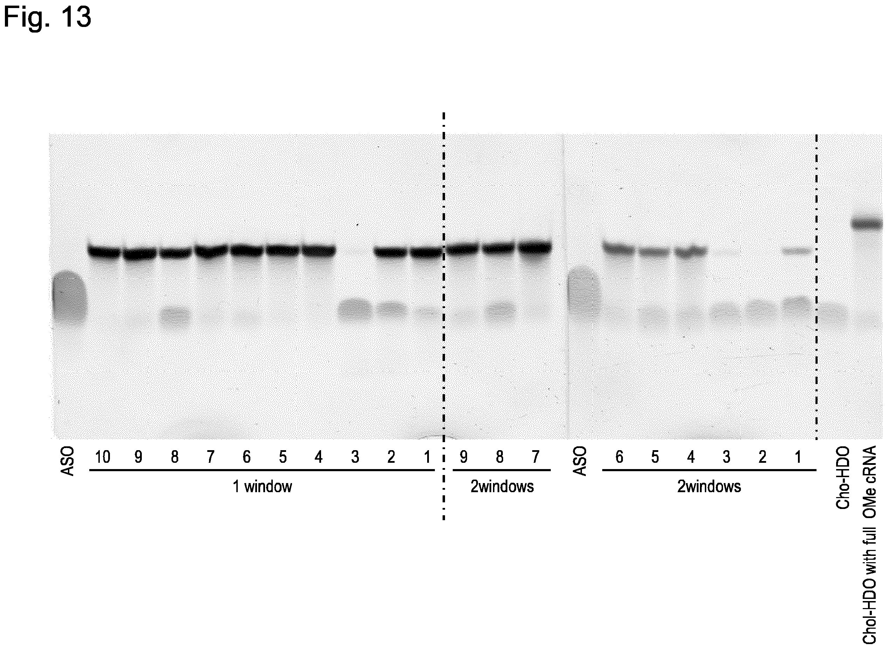

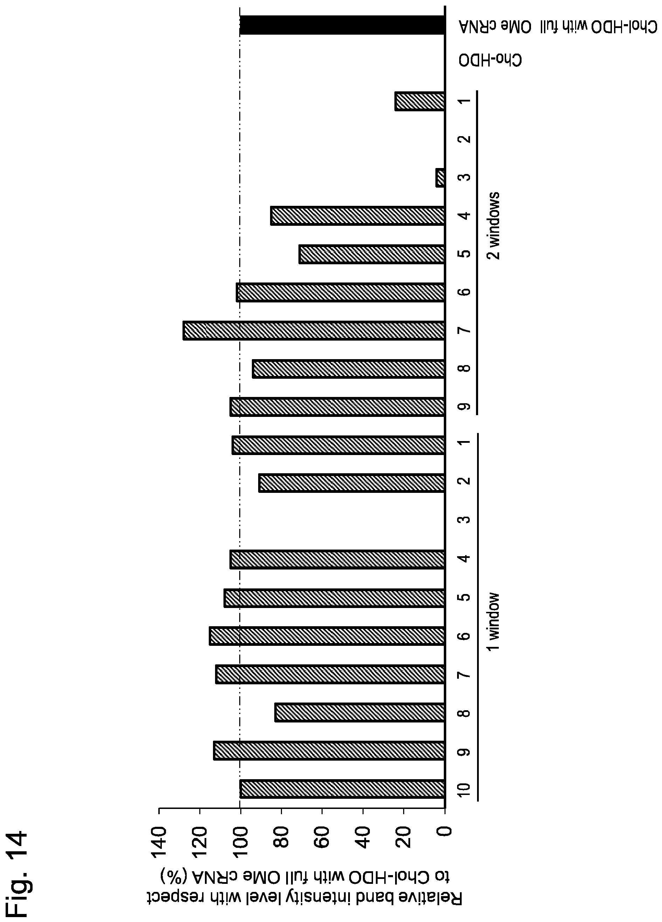

Figure 13 shows the results of electrophoresis after cleavage by RNase A. In addition, Figure 14 is a graph showing the densities of the uncleaved bands based on the electrophoresis results in terms of the relative intensity level (%) with respect to Chol-HDO with full OMe cRNA.

As shown in Figures 13 and 14, in the double-stranded complex in which all 10 nucleic acids in the second nucleic acid strand that were complementary to the gap region of the first nucleic acid strand were sugar-unmodified ribonucleosides (Chol-HDO), the complementary strand was cleaved by RNase A. In contrast, in the double-stranded complex in which all 10 nucleic acids in the second nucleic acid strand that were complementary to the gap region of the first nucleic acid strand were 2'-O-methyl-modified ribonucleosides (Chol-HDO with full OMe cRNA), the complementary strand was not cleaved by RNase A.

As shown in Figures 13 and 14, in a case where one sugar-unmodified ribonucleoside was included, cleavage by RNase A occurred only when the sugar-unmodified ribonucleoside was present at the position of C in the second nucleic acid strand (1 window-3) while when the sugar-unmodified ribonucleoside was present at the position of U (1 window 1, 2, 6), cleavage did not occur. Meanwhile, in a case where two contiguous sugar-unmodified ribonucleosides were included, cleavage with RNase A occurred when the sugar-unmodified ribonucleosides were present at the positions of CA (2 window-3), UC (2 window-2), or UU (2 window-1). These results suggest that RNase A cleaves C or UC/UU contiguous sequence.

### [Example 4: In vivo effect of double-stranded nucleic acid complex comprising second nucleic acid strand having sugar-modified ribonucleoside at position of C/U for inhibiting Dmpk mRNA expression]

An *in vivo* effect of a double-stranded nucleic acid complex comprising a first nucleic acid strand comprising an antisense oligonucleotide targeting the Dmpk gene and a cholesterol-conjugated complementary strand (second nucleic acid strand) having a sugar-modified ribonucleoside at the position of C/U for inhibiting mRNA expression in tissue was examined.

### (Preparation of nucleic acid agents)

An antisense oligonucleotide (ASO (DMPK)) targeting the Dmpk gene, a cholesterol-conjugated complementary strand having a 2'-O-methyl modification at the position of C/U (Chol-cRNA (CU OMe)), and a complementary strand (Chol-cRNA (Default)) as a control were prepared. The sequences of ASO (mDMPK), Chol-cRNA (Default), and Chol-cRNA (CU OMe) are shown below.

**[Table 4]**

| Oligonucleotide name | Sequence (5'-3') | SEQ ID NO: |
|---|---|---|
| ASO (mDMPK) | A(L)*5(L)*A(L)*a*t*a*a*a*t*a*c*c*g*A(L)*G(L)*G(L) | 43 |
| Chol-cRNA (Default) | Chol-C*C*U*CGGUAUUUAU*U*G*U | 44 |
| Chol-cRNA (CU OMe) | Chol-C*C*U*CGGUAUUUAU*U*G*U | 45 |

| | | |
|---|---|---|
| Upper case (L): LNA (5(L) represents 5-methylcytosine LNA) Lower case: DNA Upper case: RNA Upper case (underlined): 2'-O-Me RNA *: Phosphorothioate bond Chol: Cholesterol | | |

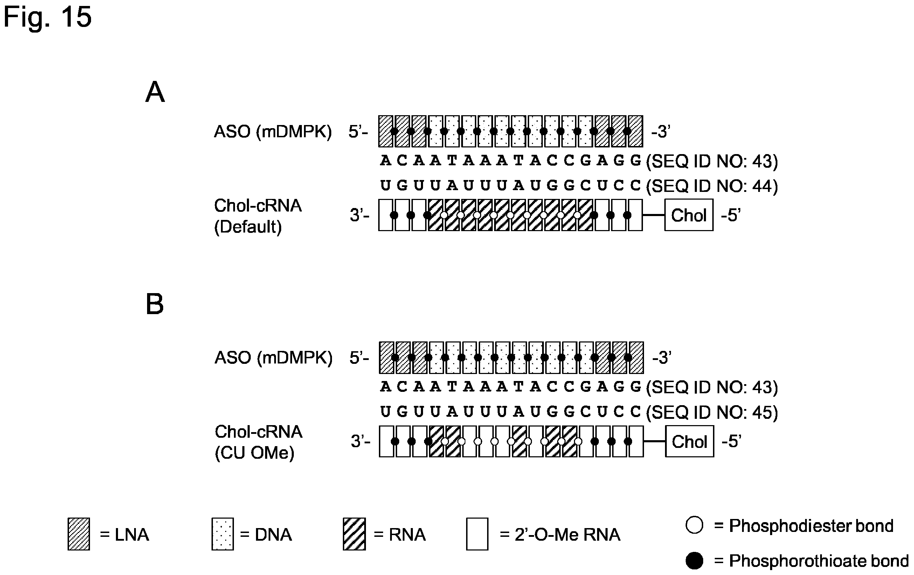

The aforedescribed ASO (mDMPK) is constituted by a 16-mer single-stranded LNA/DNA gapmer that targets the murine DMPK gene and has a base sequence complementary to positions 2682 to 2697 which targets a transcription product DMPK mRNA (GenBank Accession No. NM_032418; SEQ ID NO: 46). More specifically, this LNA/DNA gapmer consists of three LNA nucleosides from the 5' end, three LNA nucleosides from 3' end, respectively, and ten DNA nucleosides therebetween (Figure 15).

The aforedescribed Chol-cRNA (Default) has a sequence complementary to ASO (mDMPK) and comprises three 2'-O-methyl-modified ribonucleosides at the both ends and ten sugar-unmodified ribonucleosides therebetween, and a cholesterol is conjugated to the 5' end (Figure 15A).

The aforedescribed Chol-cRNA (CU OMe) has a 2'-O-methyl modification at all of the positions of C and U, except the position of U located on the 3' side, among 10 sugar-unmodified ribonucleosides located at the center of Chol-cRNA (Default)(Figure 15B).

As in Example 1, ASO (mDMPK) and Chol-cRNA (Default) or ASO (mDMPK) and Chol-cRNA (CU OMe) were dissolved in PBS, mixed in equal molar volumes, the solution was heated at 98°C for 5 minutes, then cooled to 37°C, and held for 1 hour, whereby the nucleic acid strand was annealed. Thus, double-stranded nucleic acid complexes were prepared. The prepared double-stranded nucleic acid complexes are referred to as "Chol-HDO (Default)" or "Chol-HDO(CU OMe)", respectively.

### (In vivo experiment)

As mice to which the double-stranded nucleic acid complexes were administered, male C57BL/6 mice weighing 20 g and aged 4 to 5 weeks were used. Four mice were used for each condition.

Each double-stranded nucleic acid complex was intravenously injected into mice at a dose of 12.5 mg/kg via a tail vein in a single dose. In addition, mice injected with a single dose of PBS alone were also prepared as a negative control group.

### (Expression analysis)

At 72 hours after administration, mice were perfused with PBS and then dissected, whereby heart muscle (Heart), quadriceps (Quadriceps), musculi dorsi proprii (Back), tibialis anterior (TA), gastrocnemius (GC), triceps brachii (TB), kidney, and liver were isolated. Subsequently, mRNA was extracted from each tissue according to the protocol using a high-throughput fully automated nucleic acid extractor MagNA Pure 96 (Roche Life Science). cDNA was synthesized according to the protocol of Transcriptor Universal cDNA Master (Roche Life Science). Quantitative RT-PCR was performed by TaqMan (Roche Life Science). The primers used in quantitative RT-PCR were products designed and manufactured by Thermo Fisher Scientific based on various gene numbers. The PCR conditions (temperature and time) were 95°C for 15 seconds, 60°C for 30 seconds, and 72°C for 1 second per cycle, which were repeated 40 cycles. Each obtained amplification product was quantified by quantitative RT-PCR, and based on the results, the expression level of mRNA (DMPK)/the expression level of mRNA (ACTB; internal standard gene) was calculated, thereby obtaining the relative expression level. Mean values and standard errors of relative expression levels were calculated.

### (Results)

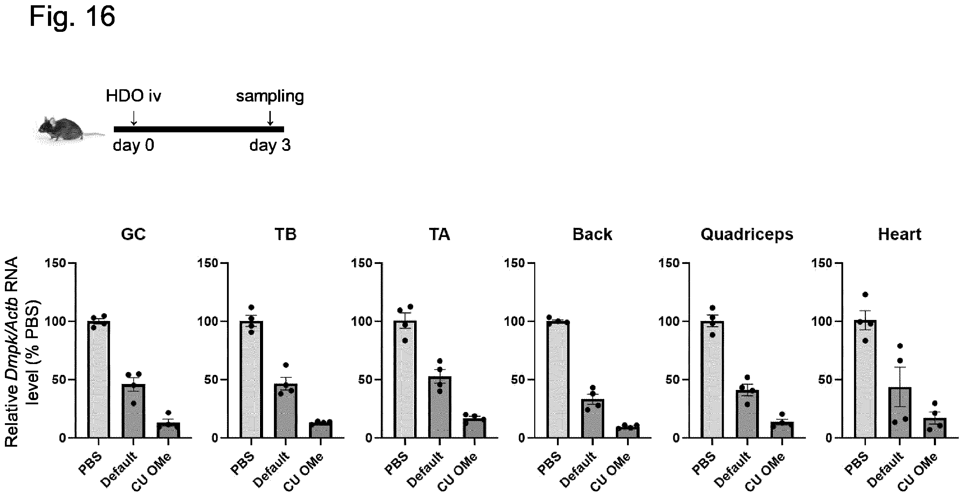

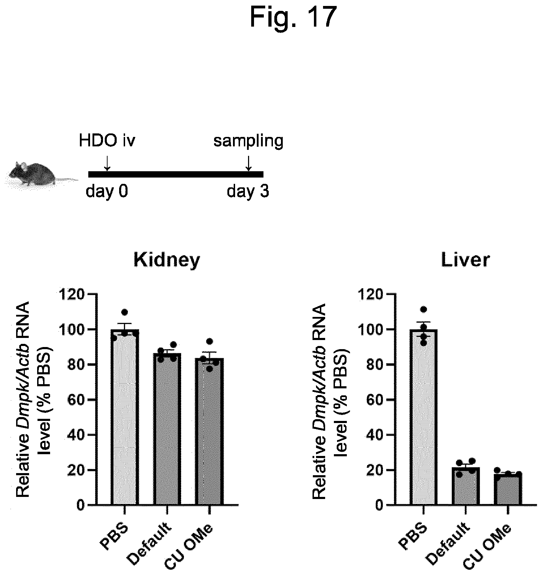

Figures 16 and 17 show the results. Figure 16 shows the effects of double-stranded nucleic acid complexes Chol-HDO (Default) and Chol-HDO (CU OMe) for inhibiting the expression of the target mDMPK gene in gastrocnemius (GC), triceps brachii (TB), tibialis anterior (TA), musculi dorsi proprii (Back), quadriceps (Quadriceps), and heart muscle (Heart). Figure 17 shows the effects of double-stranded nucleic acid complexes Chol-HDO (Default) and Chol-HDO (CU OMe) for inhibiting the expression of the target mDMPK gene in the kidney and liver. Error bars indicate the respective standard errors.

Chol-HDO (CU OMe) showed a stronger expression inhibition effect than Chol-HDO (Default) in any of gastrocnemius, triceps brachii, tibialis anterior, musculi dorsi proprii, quadriceps, and heart muscle. The results suggested that by adding a sugar modification to a ribonucleoside at the position of C/U of the second nucleic acid strand, resistance to cleavage by RNase A is produced, and as a result, more efficient expression inhibition of the target gene becomes possible.

In addition, Chol-HDO (CU OMe) showed a comparable expression inhibition effect as Chol-HDO (Default) in the kidney and liver.

### [Example 5: In vivo effect of double-stranded nucleic acid complex comprising second nucleic acid strand having sugar-modified ribonucleoside at position of C/U for inhibiting human SOD1 mRNA expression]

An *in vivo* effect of a double-stranded nucleic acid complex comprising a first nucleic acid strand comprising an antisense oligonucleotide targeting the human SOD1 gene and a cholesterol-conjugated complementary strand (second nucleic acid strand) having a sugar-modified ribonucleoside at the position of C/U for inhibiting mRNA expression in tissue was examined.

### (Preparation of nucleic acid agents)

An antisense oligonucleotide (ASO (SOD1)) targeting the SOD1 gene, a cholesterol-conjugated complementary strand having a 2'-O-methyl modification at the position of C/U (Chol-cRNA (CU OMe)), and a complementary strand (Chol-cRNA (Default)) as a control were prepared. The sequences of ASO (mDMPK), Chol-cRNA (Default), and Chol-cRNA (CU OMe) are shown below.

**[Table 5]**

| Oligonucleotide name | Sequence (5'-3') | SEQ ID NO: |
|---|---|---|
| ASO (hSOD1) | T(L)*T(L)*A(L)*a*t*g*t*t*t*a*t*c*a*g*G(L)*A(L)*T(L) | 67 |
| Chol-cRNA (Default) | Chol-A*U*C*CUGAUAAACAU*U*A*A | 47 |
| Chol-cRNA (CU OMe) | Chol-A*U*C*CUGAUAAACAU*U*A*A | 48 |

| | | |
|---|---|---|
| Upper case(L):LNA (5(L) represents 5-methylcytosine LNA) Lower case: DNA Upper case: RNA Upper case (underlined): 2'-O-Me RNA *: Phosphorothioate bond Chol: Cholesterol | | |

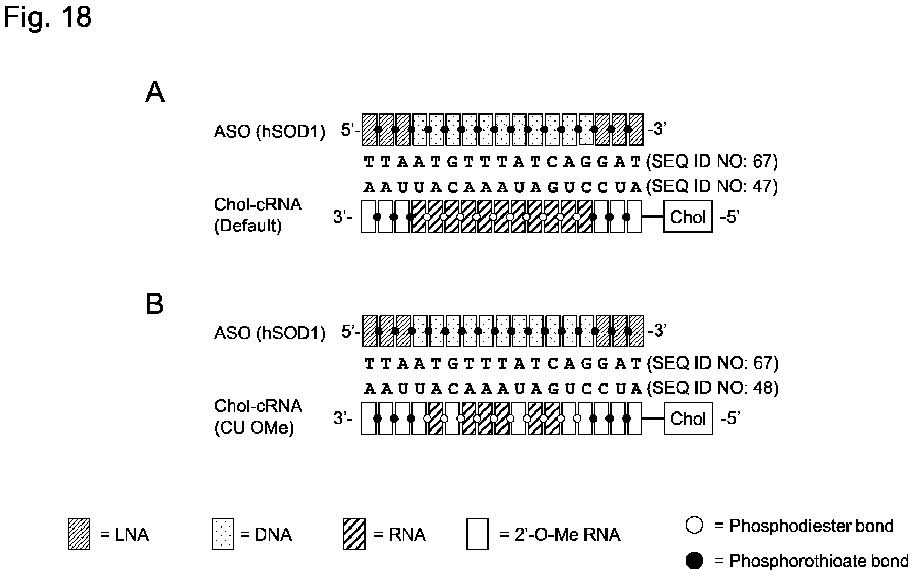

The aforedescribed ASO (hSOD1) is constituted by a 17-mer single-stranded LNA/DNA gapmer that targets the human SOD1 gene and has a base sequence complementary to positions 679 to 695 which targets its transcription product hSOD1 mRNA (GenBank Accession No. NM_000454.4) of the gene (SEQ ID NO: 67). More specifically, this LNA/DNA gapmer consists of three LNA nucleosides from the 5' end, three LNA nucleosides from 3' end, respectively, and ten DNA nucleosides therebetween (Figure 18).

The aforedescribed Chol-cRNA (Default) has a sequence complementary to ASO (hSOD1) and comprises three 2'-O-methyl-modified ribonucleosides at the both ends and ten sugar-unmodified ribonucleosides therebetween, and a cholesterol is conjugated to the 5' end (Figure 18A).

The aforedescribed Chol-cRNA (CU OMe) has a 2'-O-methyl modification at all of the positions of C and U among the ten sugar-unmodified ribonucleosides located at the center of Chol-cRNA (Default) (Figure 18B).

As in Example 1, ASO (hSOD1) and Chol-cRNA (Default) or ASO (hSOD1) and Chol-cRNA (CU OMe) were dissolved in PBS, mixed in equal molar volumes, the solution was heated at 98°C for 5 minutes, then cooled to 37°C, and held for 1 hour, whereby the nucleic acid strand was annealed. Thus, double-stranded nucleic acid complexes were prepared. The prepared double-stranded nucleic acid complexes are referred to as "Chol-HDO (Default)" or "Chol-HDO(CU OMe)", respectively.

### (In vivo experiment)

As mice to which the double-stranded nucleic acid complexes were administered, male human SOD1G93A transgenic mice (G93A Tg mice) weighing 20 g and aged 4 to 5 weeks were used. Two to four mice were used for each condition.

Each double-stranded nucleic acid complex was subcutaneously injected into mice at a dose of 50 mg/kg in a single dose. In addition, mice injected with a single dose of PBS alone were also prepared as a negative control group.

### (Expression analysis)

At 72 hours after administration, mice were perfused with PBS and then dissected, whereby heart muscle (Heart), quadriceps (Quadriceps), diaphragm (Diaphragm), and musculi dorsi proprii (Back) were isolated. Subsequently, mRNA was extracted from each tissue according to the protocol using a high-throughput fully automated nucleic acid extractor MagNA Pure 96 (Roche Life Science). cDNA was synthesized according to the protocol of Transcriptor Universal cDNA Master (Roche Life Science). Quantitative RT-PCR was performed by TaqMan (Roche Life Science). The primers used in quantitative RT-PCR were products designed and manufactured by Thermo Fisher Scientific based on various gene numbers. The PCR conditions (temperature and time) were 95°C for 15 seconds, 60°C for 30 seconds, and 72°C for 1 second per cycle, which were repeated 40 cycles. Each obtained amplification product was quantified by quantitative RT-PCR, and based on the results, the expression level of mRNA (SOD1)/the expression level of mRNA (ACTB; internal standard gene) was calculated, thereby obtaining the relative expression level. Mean values and standard errors of relative expression levels were calculated.

### (Results)

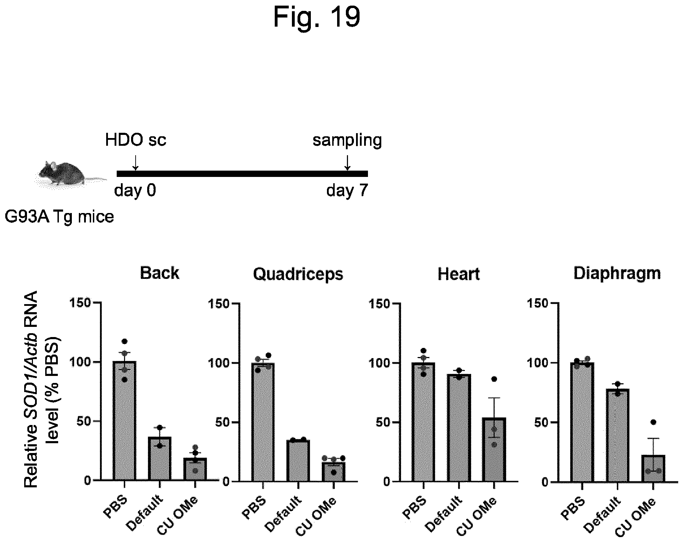

Figure 19 shows the results. Figure 19 shows the effects of double-stranded nucleic acid complexes Chol-HDO (Default) and Chol-HDO (CU OMe) for inhibiting the expression of the target hSOD1 gene in musculi dorsi proprii (Back), quadriceps (Quadriceps), diaphragm (Diaphragm), and heart muscle (Heart). Error bars indicate the respective standard errors.

Chol-HDO (CU OMe) showed a stronger expression inhibition effect than Chol-HDO (Default) in all of musculi dorsi proprii, quadriceps, diaphragm, and heart muscle. The results suggested that by adding a sugar modification to a ribonucleoside at the position of C/U of the second nucleic acid strand, resistance to cleavage with RNase A is produced, and as a result, more efficient expression inhibition of the target gene becomes possible.

### [Example 6: In vivo effect of double-stranded nucleic acid complex comprising second nucleic acid strand having deoxyribonucleoside at position of C/U for inhibiting murine Malat1 RNA expression]

An *in vivo* effect of a double-stranded nucleic acid complex comprising a first nucleic acid strand comprising an antisense oligonucleotide targeting the murine Malat1 gene and a cholesterol-conjugated complementary strand (second nucleic acid strand) having a deoxyribonucleoside at the position of C/U for inhibiting mRNA expression in tissue was examined.

### (Preparation of nucleic acid agents)

An antisense oligonucleotide (ASO (mMalat1)) targeting the murine Malat1 gene, a cholesterol-conjugated complementary strand having a DNA at the position of C/U (Chol-cRNA (CT DNA)), and a complementary strand (Chol-cRNA (Default)) as a control were prepared. The sequences of ASO (mMalat1), Chol-cRNA (Default), and Chol-cRNA (CT DNA) are shown below.

**[Table 6]**

| Oligonucleotide name | Sequence (5'-3') | SEQ ID NO: |
|---|---|---|
| ASO (mMalat1) | G(L)*5(L)*A(L)*t*t*c*t*a*a*t*a*g*c*A(L)*G(L)*5(L) | 49 |
| Chol-cRNA (Default) | Chol-G*C*U*GCUAUUAGAA*U*G*C | 50 |
| Chol-cRNA (CT DNA) | Chol-G*C*U*GctAttAGAA*U*G*C | 51 |

| | | |
|---|---|---|
| Upper case (L):LNA (5(L) represents 5-methylcytosine LNA) Lower case: DNA Upper case: RNA Upper case (underlined): 2'-O-Me RNA *: Phosphorothioate bond Chol: Cholesterol | | |

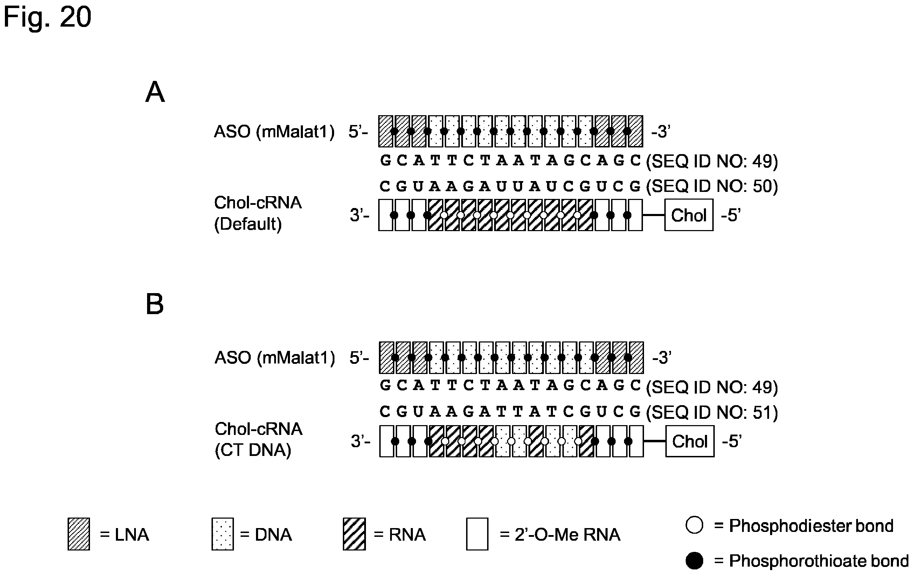

The aforedescribed ASO (mMalat1) is constituted by a 16-mer single-stranded LNA/DNA gapmer that targets the murine Malat1 gene and has a base sequence complementary to positions 5032 to 5047 which targets its transcription product Malat1 ncRNA (GenBank Accession No. NR_002847.3) (SEQ ID NO: 49). More specifically, this LNA/DNA gapmer consists of three LNA nucleosides from the 5' end, three LNA nucleosides from 3' end, respectively, and ten DNA nucleosides therebetween (Figure 20).

The aforedescribed Chol-cRNA (Default) has a sequence complementary to ASO (mMalat1) and comprises three 2'-O-methyl-modified ribonucleosides at the both ends and ten sugar-unmodified ribonucleosides therebetween, and a cholesterol is conjugated to the 5' end (Figure 20A).

The aforedescribed Chol-cRNA (CT DNA) has a deoxyribonucleoside at all of the positions of C and U among the ten sugar-unmodified ribonucleosides located at the center of Chol-cRNA (Default) (Figure 20B).

As in Example 1, ASO (mMalat1) and Chol-cRNA (Default) or ASO (mMalat1) and Chol-cRNA (CT DNA) were dissolved in PBS, mixed in equal molar volumes, the solution was heated at 98°C for 5 minutes, then cooled to 37°C, and held for 1 hour, whereby the nucleic acid strand was annealed. Thus, double-stranded nucleic acid complexes were prepared. The prepared double-stranded nucleic acid complexes are referred to as "Chol-HDO (Default)" or "Chol-HDO (CT DNA)", respectively.

### (In vivo experiment)

As mice to which the double-stranded nucleic acid complexes were administered, male C57BL/6 mice weighing 20 g and aged 4 to 5 weeks were used. Three mice were used for each condition.

Each double-stranded nucleic acid complex was intravenously injected into mice at a dose of 40 mg/kg via a tail vein in a single dose. In addition, mice injected with a single dose of PBS alone were also prepared as a negative control group.

### (Expression analysis)

At 72 hours after administration, mice were perfused with PBS and then dissected, whereby the cervical spinal cord was isolated. Subsequently, mRNA was extracted from each tissue according to the protocol using a high-throughput fully automated nucleic acid extractor MagNA Pure 96 (Roche Life Science). cDNA was synthesized according to the protocol of Transcriptor Universal cDNA Master (Roche Life Science). Quantitative RT-PCR was performed by TaqMan (Roche Life Science). The primers used in quantitative RT-PCR were products designed and manufactured by Thermo Fisher Scientific based on various gene numbers. The PCR conditions (temperature and time) were 95°C for 15 seconds, 60°C for 30 seconds, and 72°C for 1 second per cycle, which were repeated 40 cycles. Each obtained amplification product was quantified by quantitative RT-PCR, and based on the results, the expression level of RNA (mMalat1)/the expression level of mRNA (ACTB; internal standard gene) was calculated, thereby obtaining the relative expression level. Mean values and standard errors of relative expression levels were calculated.

### (Results)

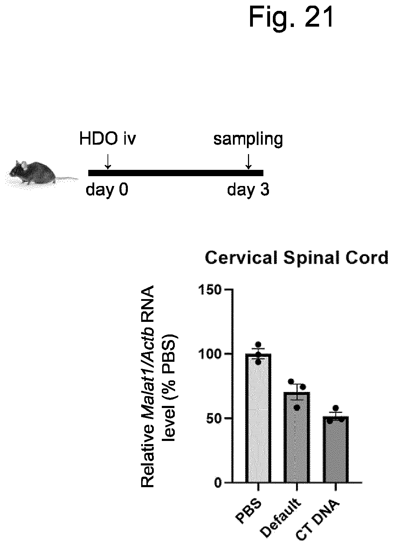

Figure 21 shows the results. Figure 21 shows the effects of double-stranded nucleic acid complexes Chol-HDO (Default) and Chol-HDO (CT DNA) for inhibiting the expression of the target mMalat1 gene in the cervical spinal cord. Error bars indicate the respective standard errors.

Chol-HDO (CT DNA) showed a stronger expression inhibition effect than Chol-HDO (Default) in the cervical spinal cord as well. The results suggested that by substituting a ribonucleosides with a deoxyribonucleosides at the positions of C/U in the second nucleic acid strand, resistance to cleavage by RNase A is produced, and as a result, more efficient expression inhibition of the target gene becomes possible.

### [Example 7: Stability of double-stranded nucleic acid complex comprising second nucleic acid strand comprising modified ribonucleosides in mouse and human sera]

A double-stranded nucleic acid complex comprising a first nucleic acid strand comprising an antisense oligonucleotide and a complementary strand (second nucleic acid strand) comprising modified ribonucleosides was mixed with mouse or human serum to evaluate double-strand stability in serum.

### (Preparation of nucleic acid agents)

Nucleic acids similar to Example 1 were prepared.

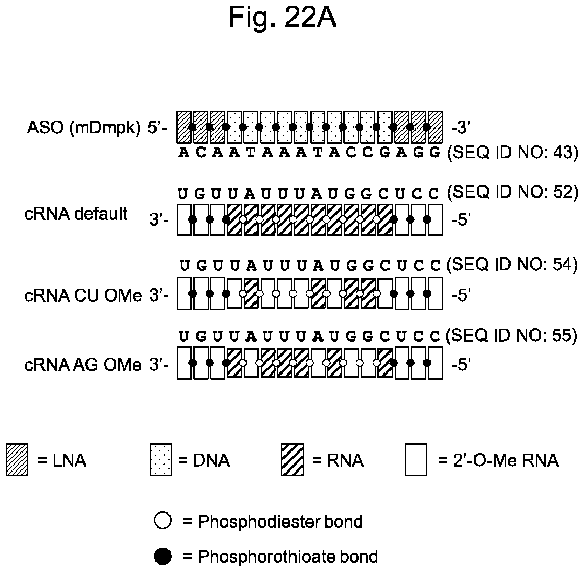

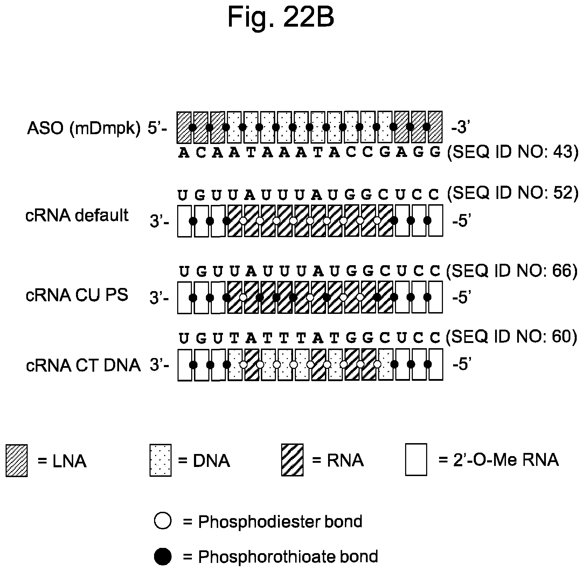

The sequences, chemical modifications, and structures of the oligonucleotides used in Example 1 are shown in Table 7 and Figure 22A and Figure 22B. All oligonucleotides were produced by Gene Design, Inc. (Osaka, Japan).

**[Table 7]**

| Oligonucleotide name | Sequence (5'-3') | SEQ ID NO: |
|---|---|---|
| ASO (mDmpk) | A(L)*5(L)*A(L)*a*t*a*a*a*t*a*c*c*g*A(L)*G(L)*G(L) | 43 |
| cRNA default | C*C*U*CGGUAUUUAU*U*G*U | 52 |
| cRNACU OMe | C*C*U*CGGUAUUUAU*U*G*U | 54 |
| cRNA AG OMe | C*C*U*CGGUAUUUAU*U*G*U | 55 |
| cRNA CT DNA | C*C*U*cGGtAtttAt*U*G*U | 60 |
| cRNA CU PS | C*C*U*C*GGU*AU*U*U*AU*U*G*U | 66 |

| | | |
|---|---|---|
| Upper case (L):LNA (5(L) represents 5-methylcytosine LNA) Lower case: DNA Upper case: RNA Upper case (underlined): 2'-O-Me RNA *: Phosphorothioate bond | | |

### (Evaluation experiment for stability in serum)

Ten microliters (10 µL) of 10 µM double-stranded nucleic acid annealed as described above was prepared. Serum was prepared from blood collected from Crl:CD1 (ICR) mice and human blood. Thirty microliters (30 µL) of mouse serum or human serum was mixed with 10 µL of 10 µM nucleic acid, and incubated at 37°C in an incubator. After 0 hr, 2 hr, or 24 hr incubation, a reaction stop buffer (50 mM Tris-HCl, pH 7.5, 48.5 mM borate, 20 mM EDTA, 10% SDS; final volume: 60 µL) was added and placed in liquid nitrogen, whereby the reaction was stopped.

### (Extraction)

The thawed sample was mixed with 60 µL of phenol:chloroform:isoamylalcohol (25:24:1) (NACALAI TESQUE, INC.), vortexed, and then incubated in a 37°C incubator for 20 minutes. Then, centrifugation was performed at 35°C and 12000 g for 30 minutes. The supernatant was mixed again with 60 µL of phenol:chloroform:isoamylalcohol (25:24:1) and the same procedure was repeated 3 times in total. The final supernatant was collected and mixed with a 7.5% sucrose solution to prepare an electrophoresis sample.

### (Electrophoresis/Analysis)

20% acrylamide gel (1 × TBE) was prepared. The aforedescribed electrophoresis sample was electrophoresed on gel at 100 V for 80 minutes. ASO alone was electrophoresed simultaneously as a control. Then, a solution was prepared by diluting a GelRed (× 10000) aqueous solution (Biotium) to a concentration of 1/10000 with 1 × TBE. The gel was impregnated with the solution for 10 minutes. The gel was then photographed with the ChemiDoc Touch Imaging System (BioRad). The band density was analyzed with Image J software.

### (Results)

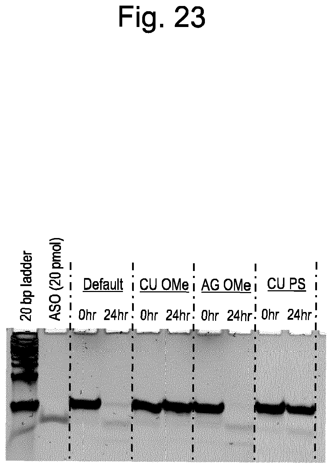

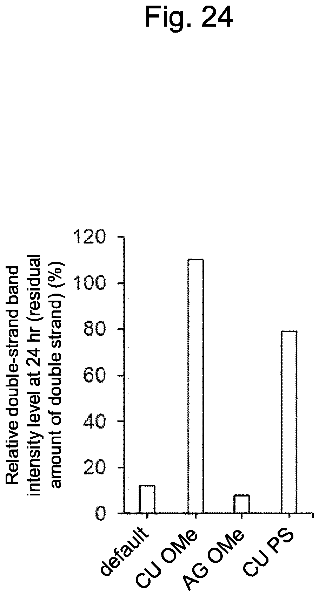

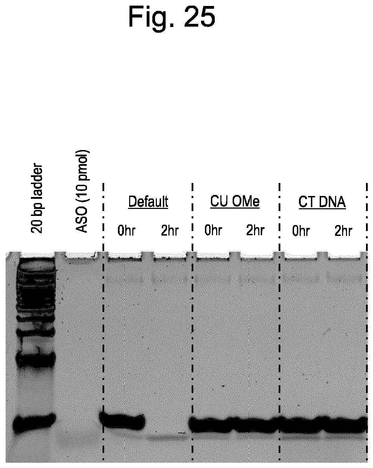

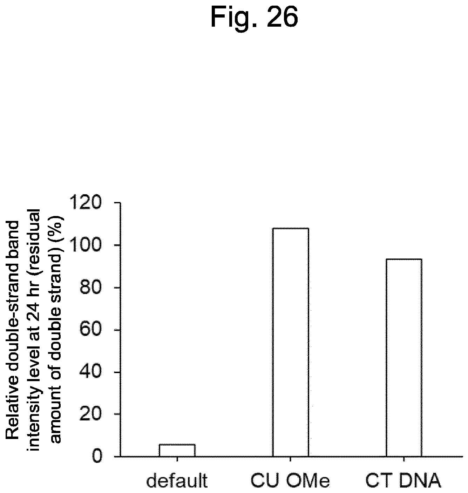

Figure 23 shows the results of electrophoresis of ASO (mDmpk) alone and the double-stranded complexes of ASO (mDmpk) and cRNA (default, CU OMe, AG OMe, CUPS) after mixing with mouse serum and the subsequent incubation. Figure 25 shows the results of electrophoresis of ASO (mDmpk) alone and the double-stranded complexes of ASO (mDmpk) and cRNA (default, CU OMe, CT DNA) after mixing with human serum and the subsequent incubation. Figures 24 and 26 show the densities of the double-stranded bands after 24 hr incubation in terms of the relative intensity level (%) with respect to the double-stranded band after 0 hr incubation. As shown in Figures 23 and 24, those in which C and U in the second nucleic acid strand were a 2'-O-methyl-modified ribonucleoside had improved stability in mouse serum. In addition, by changing the phosphodiester bond (PO) on the 3' side of ribonucleosides C and U in the second nucleic acid strand to a phosphorothioate bond (PS), the stability in mouse serum was also improved. Further, as shown in Figures 25 and 26, those in which C and U or T in the second nucleic acid strand were a 2'-O-methyl modified ribonucleoside or DNA had improved stability in human serum.

All publications, patents and patent applications cited herein are incorporated herein by reference in their entirety.

## Claims

1. A nucleic acid complex comprising a first nucleic acid strand and a second nucleic acid strand, wherein
said first nucleic acid strand:
(1) is capable of hybridizing to at least a part of a target transcriptional product;
(2) has an antisense effect on the target transcriptional product; and
(3) is a gapmer comprising: a central region comprising at least four contiguous deoxyribonucleosides; and a 5' wing region and a 3' wing region each comprising a nonnatural nucleoside, on the 5' end side and the 3' end side of the central region, respectively,
said second nucleic acid strand comprises at least one first exposed region and at least one protected region,
said first exposed region consists of one sugar-unmodified ribonucleoside or two or three contiguous sugar-unmodified ribonucleosides linked by an internucleoside bond, which is or are complementary to a part of said first nucleic acid strand,
said protected region consists of one of (a) a deoxyribonucleoside; (b) a sugar-modified nucleoside; and/or (c) a nucleoside having a modified internucleoside bond on the 3' side, or two or more of said (a) to (c) linked by an internucleoside bond, and
said first nucleic acid strand is annealed to said second nucleic acid strand.

2. The nucleic acid complex according to claim 1, wherein said first nucleic acid strand is from 10 to 26 base in length.

3. The nucleic acid complex according to claim 1 or 2, wherein said first exposed region consists of three contiguous sugar-unmodified ribonucleosides linked by an internucleoside bond.

4. The nucleic acid complex according to any one of claims 1 to 3, wherein said first exposed region comprises a nucleoside comprising a modified or unmodified purine base.

5. The nucleic acid complex according to any one of claims 1 to 4, wherein the second nucleic acid strand comprises only one said first exposed region.

6. The nucleic acid complex according to any one of claims 1 to 4, wherein said second nucleic acid strand comprises at least two said first exposed regions.

7. The nucleic acid complex according to any one of claims 1 to 6, wherein the sugar-unmodified ribonucleoside in said first exposed region is a natural ribonucleoside.

8. The nucleic acid complex according to any one of claims 1 to 7, further comprising a second exposed region consisting of four or more contiguous sugar-unmodified ribonucleosides linked by an internucleoside bond complementary to a part of said first nucleic acid strand, wherein the sugar-unmodified ribonucleoside in said second exposed region comprises a modified or unmodified purine base.

9. The nucleic acid complex according to any one of claims 1 to 8, wherein at least one of said protected region comprises: (a) a deoxyribonucleoside; (b) a sugar-modified nucleoside; and/or (c) a nucleoside having a modified internucleoside bond on the 3' side, each of (a) to (c) comprising a modified or unmodified pyrimidine base.

10. The nucleic acid complex according to claim 9, wherein all of said protected region comprises: (a) a deoxyribonucleoside; (b) a sugar-modified nucleoside; and/or (c) a nucleoside having a modified internucleoside bond on the 3' side, each of (a) to (c) comprising a modified or unmodified pyrimidine base.

11. The nucleic acid complex according to any one of claims 1 to 10, wherein at least one of said protected region consists of one of (a) a deoxyribonucleoside; (b) a sugar-modified nucleoside; and/or (c) a nucleoside having a modified internucleoside bond on the 3' side, each of (a) to (c) comprising a modified or unmodified pyrimidine base, or two or more of said (a) to (c) linked by an internucleoside bond.

12. The nucleic acid complex according to any one of claims 1 to 11, wherein the sugar-modified nucleoside in said protected region is 2'-O-methyl modified nucleoside and/or the nucleoside having a modified internucleoside bond on the 3' side is a nucleoside having a phosphorothioate bond on the 3' side.

13. The nucleic acid complex according to any one of claims 1 to 12, wherein said first exposed region and/or second exposed region comprises at least one modified internucleoside bond.

14. The nucleic acid complex according to any one of claims 1 to 13, wherein the second nucleic acid strand does not comprise a sugar-unmodified region other than the first exposed region and the second exposed region.

15. The nucleic acid complex according to any one of claims 1 to 13, wherein the second nucleic acid strand comprises a sugar-modified terminal region comprising at least one deoxyribonucleoside and/or sugar-modified nucleoside at the 5' end and/or the 3' end.

16. The nucleic acid complex according to any one of claims 1 to 15, wherein the first exposed region and/or the second exposed region in the second nucleic acid strand comprise at least one phosphorothioate bond chirally controlled in an Rp or Sp configuration, internucleoside bond comprising a guanidine moiety substituted with one to four C₁ to C₆ alkyl groups, and/or internucleoside bond comprising a cyclic guanidine moiety.

17. The nucleic acid complex according to any one of claims 1 to 16, wherein the protected region in said second nucleic acid strand comprises at least one phosphorothioate bond chirally controlled in an Rp or Sp configuration, internucleoside bond comprising a guanidine moiety substituted with one to four C₁ to C₆ alkyl groups, and/or internucleoside bond comprising a cyclic guanidine moiety.

18. The nucleic acid complex according to any one of claims 15 to 17, wherein said second nucleic acid strand does not comprise a sugar-unmodified region other than the first exposed region and the second exposed region.

19. The nucleic acid complex according to any one of claims 1 to 18, wherein said second nucleic acid strand is linked to a functional moiety having a function selected from a labeling function, a purifying function, and a function for delivery to a target.

20. A pharmaceutical composition comprising the nucleic acid complex according to any one of claims 1 to 19 as an active ingredient.
